# EUROPEAN PATENT APPLICATION

(11) **EP 3 831 408 A1**
(43) Date of publication of application: **09.06.2021**
(21) Application number: 19839942.0
(22) Date of filing: 26.07.2019
(51) Int. Cl.: A61K 45/00, A61K 31/7088, A61K 39/395, A61K 48/00, A61P 43/00, C12Q 1/6813, G01N 33/15, G01N 33/50, G01N 33/53

(54) **COMPOSITION FOR INHIBITING AGING, PREVENTING, IMPROVING, OR TREATING AGE-RELATED DISEASES, OR EXTENDING LIFESPAN**

(30) Priority: 27.07.2018 JP 2018141252
(71) Applicant: Osaka University, Suita-shi, Osaka 565-0871 (JP)
(72) Inventor: YOSHIMORI Tamotsu, Suita-shi, Osaka 565-0871 (JP); NAKAMURA Shuhei, Suita-shi, Osaka 565-0871 (JP)
(74) Representative: J A Kemp LLP
(86) International application number: PCT/JP2019/029502
(87) International publication number: WO 2020/022499

(57) **Abstract**

It was found that Rubicon is involved in aging through suppression of autophagic activity, and it is possible to achieve suppression of aging, prevention, amelioration, or treatment of an age-related disease or symptom, or extension of lifespan, by targeting Rubicon.

## Description

### [Technical Field]

The present invention relates to a composition targeting Rubicon for suppression of aging, prevention, amelioration, or treatment of an age-related disease or symptom, or extension of lifespan. In addition, the present invention relates to a method for evaluating a probability that a test compound has an activity of suppressing aging, an activity of preventing, ameliorating, or treating an age-related disease or symptom, or an activity of extending lifespan. Moreover, the present invention relates to a method for testing a degree of aging, a risk of developing an age-related disease or symptom, or a risk of shortening lifespan, as well as a molecule used for the test.

### [Background Art]

Macroautophagy (hereinafter, referred to simply as "autophagy") is an evolutionarily conserved intracellular membrane trafficking process in which double-membrane structures called autophagosomes sequester cytoplasmic materials and fuse with lysosomes, where their contents are degraded. Initially, autophagy was described as a bulk degradation system, but it has become clear that autophagy also selectively targets aggregated proteins, lipids, damaged organelles, and invading bacteria. By driving the degradation of a wide range of targets, autophagy maintains cellular homeostasis. Consequently, dysfunction in autophagy has been implicated in many human diseases, including cancer, neurodegeneration, and metabolic disorders.

Recent evidence has shown that autophagy is also involved in animal aging. Autophagic activity decreases with age in many species (NPLs 1 to 3). Studies using C. elegans led to the discovery of several conserved longevity pathways, including mild reduction of insulin/IGF-1 signalling, calorie restriction, germline removal, reduced mitochondrial respiration, and reduced TOR signalling. Importantly, all of these interventions activate autophagy and extend animal lifespan in a manner that depends on active autophagy. This suggests that autophagy is one of convergent mechanisms of many longevity pathways (NPLs 4, 5).

However, the detail of the mechanism by which the relation between the autophagic activity and aging is established has not yet been clarified.

### [Prior Art Document]

### [NPL]

[NPL 1] Chang, J. T. et al., Elife 6, 18459 (2017)
[NPL 2] Uddin, M. N. et al., Age (Dordr) 34, 75-85 (2012)
[NPL 3] Donati, A. et al., J Gerontol A Biol Sci Med Sci 56, B375-383 (2001)
[NPL 4] Toth, M. L. et al. Autophagy 4, 330-338 (2008)
[NPL 5] Madeo, F. et al, J Clin Invest 125, 85-93 (2015)
[NPL 6] Matsunaga, K. et al., Nature cell biology 11, 385-396 (2009)
[NPL 7] Zhong, Y. et al., Nat Cell Biol 11, 468-476 (2009)

### [Summary of Invention]

### [Technical Problem]

The present invention has been made in view of the above-described circumstance, and an object of the present invention is to identify a molecule based on which autophagic activity and aging are related. In addition, the present invention aims to achieve suppression of aging, prevention, amelioration, or treatment of an age-related disease or symptom, or extension of lifespan, by targeting the molecule. A further object of the present invention is to test the degree of aging, the risk of developing an age-related disease or symptom, or the risk of shortening lifespan, by targeting the molecule.

### [Solution to Problem]

Previously, the present inventors identified a negative regulator of autophagy, Rubicon (Run domain Beclin-1 interacting and cysteine-rich containing protein), which inhibits autophagosome-lysosome fusion process (NPLs 6, 7) . In view of this, the present inventors investigated whether or not Rubicon is involved in a mechanism based on which autophagic activity and aging are related.

As a result, the present inventors found that the expression of Rubicon increases with age in worm and mouse tissues. This suggests that an age-dependent increase of Rubicon impairs autophagy over time, and thereby curtails animal healthspan. This idea was also supported by the fact that knockdown of Rubicon extended worm and fly lifespan and ameliorated several age-related phenotypes. Tissue-specific experiments revealed that Rubicon knockdown in neurons had the greatest effect on lifespan. Moreover, Rubicon knockout mice exhibited reductions in interstitial fibrosis in kidney and α-synuclein accumulation in the brain, both of which are major age-onset phenotypes. In addition, Rubicon expression was suppressed by several lifespan-extending conditions, including calorie restriction, in both worms and mice.

Moreover, the present inventors found that Rubicon acts downstream of MML-1/Mondo, a convergent transcription factor involved in multiple longevity paradigms. Furthermore, epistatic analysis revealed that two major negative regulators of autophagy, mTOR and Rubicon, regulate lifespan partly independent of each other.

From the above, the present inventors found that Rubicon is a key molecule in aging through suppression of autophagic activity, and it is possible to suppress aging, prevent, ameliorate, or treat an age-related disease or symptom, or extend lifespan, or test the degree of aging, the risk of developing an age-related disease or symptom, or the risk of shortening lifespan, by targeting Rubicon, and completed the present invention.

More specifically, the present invention provides the following:
[1] A composition for suppression of aging, prevention, amelioration, or treatment of an age-related disease or symptom, or extension of lifespan in a subject, the composition comprising a molecule that suppresses functions of a Rubicon gene as an active ingredient.
[2] The composition according to [1], wherein
   the molecule that suppresses functions of a Rubicon gene is a molecule described in any one of the following (a) to (c):
   (a) an RNA binding to a transcription product of the Rubicon gene or a DNA encoding the RNA,
   (b) an RNA or DNA binding to a Rubicon protein, and
   (c) an antibody binding to the Rubicon protein.
[3] The composition according to [1] or [2], wherein
   the age-related disease or symptom is a metabolic bone disease or a symptom thereof, improvement in stress resistance, or an organ fibrosis.
[4] The composition according to any one of [1] to [3], wherein
   the subject is a vertebrate.
[5] A method for evaluating a probability that a test compound has an activity of suppressing aging, an activity of preventing, ameliorating, or treating an age-related disease or symptom, or an activity of extending lifespan in a subject, the method comprising the steps of:
   bringing the test compound and a Rubicon protein into contact; and
   detecting binding of the test compound and the Rubicon protein, wherein
   in a case where the test compound binds to the Rubicon protein, it is evaluated that there is the probability that the test compound has an activity of suppressing aging, an activity of preventing, ameliorating, or treating an age-related disease or symptom, or an activity of extending lifespan.
[6] A method for evaluating a probability that a test compound has an activity of suppressing aging, an activity of preventing, ameliorating, or treating an age-related disease or symptom, or an activity of extending lifespan in a subject, the method comprising the steps of:
   bringing the test compound into contact with a cell expressing a Rubicon gene; and
   detecting expression of the Rubicon gene in the cell, wherein
   in a case where the test compound reduces the expression of the Rubicon gene compared with a case where the test compound is not brought into contact, it is evaluated that there is the probability that the test compound has an activity of suppressing aging, an activity of preventing, ameliorating, or treating an age-related disease or symptom, or an activity of extending lifespan.
[7] A method for evaluating a probability that a test compound has an activity of suppressing aging, an activity of preventing, ameliorating, or treating an age-related disease or symptom, or an activity of extending lifespan in a subject, the method comprising the steps of:
   bringing the test compound into contact with a cell having a DNA in which a reporter gene is functionally bound to downstream of a promoter region of a Rubicon gene; and
   detecting expression of the reporter gene in the cell, wherein
   in a case where the test compound reduces the expression of the reporter gene compared with a case where the test compound is not brought into contact, it is evaluated that there is the probability that the test compound has an activity of suppressing aging, an activity of preventing, ameliorating, or treating an age-related disease or symptom, or an activity of extending lifespan.
[8] A method for testing a degree of aging, a risk of developing an age-related disease or symptom, or a risk of shortening lifespan in a subject, the method comprising the step of:
   detecting an expression product of a Rubicon gene in a test sample, wherein
   in a case where an amount of the expression product of the Rubicon gene is higher than that of control, it is evaluated that the degree of aging, the risk of developing an age-related disease or symptom, or the risk of shortening lifespan is high.
[9] The method according to any one of [5] to [8], wherein
   the age-related disease or symptom is a metabolic bone disease or a symptom thereof, improvement in stress resistance, or an organ fibrosis.
[10] The method according to any one of [5] to [9], wherein
   the subject is a vertebrate.
[11] A composition for detecting an expression product of a Rubicon gene in the method according to any one of [8] to [10], the composition comprising the following (a) or (b):
   (a) an oligonucleotide primer or oligonucleotide probe binding to a transcription product of the Rubicon gene; or
   (b) an antibody binding to a Rubicon protein.

### [Advantageous Effects of Invention]

The present invention makes it possible to achieve suppression of aging, prevention, amelioration, or treatment of an age-related disease or symptom, or extension of lifespan through activation of autophagy by suppressing functions of a Rubicon gene. In particular, it is very surprising that by suppressing functions of a single gene, various advantageous effects can be achieved, such as amelioration of decrease in bone mineral density and bone volume/bone tissue volume fraction, and change in bone morphology, which would cause metabolic bone diseases, reduction of aggregative proteins, which would cause neurodegenerations, reduction of eye degeneration, amelioration of decrease in locomotor function, improvement in stress resistance, reduction of organ fibrogenesis, extension of lifespan, and the like. In addition, the present invention also makes it possible to test the degree of aging, the risk of developing the above-described disease or symptom, or the risk of shortening lifespan by detecting an expression product of the Rubicon gene.

### [Brief Description of Drawings]

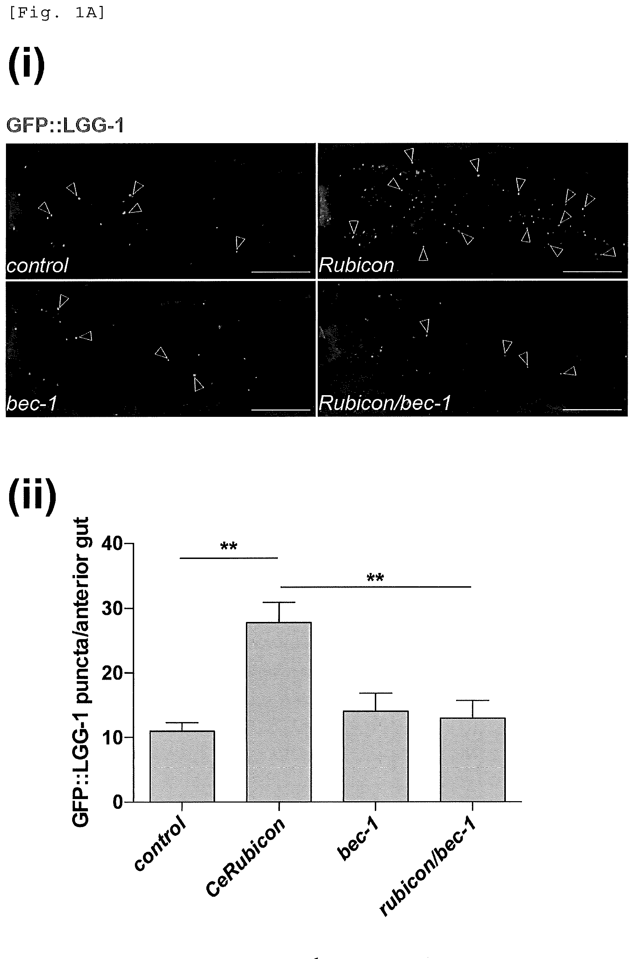

[Fig. 1A] Fig. 1A is diagrams showing that C. elegans Rubicon regulates lifespan via modulating autophagy. (i) shows pictures of anterior intestines at L4 stage. Each RNAi is shown. Autophagosomes (open white arrowheads) labelled with GFP: :LGG-1 were more abundant in CeRubicon knockdown, but concomitant knockdown of bec-1/Beclin 1 abolished the increase. The scale bar represents 20 µm. (ii) is a graph of quantification of GFP: :LGG-1 puncta in the anterior intestines under each knockdown condition are quantified. Values represent means ± s.e.m. (N=3). P-value (**P<0.01) was determined by one-way ANOVA with Tukey's test, relative to control.

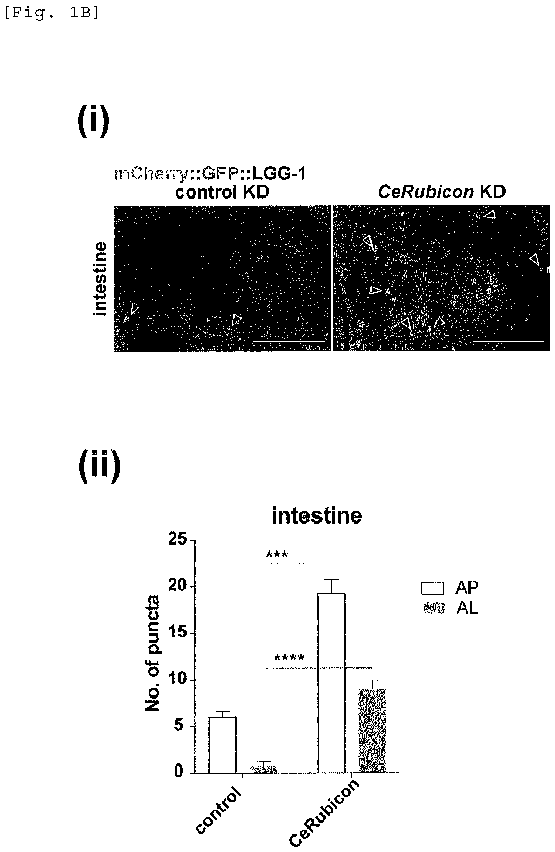

[Fig. 1B] (i) shows representative pictures of intestine in MAH215 (mCherry::GFP::LGG-1) strain subjected to control or CeRubicon RNAi. Autophagic flux is increased by CeRubicon knockdown. Both autophagosome (AP, GFP+ mCherry+, indicated by open white arrowheads) and autolysosome (AL, GFP- mCherry+, indicated by open magenta arrowheads) are increased by CeRubicon knockdown. (ii) is a graph in which AP and AL are quantified in each condition. P-value (***P<0.001, ****P<0.0001) was determined by t-test. The scale bar represents 10 µm.

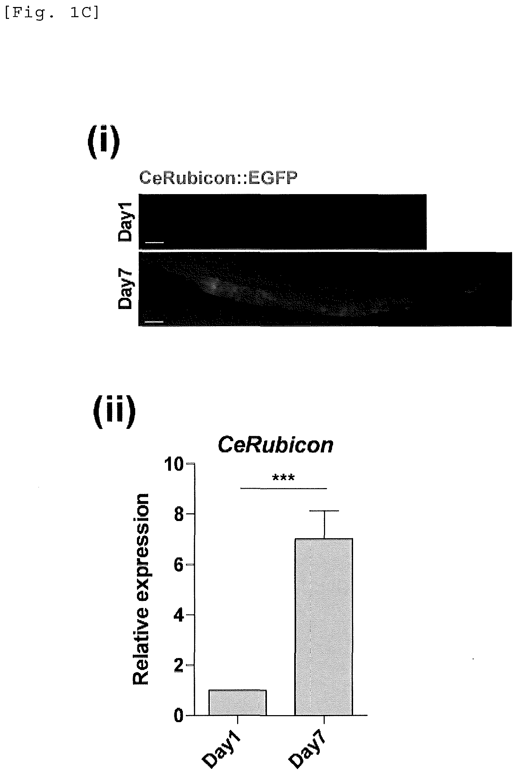

[Fig. 1C] (i) shows pictures of transgenic worms expressing CeRubicon::EGFP, showing that overall fluorescence is increased at day 7 compared to day 1. The scale bar represents 50 µm. (ii) is a graph of qRT-PCR analysis showing CeRubicon expression at day 1 and day 7 in wild-type worms. Mean ± s.e.m. from three independent experiments are depicted and are normalized to wild-type N2 Day-1 samples. P-value (***P<0.001) was determined by t-test.

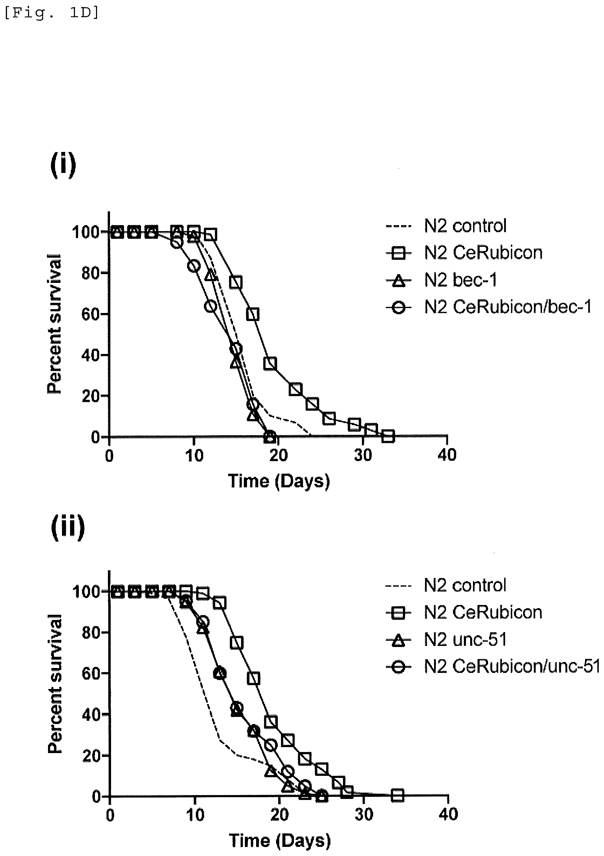

[Fig. 1D] Longevity conferred by CeRubicon was abolished by concomitant knockdown of (i) bec-1/Beclin 1 or (ii) unc-51/ULK1. Log-rank test was conducted for statistical analysis.

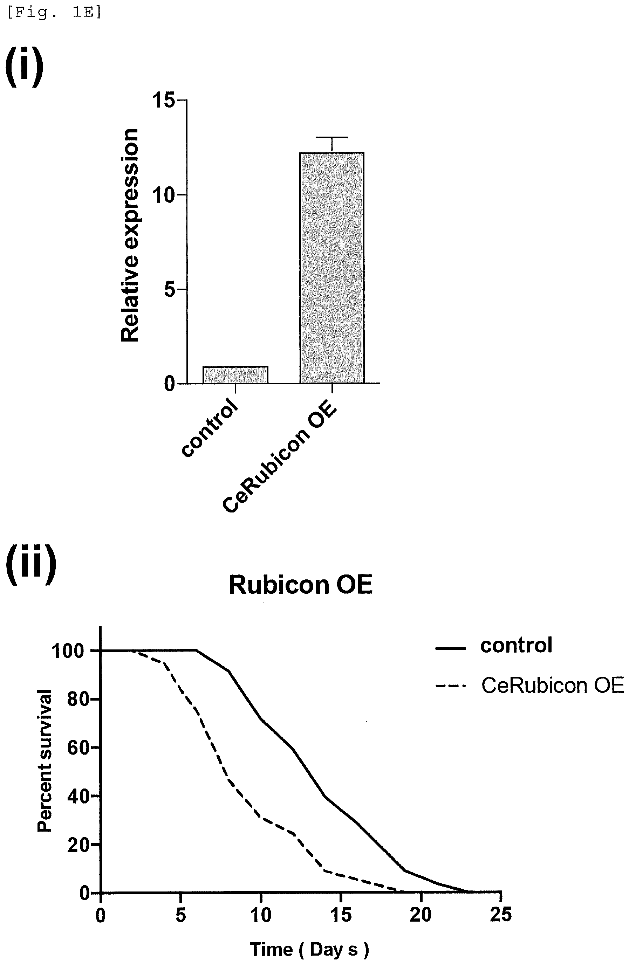

[Fig. 1E] (i) is a graph showing a result of qRT-PCR that confirmed that the transgenic worm increased CeRubicon expression. (ii) is a graph showing that overexpression of CeRubicon shortened lifespan of wild-type worms.

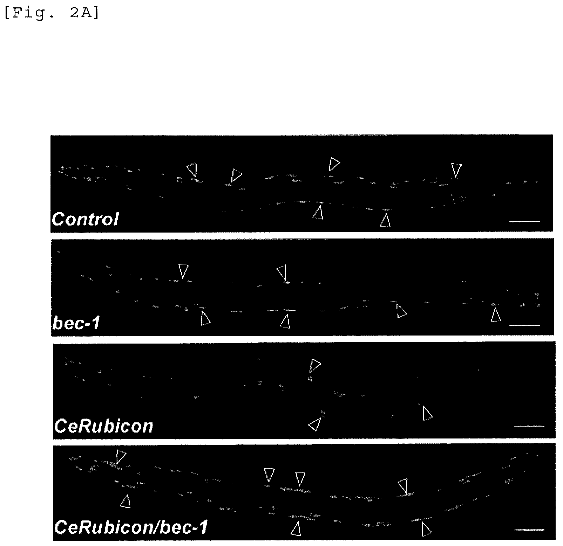

[Fig. 2A] Fig. 2A is a diagram showing that whole body Rubicon knockdown ameliorates age-dependent phenotypes. Transgenic worms expressing polyQ35::YFP in body wall muscle exhibited age-dependent accumulation of polyQ inclusions (open white arrowheads) at day 5 stage. CeRubicon knockdown decreased the accumulation of inclusions, and concomitant knockdown of bec-1 reverted the phenotypes. Each RNAi is indicated. The scale bar represents 50 pm.

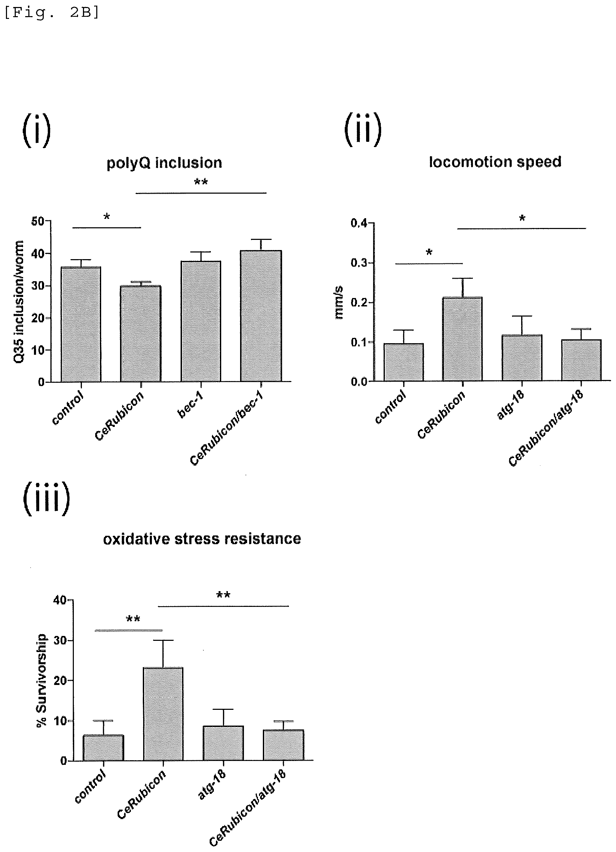

[Fig. 2B] (i) is a graph showing the number of polyQ inclusions per worm in Fig. 2A. More than 20 worms were analyzed in each experiment, and the experiments were repeated three times. P-values (*P<0.05, **P<0.01) were determined by t-test. (ii) is a graph showing locomotion speed, calculated from multi-worm tracking analysis at day 5. CeRubicon knockdown worms maintain high locomotion activity. P-value (*P<0.05) was determined by t-test. (iii) is a graph showing that CeRubicon knockdown increased the oxidative stress resistance in an autophagy-dependent manner, and is a graph showing survivorship of wild-type Day-1 worms subjected to indicated RNAi after 4.4 mM H₂O₂ treatment for 2 hours. P-value (**P<0.01) was determined by t-test.

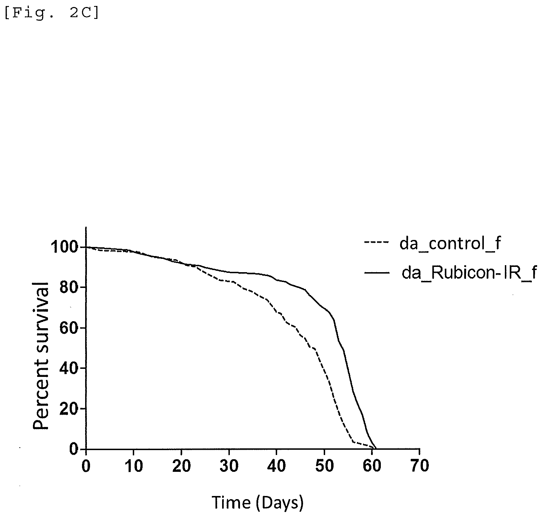

[Fig. 2C] Fig. 2C is a graph showing that systemic knockdown of dRubicon significantly extends lifespan of female fly.

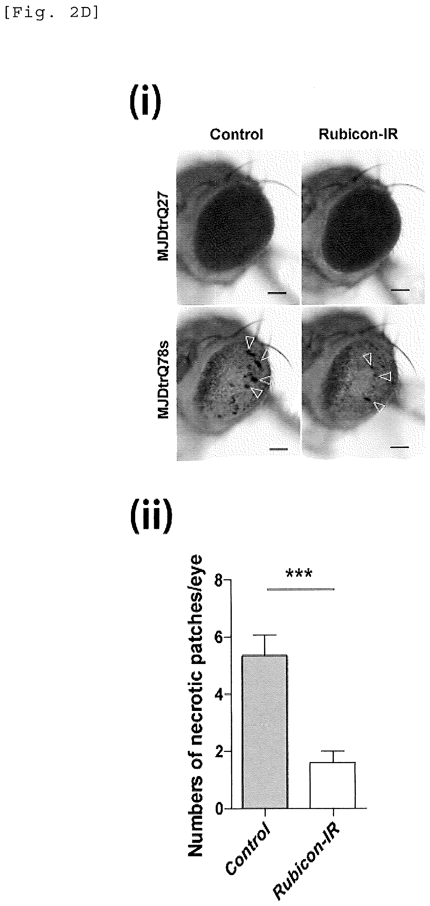

[Fig. 2D] (i) is pictures showing that compound eye degeneration was attenuated by dRubicon knockdown (dRubicon-IR) in flies expressing the expanded polyQ protein MJDtr-Q78s (gmr-GAL4>MJDtr-Q78s flies) compared to control. Open white arrowheads indicate necrotic patches. The scale bar represents 100 µm. (ii) is a graph of quantification of the numbers of necrotic patches in gmr-GAL4>MJDtr-Q78s flies in (i). More than 20 eyes were analyzed for each genotype. P-value (***P<0.001) was determined by t-test.

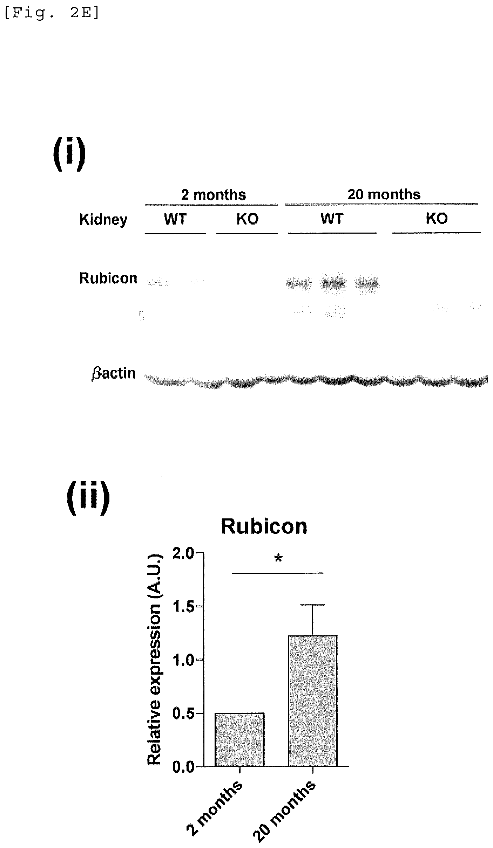

[Fig. 2E] (i) is western blotting image showing that Rubicon protein level in kidney was elevated in 20-month-old mice relative to 2-month-old mice. Western blot from WT mice and Rubicon KO (KO) mice shows the specific bands of Rubicon. (ii) is a graph of quantification of western blot shown in (i).

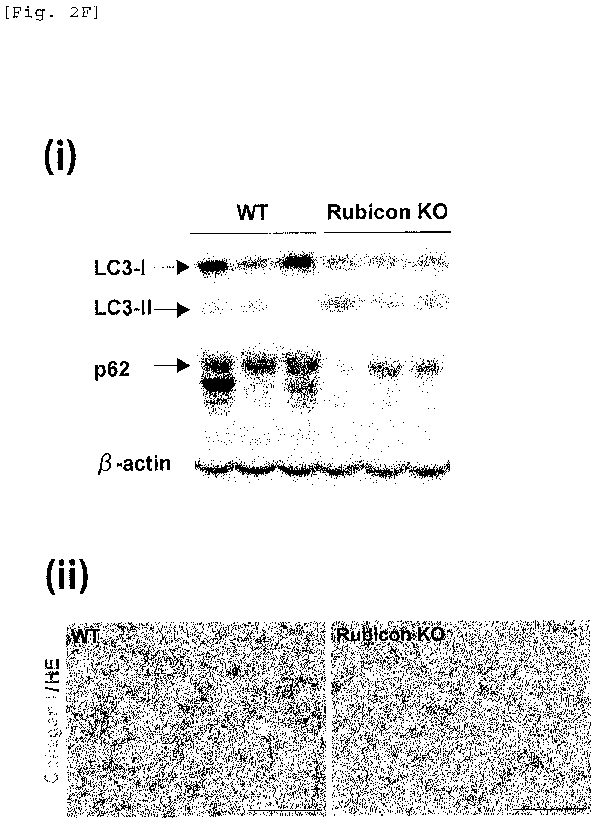

[Fig. 2F] (i) is western blotting images showing LC-3 and p62 in mice kidney at 20 months of age. (ii) is immunohistochemical images of 20 -month- old kidney showing that collagen-I positive area was reduced in Rubicon knock-out (Rubicon KO) kidney relative to WT. P-value (*P<0.05) was determined by t-test. The scale bar represents 100 µm.

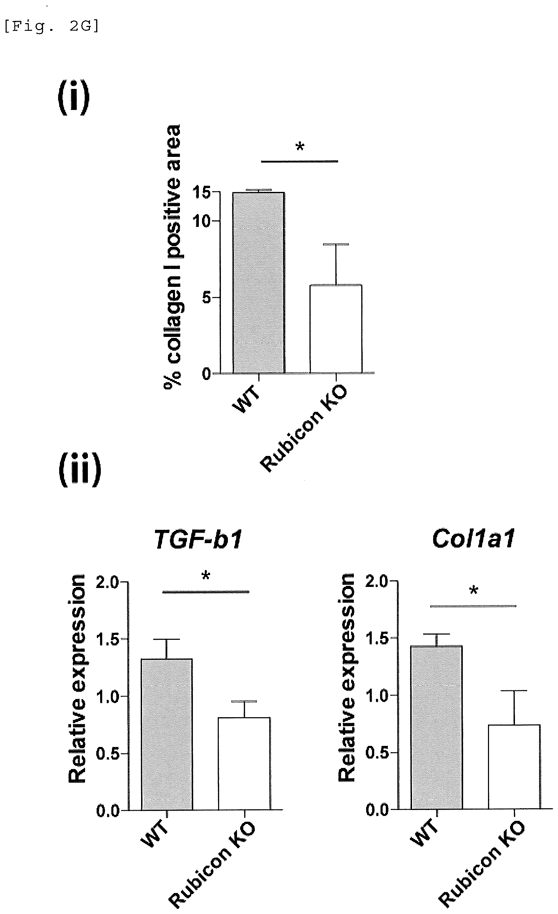

[Fig. 2G] (i) is a graph of quantification of collagen-I positive area in (ii) in Fig. 2F. Values represent means ± s.e.m. (WT: N=5; Rubicon KO: N=6). P-value (*P<0.05) was determined by t-test. (ii) is a graph showing a result of qRT-PCR analysis of several fibrosis markers (TGF-b1, Colla1) in 20-month-old kidney. Values represent means ± s.e.m. (WT: N=5; Rubicon KO: N=6). P-value (*P<0.05) was determined by t-test.

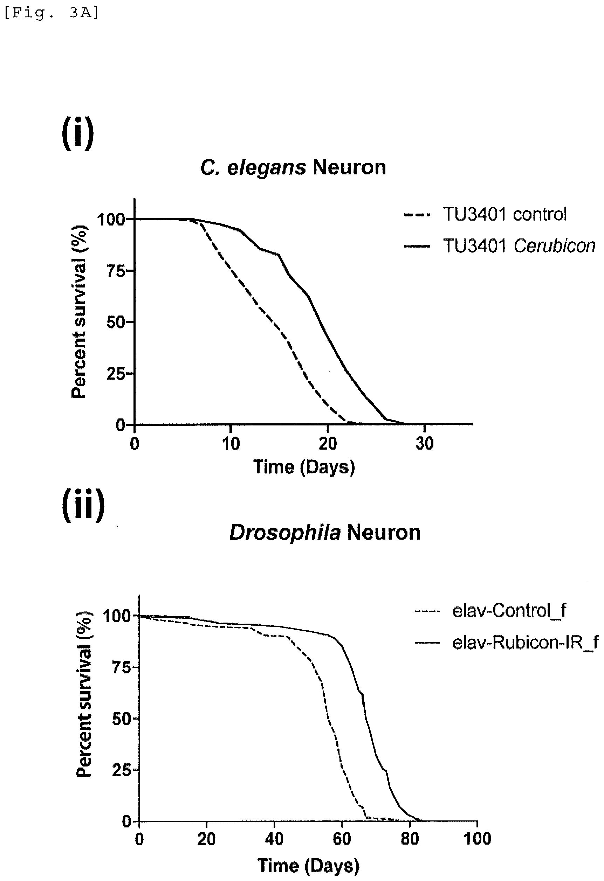

[Fig. 3A] Fig. 3A is diagrams showing that reduction of Rubicon level in neurons slows down aging. (i) is a graph showing that neuronal CeRubicon knockdown efficiently extended worm lifespan. (ii) is a graph showing that neuronal knockdown of dRubicon extended lifespan in female fly. Transgene expression of dRubicon-IR was induced by the pan-neuronal elav-GAL4 driver.

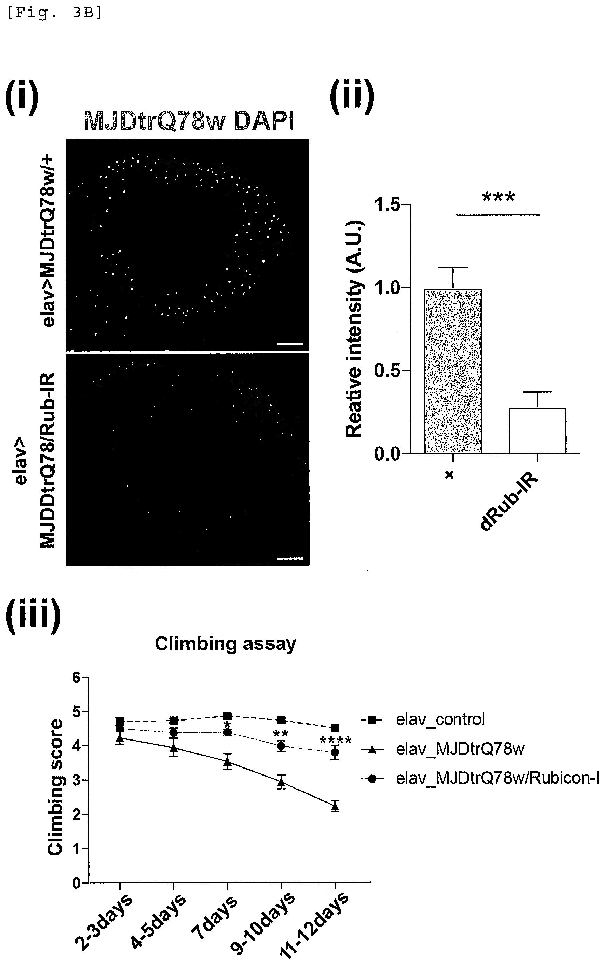

[Fig. 3B] (i) is pictures showing that neuronal knockdown of dRubicon decreased PolyQ (MJDtrQ78w) inclusions in Kenyon cell layer of female fly brain. The scale bar represents 10 pm. (ii) is a graph of quantification of PolyQ intensity in (i). 10 bilateral layers of Kenyon cell layer from 5 animals were analyzed. P-value (***P<0.001) was determined by t-test. (iii) is a graph showing that dRubicon knockdown in neurons ameliorated the locomotor dysfunction by polyQ expression in female fly. Locomotor function was evaluated by the climbing assay. Data represents mean ± s.e.m. P-values (*P<0.05, **P<0.01, ***P<0 .0001) were determined by two-way ANOVA with Tukey's test (elav_MJDtrQ78w vs elav_MJDtrQ78w/Rubicon-IR).

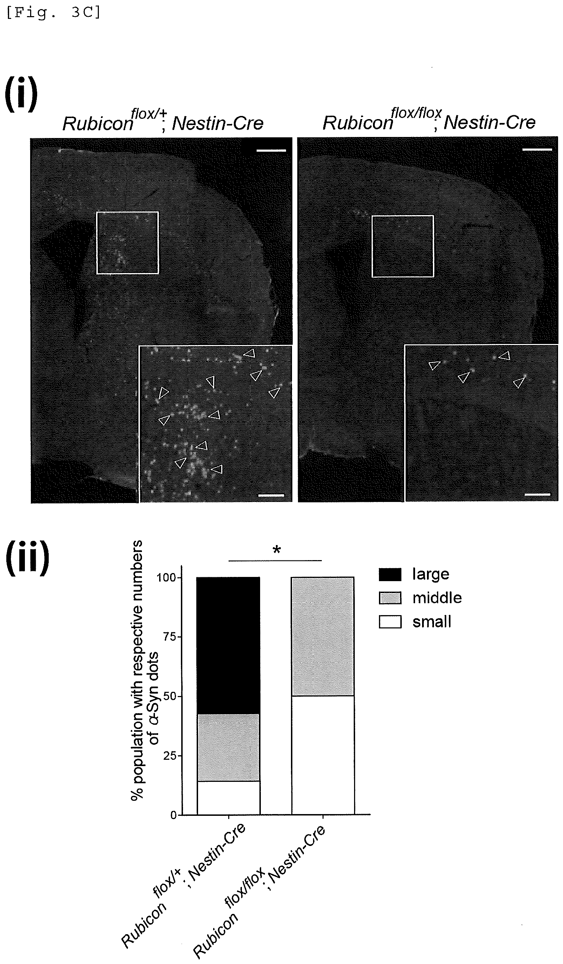

[Fig. 3C] (i) is pictures showing Lewy body and Lewy neurite-like inclusions containing phosphorylated α-Syn (open arrowheads), 10 months after the injection into mice. These were less abundant in Rubicon knock-out (Rubicon^{flow/flox}: Nestin-Cre) mice than in control (Rubicon^{flow/+}:Nestin-Cre). The scale bar represents 500 µm. (ii) is a graph showing percentages of mice having small (less than 100), moderate (100 to 200), or large (more than 200) numbers of phosphorylated α-Syn positive signals. P-value (*P<0.05) was determined by Chi-squared test (Rubicon^{flow/+}: Nestin-Cre, N=7; Rubicon^{flow/flox}: Nestin-Cre, N=6).

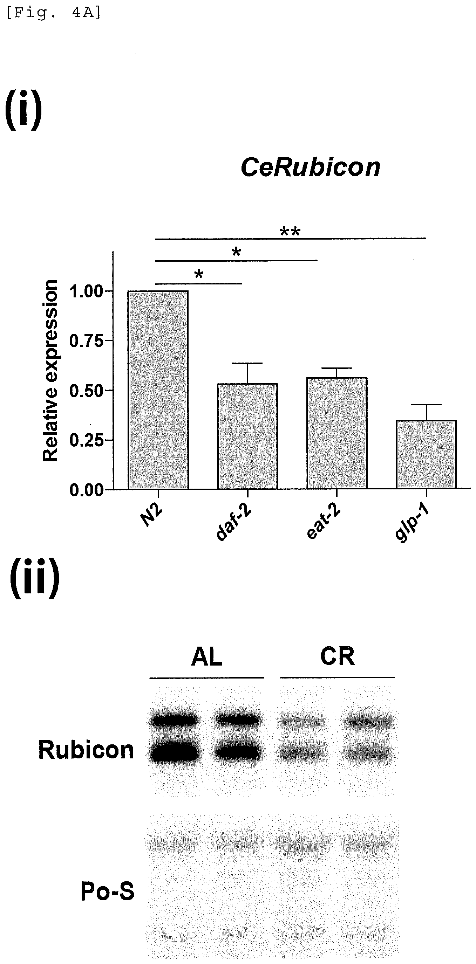

[Fig. 4A] Fig. 4A is diagrams showing that Rubicon is downregulated by several lifespan-extending conditions. (i) is a graph of qRT-PCR analysis showing CeRubicon expression in several long-lived strains including daf-2 (e1370), eat-2(ad465), and glp-1(e2141). Mean ± s.e.m. from three independent experiments are depicted and are normalized to wild-type N2 Day-1 samples. Germline-less phenotype of glp-1 was induced by exposure to elevated temperature (25°C) for 2 days. P-value (*P<0.05, **P<0.01) was determined by one-way ANOVA with Tukey's test. (ii) is a western blotting image showing that nine-month calorie restriction (CR) decreased Rubicon protein level in kidney compared to ad libitum (AL) feeding.

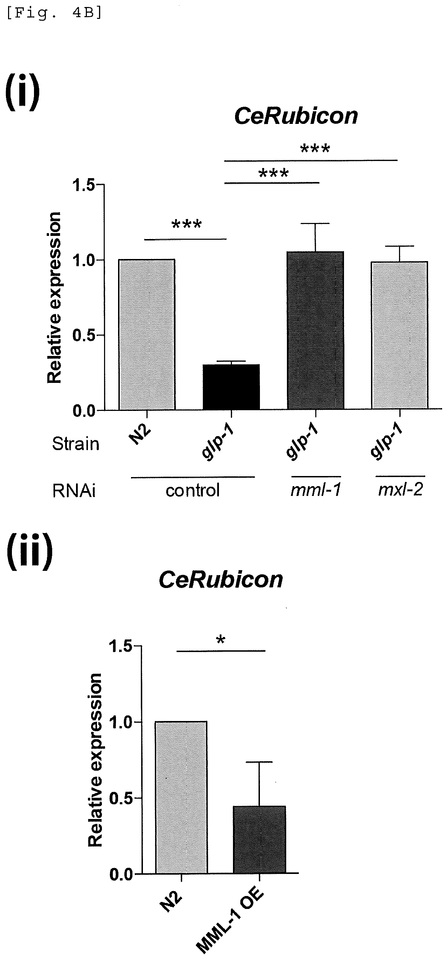

[Fig. 4B] (i) is a graph of qRT-PCR analysis showing CeRubicon expression in worms of the indicated strains exposed to the indicated RNAi treatments. Repression of CeRubicon in glp-1germline-less worms was abolished when MML-1 or mxl-2 was knocked down by RNAi. Mean ± s.e.m. from three independent experiments are depicted relative to wild-type N2 Day-1 samples. P-value (***P<0.001) was determined by one-way ANOVA with Tukey's test. (ii) is a graph of qRT-PCR analysis showing CeRubicon in wild-type (N2) and MML-1 OE (MML-1 overexpression strain). Mean ± s.e.m. from three independent experiments are depicted relative to wild-type N2 Day-1 samples. p-value (*P<0.05) was determined by t-test.

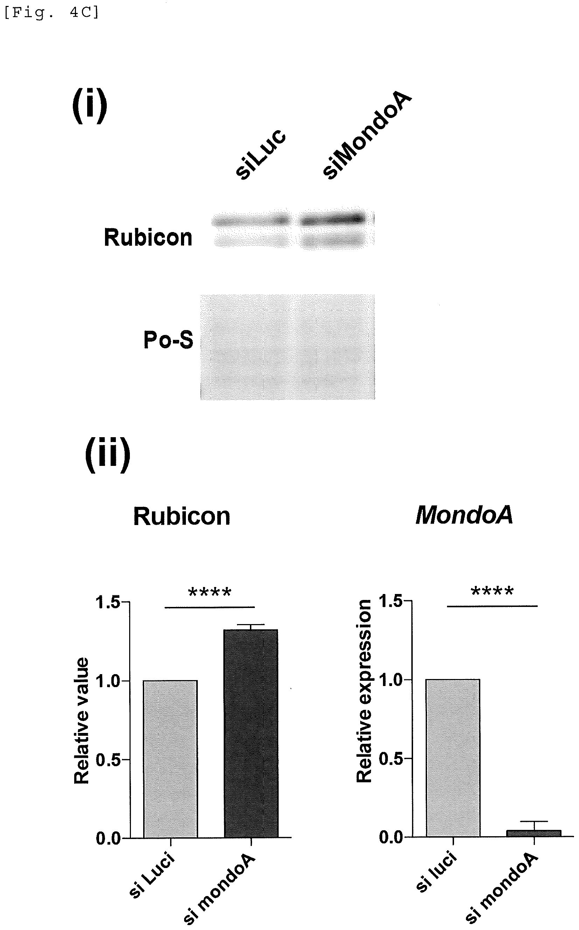

[Fig. 4C] (i) is a western blotting image showing that MondoA siRNA knockdown significantly increased Rubicon protein level in RPE-1 cells. (Left) of (ii) is a graph showing relative value of Rubicon normalized by Po-S. (Right) of (ii) is a graph of qRT-PCR analysis showing MondoA expression after knockdown with indicated siRNA. The value shows mean ± s.e.m. from three independent experiments relative to siLuc control. P-value (****P<0.0001) was determined by t-test.

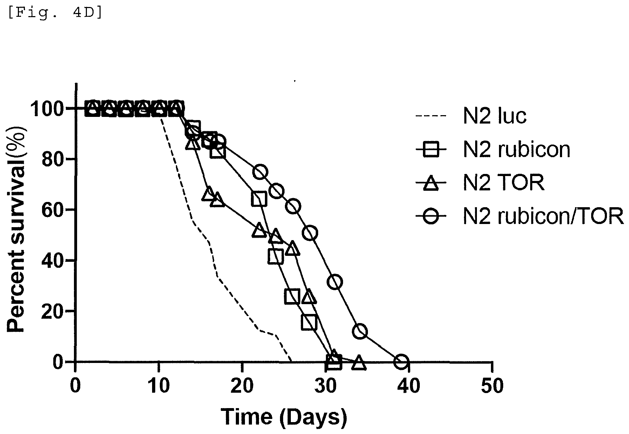

[Fig. 4D] Fig. 4D is a graph showing that double knockdown of CeRubicon and let-363/mTOR further extended lifespan in comparison with the individual knockdowns.

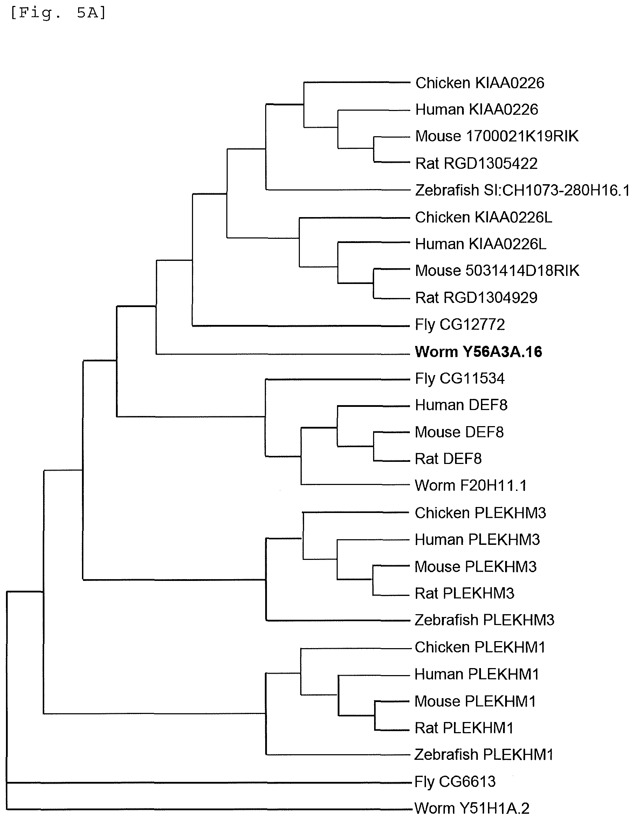

[Fig. 5A] Fig. 5A is a phylogenetic tree generated by ClustalW analysis showing that Rubicon homolog of C. elegans (Y56A3A.16) is similar to human KIAA00226 (Rubicon), human KIAA0226L, and fruit fly Rubicon (CG12772) .

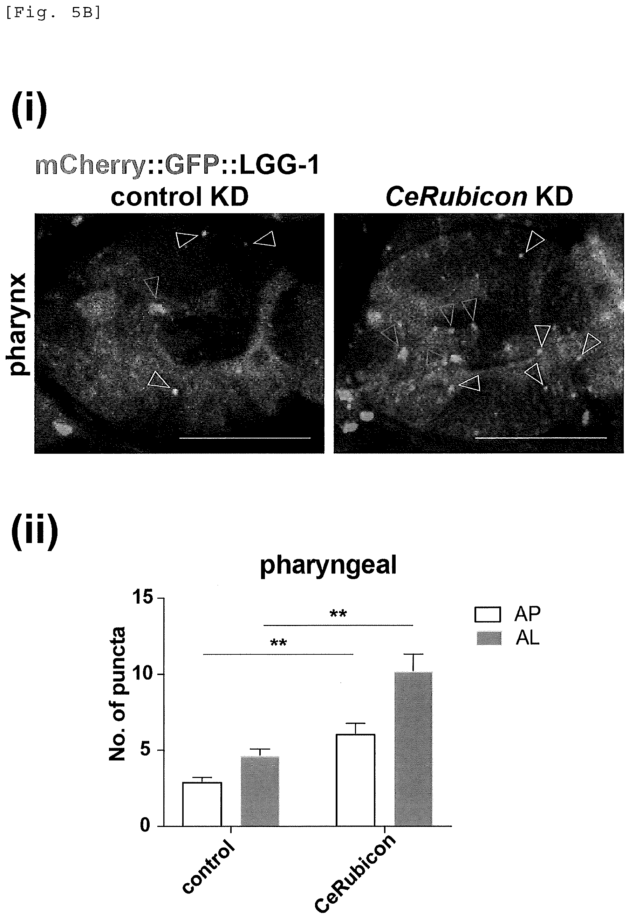

[Fig. 5B] (i) is representative pictures of pharyngeal muscle in MAH215 (mCherry::GFP::LGG-1) strains subjected to control or CeRubicon RNAi, showing that autophagic flux is increased by CeRubicon knockdown. Both autophagosome (AP, GFP+ mCherry+, indicated by open white arrowheads) and autolysosome (AL, GFP- mCherry+, indicated by open magenta arrowheads) are increased by CeRubicon knockdown. The scale bar represents 20 µm. (ii) is a graph of quantification of AP and AL in each condition. P-value (**P<0.01) was determined by t-test.

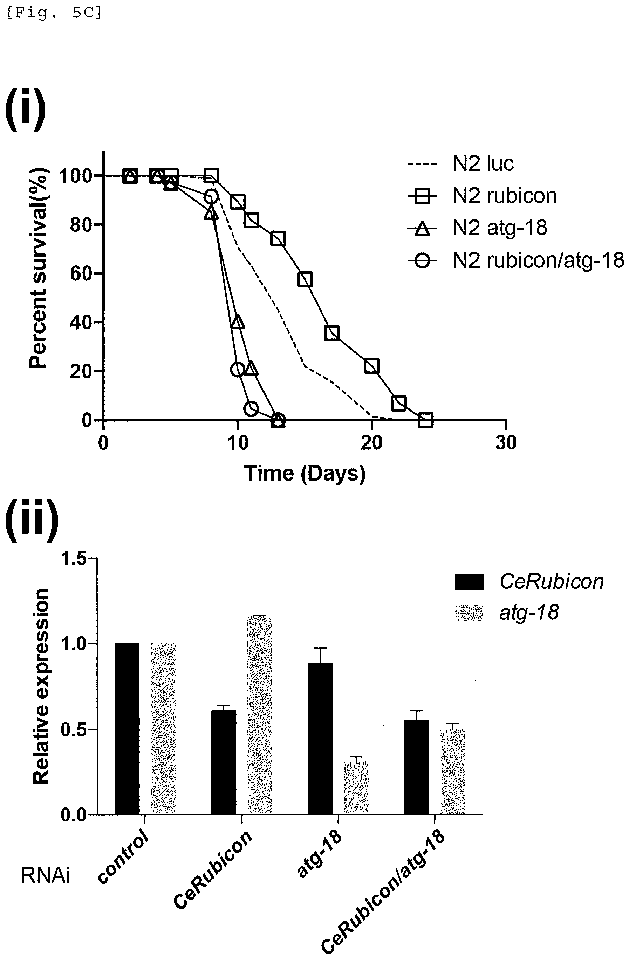

[Fig. 5C] (i) is a graph showing that CeRubicon knockdown extended lifespan, but concomitant knockdown of atg-18 abolished the longevity. (ii) is a graph of qRT-PCR analysis showing knockdown efficiency of CeRubicon and atg-18 by RNAi. Mean ± s.e.m. from three independent experiments are depicted relative to wild-type N2 with control RNAi.

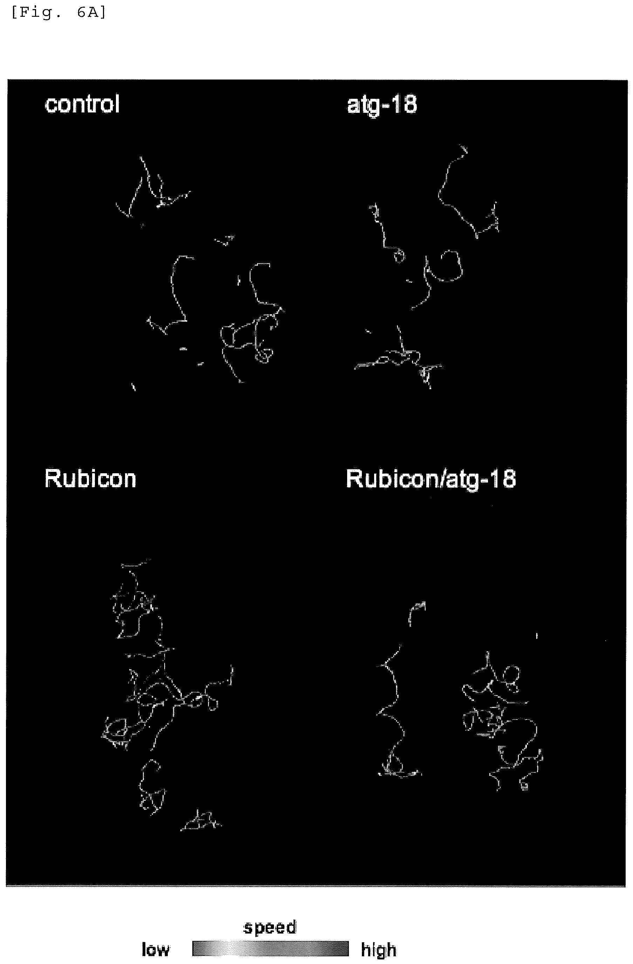

[Fig. 6A] Fig. 6A is a snapshot of multi-worm tracking analysis showing age-related changes by Rubicon knockdown. Track and speed of individual worms were visualized under the indicated RNAi treatments.

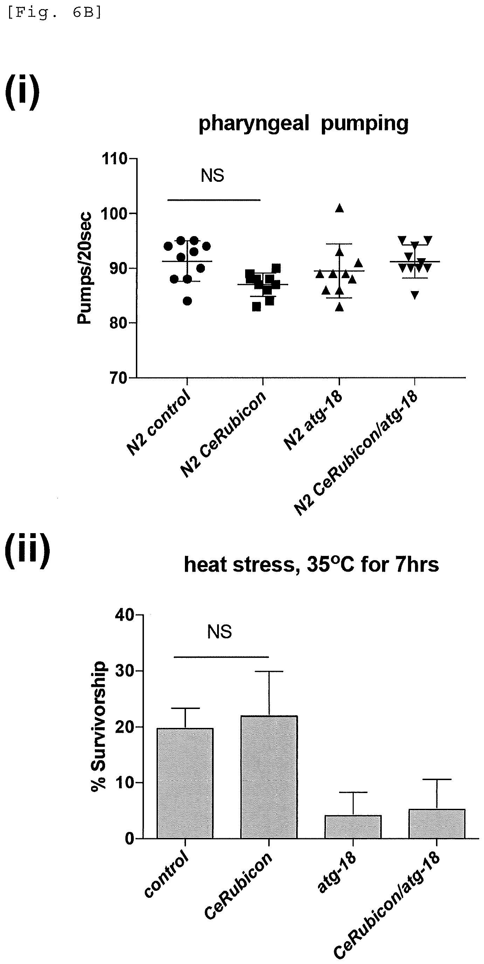

[Fig. 6B] (i) is a graph showing that pharyngeal pumping rate is not altered in CeRubicon knockdown. (ii) is a graph showing a result of heat stress resistance assay, which revealed that CeRubicon knockdown does not change the resistance.

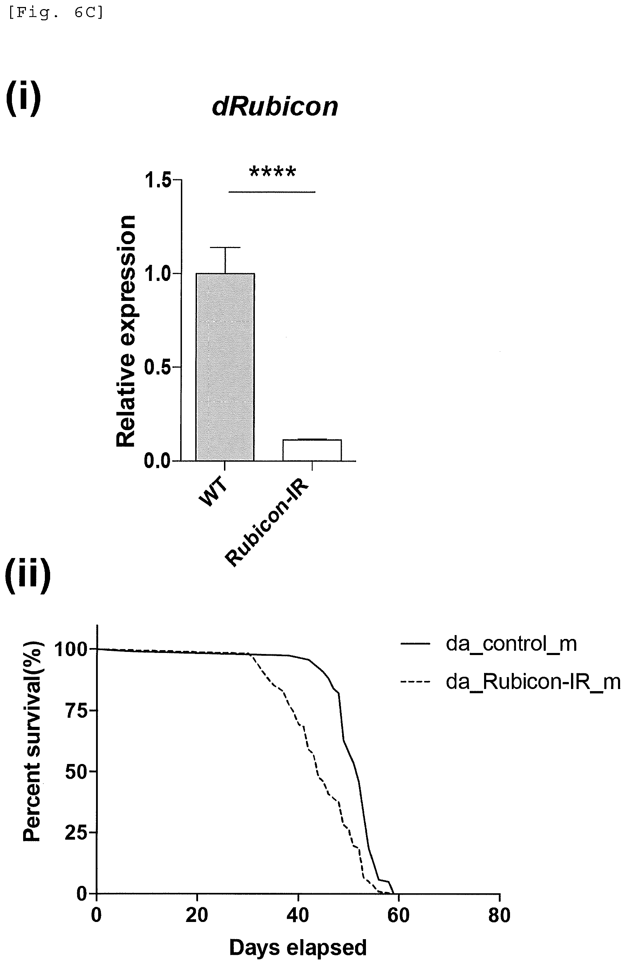

[Fig. 6C] (i) is a graph of qRT-PCR analysis showing that dRubicon was significantly downregulated by the knockdown. P-value (****P<0.0001) was determined by t-test (WT: N=3; Rubicon-IR: N=4). (ii) is a graph of demographic analysis of dRubicon knockdown in male fly.

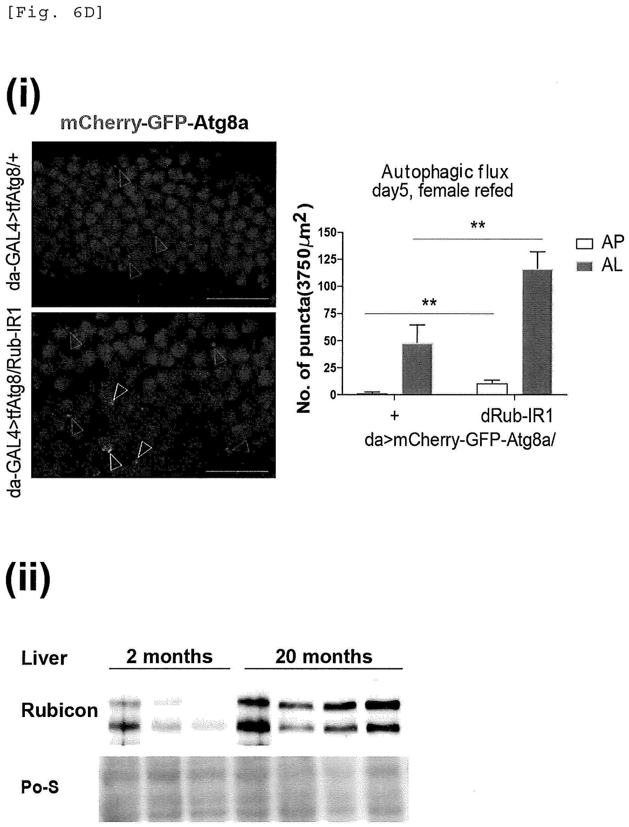

[Fig. 6D] (i) is pictures and a graph showing a result of the autophagic flux assay in KC layers of brains, which shows that dRubicon knockdown increased autophagosomes (AP, white arrowheads) and autolysosomes (AL, red arrowheads). The values represent the mean ± s.e.m. P-values (**P<0.01) were determined by t-test (N=6 for control, N=8 for dRUb-IRl) . The scale bar represents 10 µm. (ii) is a western blotting image showing that Rubicon protein levels increased with age (2 months vs 20 months) in mouse liver.

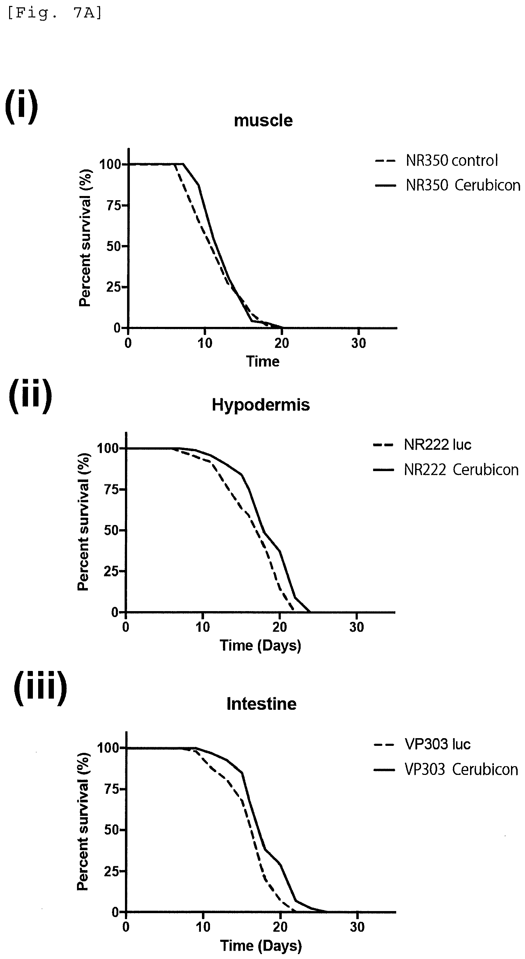

[Fig. 7A] Fig. 7A is diagrams showing contribution of tissue-specific role of Rubicon to lifespan regulation, showing graphs of demographic analysis using several tissue-specific RNAi-sensitive strains such as (i) muscle (NR350), (ii) hypodermis (NR222), and (iii) intestine (VP303). intestinal and hypodermal CeRubicon knockdown slightly but significantly extended lifespan.

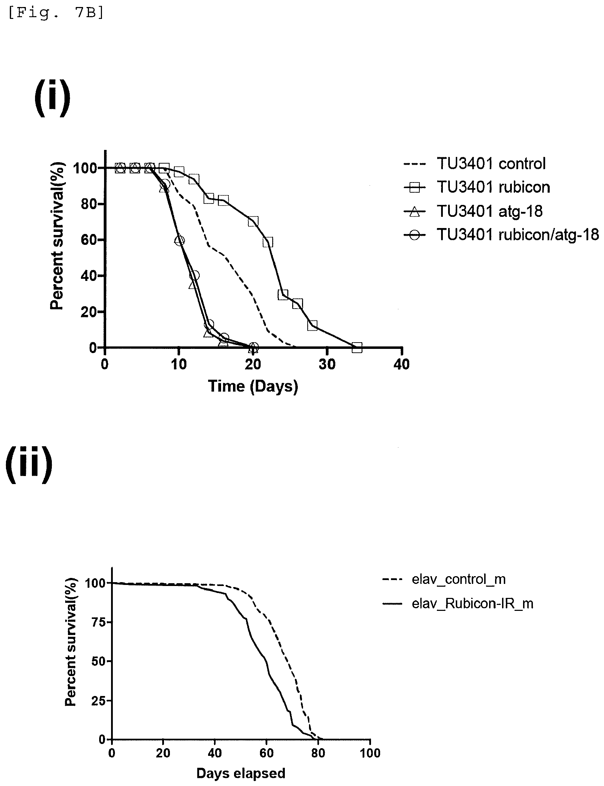

[Fig. 7B] (i) is a graph showing that longevity conferred by neuronal knockdown of CeRubicon is abolished by neuronal atg-18 knockdown. (ii) is a graph of demographic analysis of neuronal dRubicon knockdown in male fly.

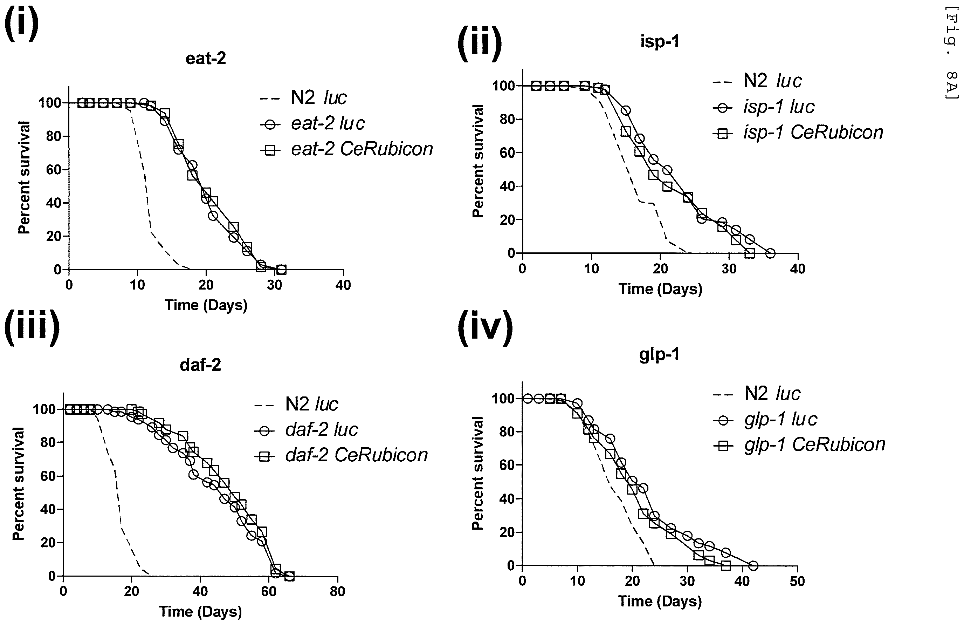

[Fig. 8A] Fig. 8A is graphs showing epistatic relationships between CeRubicon knockdown and several longevity pathways. CeRubicon knockdown does not further extend longevity of (i) eat-2 (calorie restriction), (ii) isp-1 (mitochondrial dysfunction), (iii) daf-2 (reduced insulin/IGF-1 signalling), and (iv) glp-1 (germline longevity) .

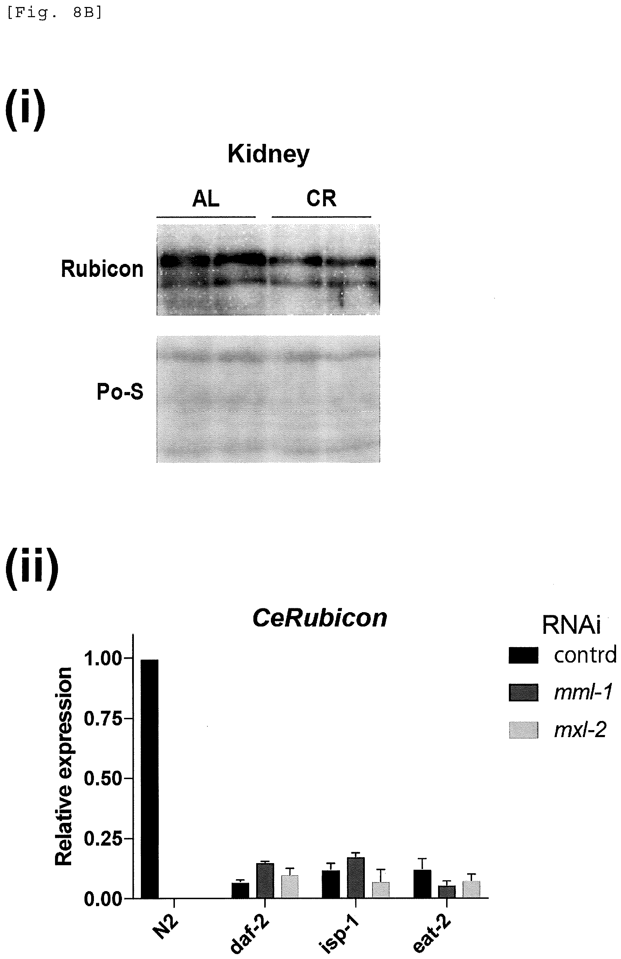

[Fig. 8B] (i) is a western blotting image showing that 6-month calorie restriction decreases Rubicon protein levels in kidney. (ii) is a graph showing qRT-PCR analysis of CeRubicon expression. Knockdown of MML-1 and mxl-2 do not revert CeRubicon expression to wild-type (N2) level.

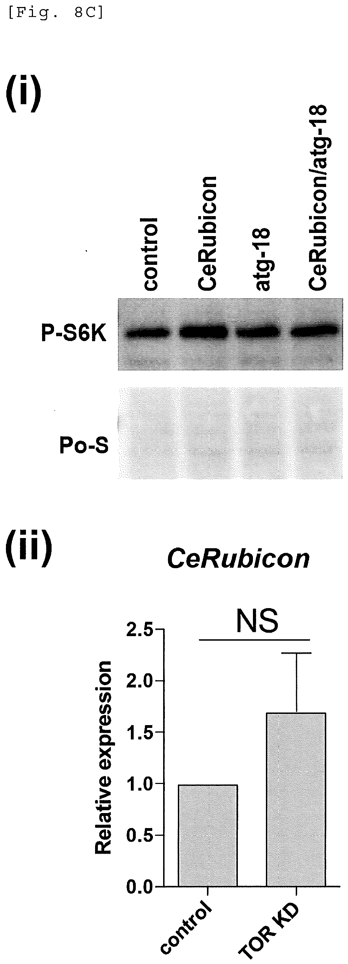

[Fig. 8C] (i) is a western blotting image showing that knockdown of CeRubicon does not change levels of phosphor S6 kinase, a direct target of mTOR. (ii) is a graph of qRT-PCR analysis of CeRubicon expression showing that CeRubicon is not altered by TOR knockdown.

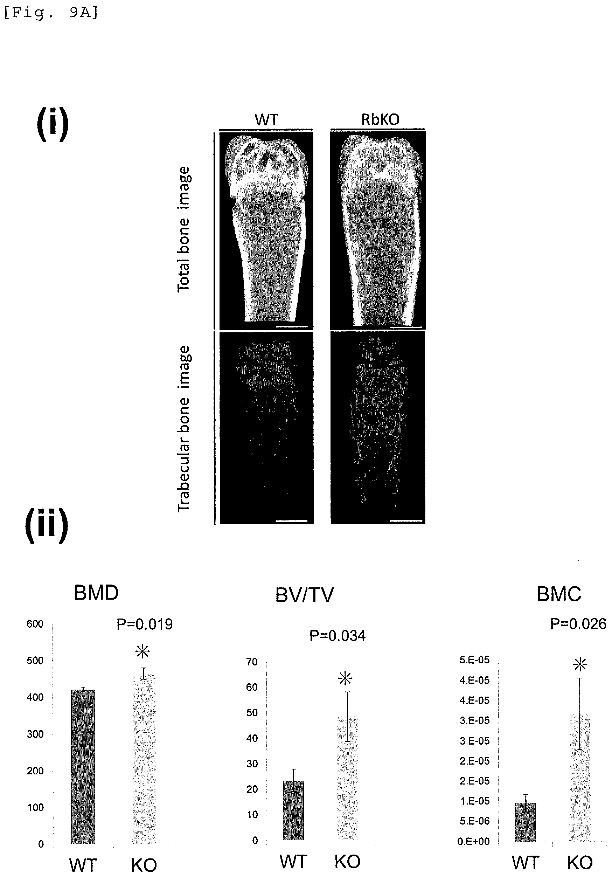

[Fig. 9A] (i) is CT images of femurs of Rubicon knockout mice. The scale bar represents 500 µm. (ii) is graphs showing the bone mineral density (BMD), the bone volume/bone tissue volume fraction (BV/TV), and the bone mineral content (BMC) of the femurs of the Rubicon knockout mice. N=6 for wild-type (WT), and N=5 for knockout mouse (KO) .

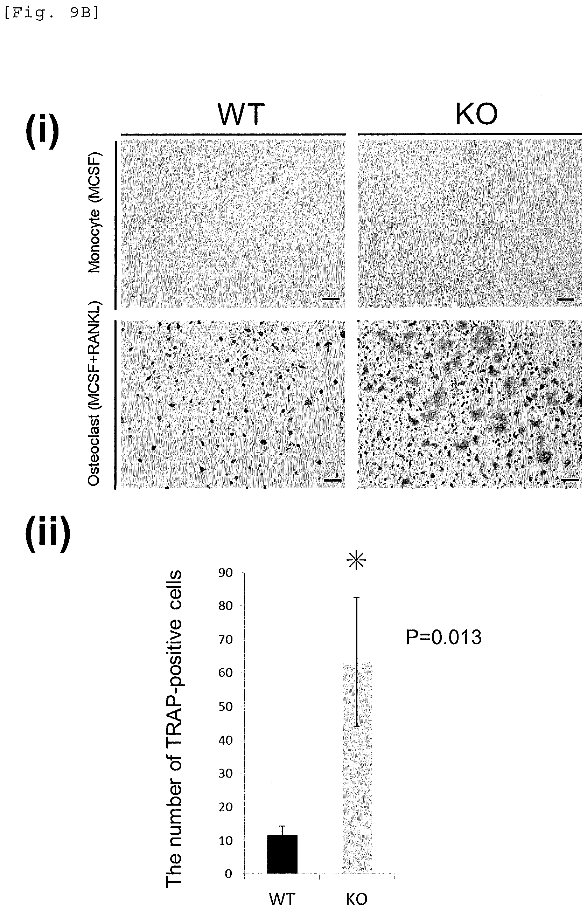

[Fig. 9B] (i) is pictures in which differentiation of osteoclasts in Rubicon knockout mice was detected through TRAP staining. The scale bar represents 200 µm. (ii) is a graph showing a result of measuring the numbers of TRAP positive cells.

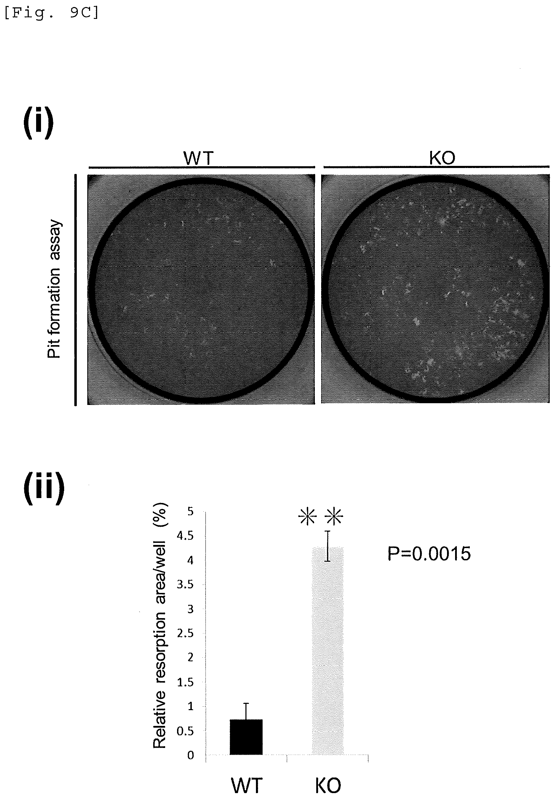

[Fig. 9C] (i) We evaluated the bone resorption function of osteoclasts in Rubicon knockout mice through pit formation assay. (i) is TRAP-stained images, and (ii) is a graph showing proportions of relative resorption regions in well.

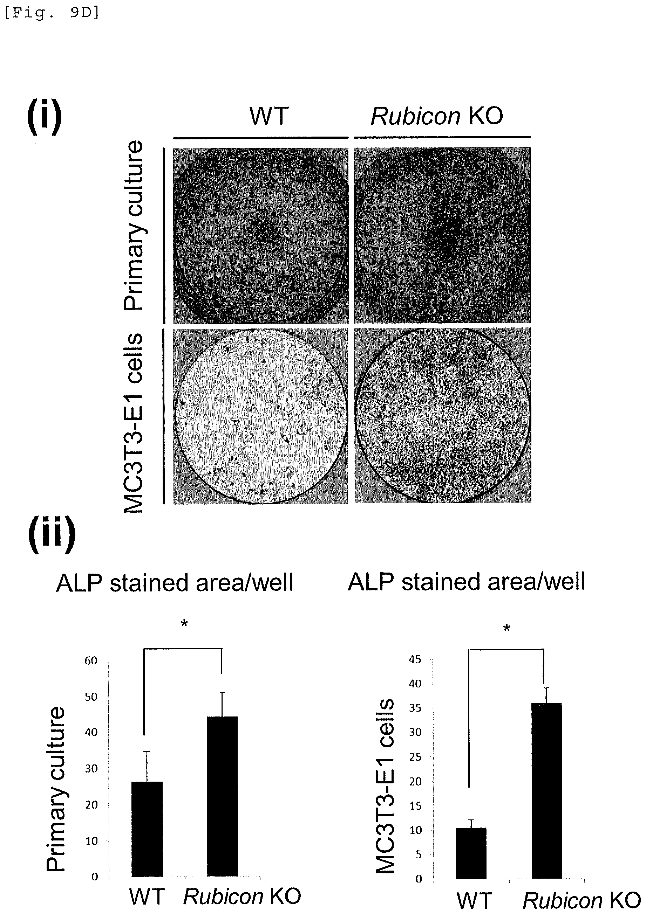

[Fig. 9D] We evaluated the differentiation potential of osteoblasts in Rubicon knockout mice. (i) is ALP-stained images, and (ii) is graphs showing proportions of ALP-stained areas in well.

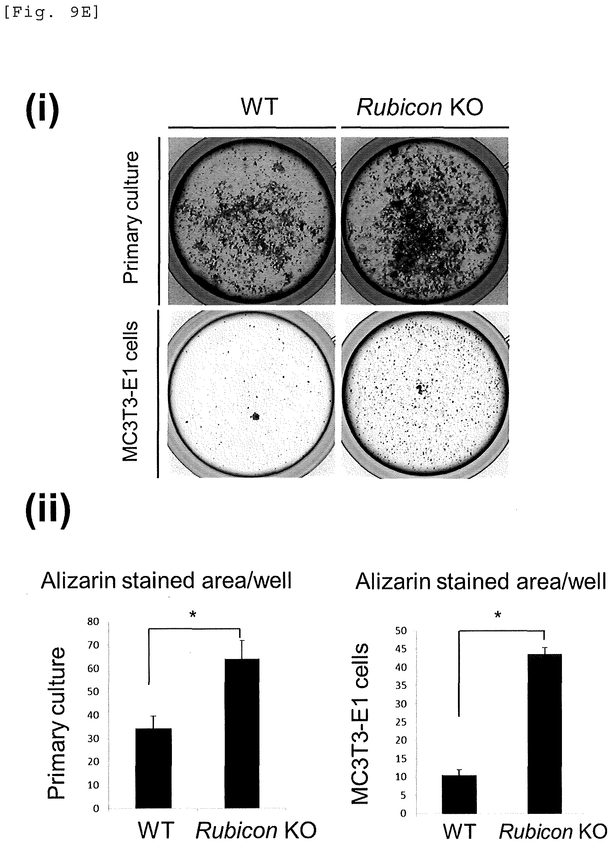

[Fig. 9E] Fig. 9E show a result of evaluating mineralization capacity of osteoblasts in Rubicon knockout mice. (i) is alizarin-stained images, and (ii) is graphs showing proportions of alizarin-stained areas in well.

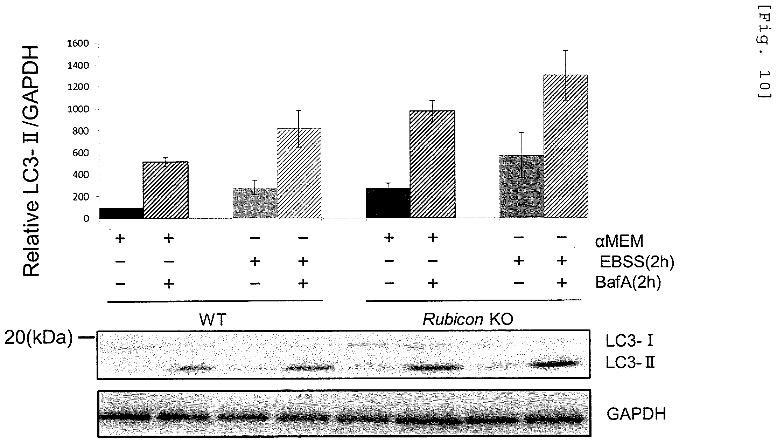

[Fig. 10] Fig. 10 is a graph and electrophoresis images showing a result of observing facilitation of autophagy in osteoblasts of Rubicon knockout mice through LC3flux assay.

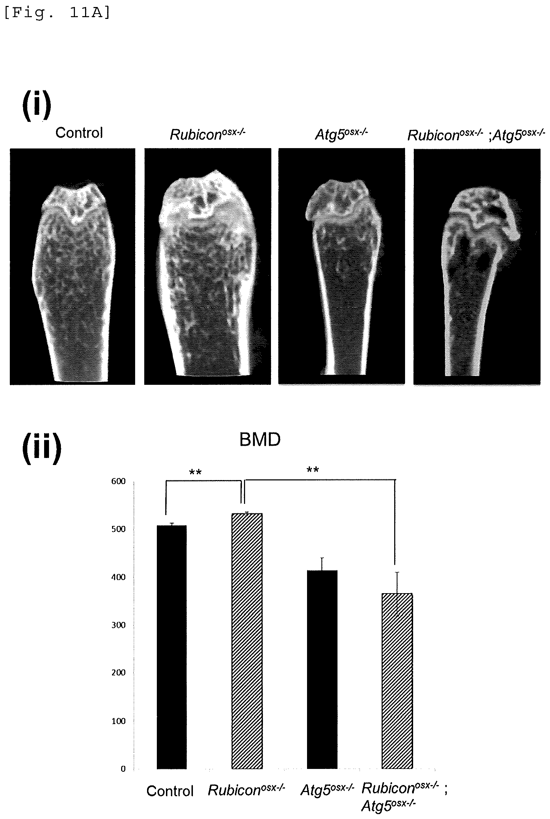

[Fig. 11A] Fig. 11A shows a result of evaluating autophagy-dependency of increase in bone mineral density in Rubicon knockout mice. (i) is pictures showing results of CT-analyzing bone morphology in a Rubicon knockout mouse, an Atg5 knockout mouse, and a Rubicon/Atg5 knockout mouse. (ii) is a graph showing bone densities in the respective mice.

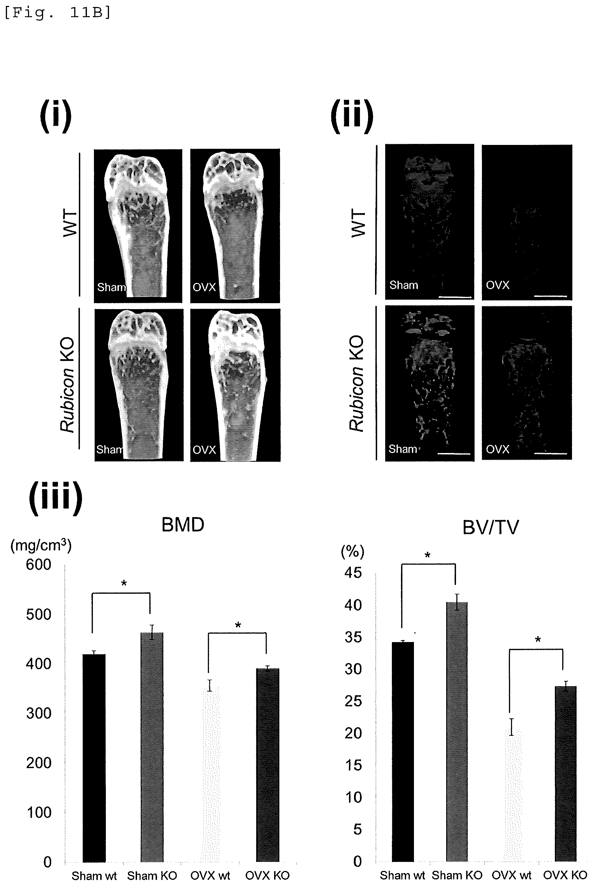

[Fig. 11B] Fig. 11B shows a result of evaluating bone mineral density and bone volume/bone tissue volume fraction in a Rubicon knockout osteoporotic model. (i) is CT images of the entire femurs, (ii) is CT images of trabeculae, and (iii) is graph showing bone mineral density and bone volume/bone tissue volume fraction.

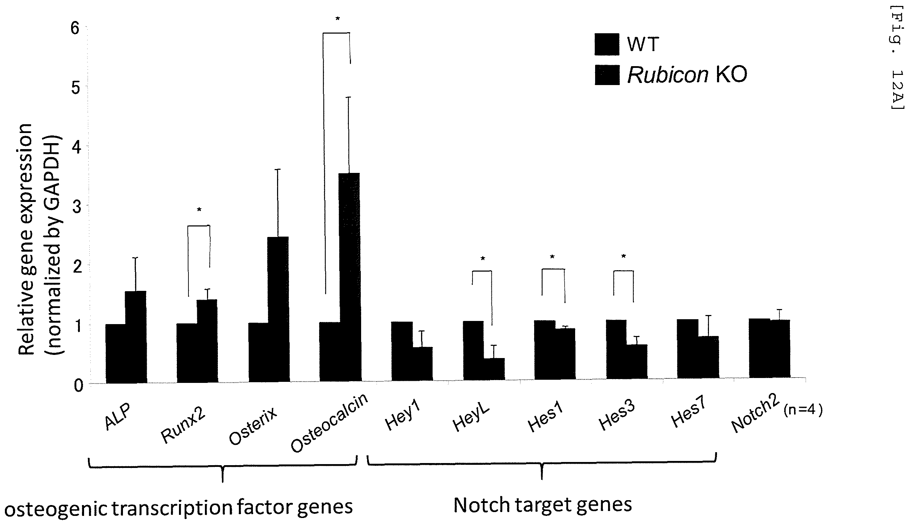

[Fig. 12A] Fig. 12A is a graph showing a result of detecting expression of mRNA in the transcription factor downstream of Notch and the osteogenic transcription factor for evaluating the relationship between autophagy and Notch signal pathway in osteoblasts of Rubicon knockout mice.

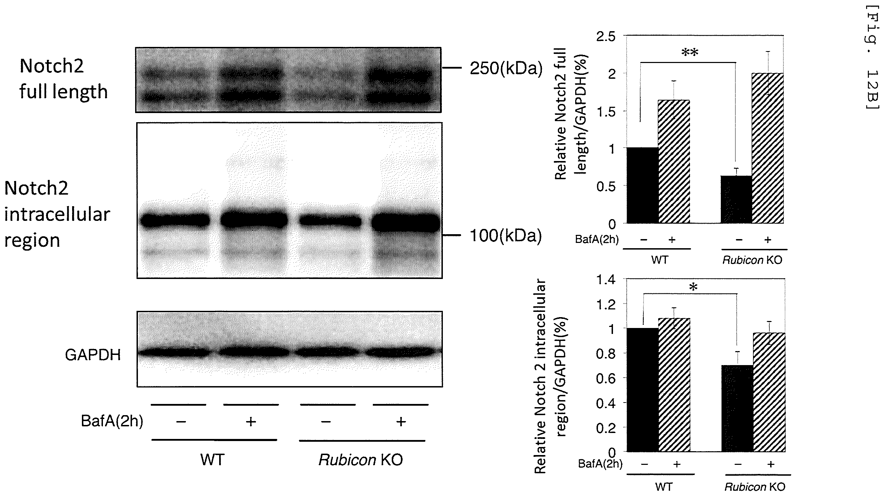

[Fig. 12B] Fig. 12B is electrophoresis images and graphs showing a result of detecting degeneration of Notch in osteoblasts of Rubicon knockout mice.

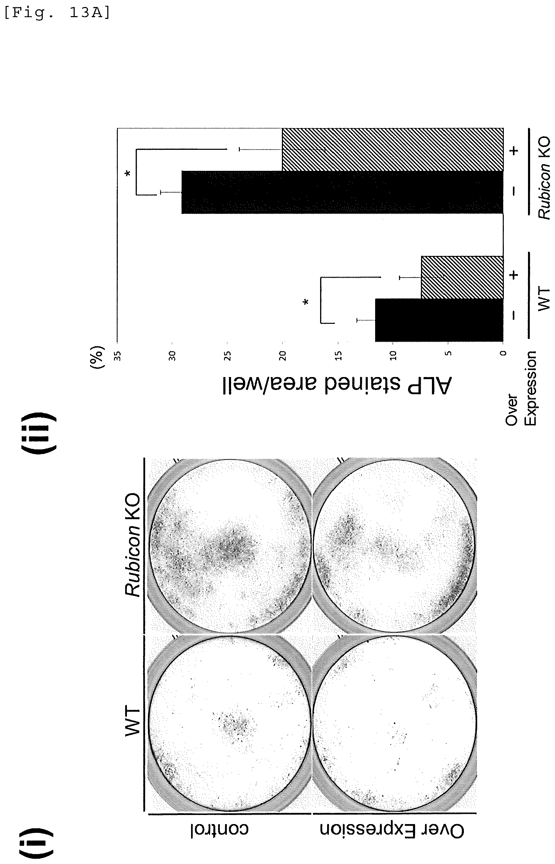

[Fig. 13A] Fig. 13A shows a result of overexpressing NICD in osteoblasts of Rubicon knockout mice and evaluating differentiation potential of the NICD through ALP staining. (i) is ALP-stained images, and (ii) is a graph showing proportions of ALP-stained areas in well.

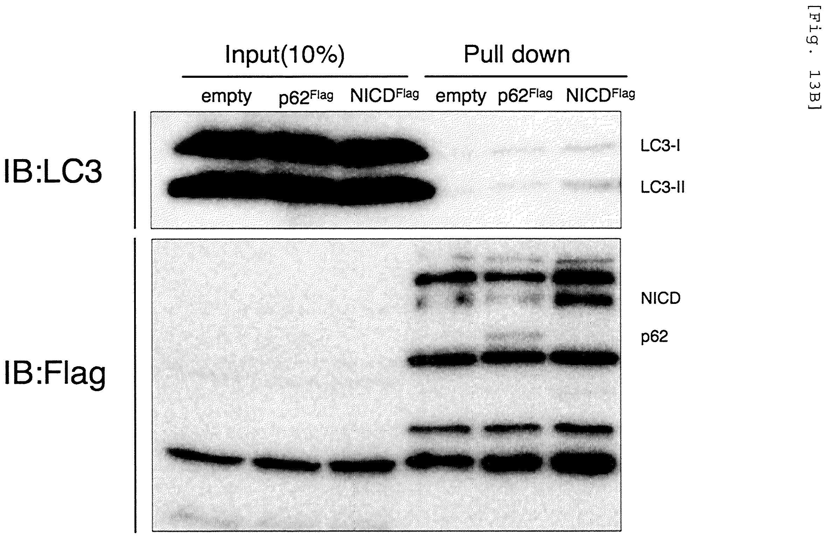

[Fig. 13B] Fig. 13B is pictures showing a result of detecting binding of NICD and LC3 by the immunoprecipitation method.

### [Description of Embodiments]

### <Composition for suppressing aging and the like>

The present invention provides a composition for suppression of aging, prevention, amelioration, or treatment of an age-related disease or symptom, or extension of lifespan in a subject, the composition comprising a molecule that suppresses functions of a Rubicon gene as an active ingredient.

"Rubicon" in the present invention is a negative regulator of autophagy, which inhibits autophagosome-lysosome fusion process (NPLs 6, 7). Rubicon in the present invention may be derived from any living organism (see Fig. 5A). For example, in the case where the composition of the present invention is for humans, Rubicon of the human is targeted. A typical amino acid sequence of the Rubicon protein of the human is shown in SEQ ID NO: 2, and a typical nucleic acid sequence of the cDNA encoding the protein is shown in SEQ ID NO: 1 (database registration No: NM_014687.3). In addition, typical amino acid sequences of the respective Rubicon proteins of the corresponding mouse, chicken, zebrafish, fruit fly, and nematode are shown in SEQ ID NOs: 3 to 7 in this order.

Note that in the sequence of Rubicon, individual differences and mutations may occur. Hence, the Rubicon protein of the present invention includes functionally equivalent proteins formed of amino acid sequences in which one or a plurality of amino acids are substituted, deleted, inserted, or added in the amino acid sequence described in any of SEQ ID NOs: 2 to 7. Here, the "one or a plurality" is normally within 20 amino acids (for example, within 10 amino acids, within 5 amino acids, within 3 amino acids, or 1 amino acid). In addition, "functionally equivalent" means that the protein has the function of negatively regulating autophagy (NPLs 6, 7) .

In addition, in comparison (Max score) using BLASTP 2.8.0+ (Altschul S.F. et al., Nucleic Acids Res. 25:3389-3402 (1997), Altschul S.F. et al., FEBS J. 272:5101-5109 (2005)) in default settings, the identity (proportion of the same amino acids) and the similarity (proportion of similar amino acids) of Rubicons between different species are respectively 85% and 89% (Gaps 2%) between mouse and human, respectively 73% and 81% (Gaps 2%) between human and chicken, respectively 56% and 66% (Gaps 10%) between chicken and zebrafish, respectively 39% and 57% (Gaps 6%) between zebrafish and fruit fly, and respectively 29% and 47% (Gaps 6%) between fruit fly and nematode. Hence, it is considered that amino acid sequences of Rubicon in species other than the above-described species normally have identity of 25% or more (for example, 30% or more, 35% or more, 40% or more, 45% or more, 50% or more, 55% or more, 60% or more, 65% or more, 70% or more, 75% or more, 80% or more, 85% or more, 90% or more, or 95% or more) and similarity of 45% or more (for example, 50% or more, 55% or more, 60% or more, 65% or more, 70% or more, 75% or more, 80% or more, 85% or more, 90% or more, or 95% or more) with respect to the amino acid sequence of Rubicon in any of the above-described species. Hence, the Rubicon protein of the present invention includes functionally equivalent proteins having amino acid sequences identical or similar to the amino acid sequence described in any of SEQ ID NOs: 2 to 7. Here, "functionally equivalent" means that the protein has the function of negatively regulating autophagy (NPLs 6, 7) .

The "aging" in the present invention means a change that occurs in an individual of a living organism along with age. It is possible for individuals to develop various diseases and symptoms with aging. The "age-related disease or symptom" targeted in the present invention is not particularly limited as long as the age-related disease or symptom is caused by the negative regulation of autophagy by Rubicon. The age-related disease or symptom includes, for example, metabolic bone diseases such as osteoporosis, neurodegenerations such as Parkinson's disease, Alzheimer's disease, polyglutamine disease (for example, Huntington's disease, spinocerebellar ataxia), amyotrophic lateral sclerosis (ALS), organ fibrosis (for example, fibrosis of kidney, fibrosis of myocardium), decrease in locomotor function (for example, muscle weakness, endurance weakness, extension of reaction time, decrease in locomotion speed, decrease in motor skill, decrease in deep sensation, decrease in balance ability), decrease in cognitive functions (for example, memory impairment, aphasia, apraxia, agnosia, executive function disorders), decrease in bone mass, bone mineral density and bone volume/bone tissue volume fraction, and change in bone morphology, eye degeneration, decrease in stress resistance, and the like, but is not limited to these. In addition, the "lifespan" in the present invention means a time from when a living organism is born to when the living organism dies. Although lifetime during which a human can maintain the life with his/her own mentality and physicality and live an independent living without depending on daily or continuous medical treatments or cares is particularly referred to as "healthspan", the "extension of lifespan" in the present invention includes an extension of lifespan achieved by an extension of healthspan.

In fact, although it was confirmed that the lifespan was extended by suppressing the functions of the Rubicon gene in Examples, besides this, the effects of suppressing many age-related diseases and symptoms were also confirmed, such as amelioration of decrease in bone mass, bone mineral density and bone volume/bone tissue volume fraction, and change in bone morphology, which would cause metabolic bone diseases, reduction of aggregative proteins, which would cause neurodegenerations, reduction of eye degeneration, amelioration of decrease in locomotor function, improvement in stress resistances, reduction of organ fibrogenesis, and the like.

As to the "suppression of functions of a gene" in the present invention, the mechanism may be suppression of the expression of the gene (for example, suppression of transcription, suppression of translation) or suppression of the activity of a translation product (protein) of the gene.

The "molecule that suppresses functions of a Rubicon gene" includes, for example, an RNA binding to a transcription product of the Rubicon gene or a DNA encoding the RNA, an RNA or DNA binding to a Rubicon protein, an antibody or low-molecular-weight compound binding to the Rubicon protein, a site-specific nuclease for editing the nucleic acid sequence of the Rubicon gene or DNA of an expression regulating region thereof or the RNA sequence of a transcription product of the Rubicon gene, and the like.

One aspect of the "RNA binding to a transcription product of the Rubicon gene or a DNA encoding the RNA" in the present invention is a dsRNA (double-stranded RNA) complementary to a transcription product of a gene encoding the Rubicon protein or a DNA encoding the dsRNA.

The dsRNA used in the present invention is preferably an siRNA, an shRNA (short hairpin RNA), or an miRNA. The siRNA is a double-stranded RNA formed of a short chain within a range that does not exhibit toxicity in a cell. In addition, the shRNA means a single-stranded RNA in which a sense RNA and an antisense RNA form a spacer sequence in which a hydrogen bond is generated between the sense RNA and the antisense RNA in a cell or the like to cause the spacer sequence to form a hairpin structure and which can be formed into an siRNA by cleavage of the hairpin structure in the cell. Although the miRNA means the form of a single-stranded RNA in general, the form of a double-stranded RNA can be employed as a specific embodiment.

For the dsRNA, the chain length is not particularly limited as long as the dsRNA can suppress the expression of a target gene and does not exhibit toxicity. However, the chain length is generally 15 to 50 base pairs, preferably 15 to 35 base pairs, and further preferably 20 to 25 base pairs. Increasing the chain length makes it possible to enhance the affinity with an mRNA to reduce off-target effects.

The dsRNA or the DNA encoding the dsRNA does not have to be completely identical to the nucleic acid sequence of a target gene, but has a sequence identity of at least 70% or more, preferably 80% or more, and further preferably 90% or more (for example, 95%, 96%, 97%, 98%, 99% or more) .

The dsRNA can be prepared by chemically synthesizing the chains, for example, based on the sequence information of the Rubicon gene (for example, SEQ ID NO: 1). The DNA encoding the dsRNA includes an antisense DNA obtained by encoding an antisense RNA corresponding to any region of a transcription product (mRNA) of the target gene and a sense DNA obtained by encoding a sense RNA of any region of the mRNA, and can express the antisense RNA and the sense RNA with the antisense DNA and the sense DNA, respectively. It is also possible to fabricate a dsRNA with these antisense RNA and sense RNA.

The configuration of the case where the expression system of a dsRNA is held in a vector or the like includes a case where an antisense RNA and a sense RNA are expressed from the same vector and a case where an antisense RNA and a sense RNA are expressed from different vectors. In the configuration in which an antisense RNA and a sense RNA are expressed from the same vector, for example, an antisense RNA-expressing cassette and a sense RNA-expressing cassette are constructed by binding promoters capable of expressing short RNAs such as pol III or the like upstream of an antisense DNA and a sense DNA, and these cassettes are inserted into a vector in the same direction or the opposite directions.

In addition, it is also possible to configure an expression system in which an antisense DNA and a sense DNA are reversely arranged to face on different chains. This configuration includes a double-stranded DNA in which an antisense RNA-coding strand and a sense RNA-coding strand are paired, and include promoters facing each other on the opposite sides of the double-stranded DNA such that the antisense RNA and the sense RNA can be expressed from the respective chains. In this case, it is preferable to include terminators at the 3' terminals of the respective chains (antisense RNA-coding strand and sense RNA-coding strand) in order to avoid addition of extra sequences downstream of the sense RNA and the antisense RNA. In addition, in this expression system of palindrome style, the types of the two promoters are preferably different.

In addition, in the configuration in which an antisense RNA and a sense RNA are expressed from different vectors, for example, an antisense RNA-expressing cassette and a sense RNA-expressing cassette are constructed by binding promoters capable of expressing short RNAs such as pol III or the like upstream of an antisense DNA and a sense DNA, and these cassettes are made to be held by different vectors.

Another aspect of the "RNA binding to a transcription product of the Rubicon gene or a DNA encoding the RNA" in the present invention includes an antisense RNA complementary to a transcription product of the Rubicon gene or a DNA encoding the RNA (antisense DNA).

Antisense RNA or antisense DNA inhibits the process of transcription, splicing, or translation to suppress the expression of a target gene (Hirashima and Inoue, "Shin-Seikagaku Zikken Kouza 2 - Kakusan IV - Idenshi No Hukusei To Hatsugen", edited by The Japanese Biochemical Society, Tokyo Kagaku Dojin Co Ltd, 319-347 (1993)), the antisense RNA or the antisense DNA used in the present invention may suppress the expression of a target gene through any action.

The sequence of the antisense RNA or the antisense DNA is preferably a sequence complementary to a transcription product of Rubicon, but does not have to be completely complementary as long as the antisense RNA or the antisense DNA can effectively inhibit the expression of the gene. The antisense RNA or the antisense DNA has a complementarity of preferably 90% or more (for example, 95%, 96%, 97%, 98%, 99% or more) to a transcription product of the target gene. The chain length of the antisense RNA or the antisense DNA is not particularly limited as long as the antisense RNA or the antisense DNA can suppress the expression of the target gene and does not exhibit toxicity. The length of the antisense RNA or the antisense DNA is generally 10 to 50 bases, and preferably 12 to 30 bases. Although the production cost increases, increasing the chain length makes it possible to enhance the affinity with an mRNA to reduce off-target effects.

The antisense RNA or the antisense DNA can be prepared through chemical synthesis, for example, based on the sequence information of the Rubicon gene.

Note that still another aspect of the "RNA binding to a transcription product of the Rubicon gene or a DNA encoding the RNA" in the present invention includes an RNA having a ribozyme activity of specifically cleaving a transcription product of the Rubicon gene or a DNA encoding the RNA. Ribozymes include those having sizes of 400 nucleotides or more such as group I intron-type and M1RNA contained in RNaseP, but also include those having active domains of around 40 nucleotides, which are called hammerhead-type and hairpin-type (Makoto Koizumi and Eiko Otsuka, Tanpakusitsu Kaksusan Kouso, 1990, vol. 35, p 2191) . In the present invention, any ribozyme having any form can be used.

The "RNA or DNA binding to a Rubicon protein" in the present invention is typically a nucleic acid aptamer. As the nucleic acid aptamer, DNA is preferable from the viewpoint that DNA has a high stability in living organisms.

The nucleic acid aptamer may contain, as a structural feature, at least one loop structure, a primary structure rich in deoxyguanosine (containing guanosine, guanosine analogue), or deoxyguanosine may have a tetramer cluster structure (so-called "G-quartet structure"). In addition, the nucleic acid aptamer may be either of a double-stranded nucleic acid and a single-stranded nucleic acid, but is preferably a single-stranded nucleic acid.

The length of the nucleic acid aptamer is not particularly limited as long as the number of bases of the nucleic acid aptamer allows the nucleic acid aptamer to specifically bind to the Rubicon protein, but is generally 10 to 100 bases, preferably 15 to 70 bases, and more preferably 20 to 50 bases. Although the production cost increases, a nucleic acid aptamer having a greater chain length can also be used.

The nucleic acid aptamer used in the present invention can be produced by a person skilled in the art selecting a publicly-known method as appropriate. The publicly-known method includes, for example, in vitro selection methods (SELEX method) (Tuerk C. & Gold L., Science. 3; 249(4968): 505-510 (1990), Green R. et al., Methods Compan Methods Enzymol. 2: 75-86 (1991), Gold L. et al., Annu Rev Biochem 64: 763-97 (1995), Uphoff K.W. et al., Curr. Opin. Struct. Biol. 6: 281-288 (1996)).

In the nucleic acid molecule used as an active ingredient of the composition of the present invention, a modified nucleic acid may be used partially or entirely as appropriate from the viewpoint of improving the stability in a living organism, enhancing the affinity with a target (mRNA or protein), or suppressing off-target effects. For example, in an antisense nucleic acid, a gapmer using modified nucleic acids only on the opposite ends can be used as necessary. In a double-stranded RNA as well, for example, a heteroduplex RNA with a gapmer as one chain can be used.

The chemical modification of the nucleic acid includes, for example, modification of a phosphoric acid moiety, modification of a sugar moiety, and modification of a base moiety. Such modification includes, for example, 5-methylation, 5-fluorination, 5-bromination, 5-iodination, and N4-methylation of cytosine; 5-demethylation, 5-fluorination, 5-bromination, and 5-iodination of thymidine; N6-methylation and 8-bromination of adenine; and N2-methylation, 8-bromination, phosphorothioation, methylphosphonation, methylthiophosphonation, chiral-methylphosphonation, phosphorodithioation, phosphoroamidation, 2'-O-methylation, 2'-MOE modification, 2'-AP modification, and 2'-fluorination of guanine, and the like. In addition, modified nucleic acids such as hexitol nucleic acid (HNA), cyclohexene nucleic acid (CeNA), peptide nucleic acid (PNA), glycol nucleic acid (GNA), threose nucleic acid (TNA), morpholino nucleic acid, tricyclo-DNA (tcDNA), 2'-O-methylated nucleic acid, 2'-MOE(2'-O-methoxyethylated) nucleic acid, 2'-AP(2'-O-aminopropylated) nucleic acid, 2'-fluorinated nucleic acid, 2'-F-arabinonucleic acid (2'-F-ANA), and BNA (bridged nucleic acid) can also be used.

BNA includes, for example, α-L-methyleneoxy(4'-CH2-O-2')BNA or β-D-methyleneoxy(4'-CH2-O-2')BNA, also called LNA (locked nucleic acid (trade mark), 2',4'-BNA), ethyleneoxy(4'-(CH2)2-O-2')BNA, also called ENA, β-D-thio(4'-CH2-S-2')BNA, aminooxy(4'-CH2-O-N(R3)-2')BNA, oxyamino(4'-CH2-N(R3)-O-2')BNA, also called 2',4' -BNANC, 2',4' -BNACOC, 3'amino-2',4'-BNA, and the like.

For the chemical modifications of nucleic acids, see, for example, documents (for example, Satoshi Obika et al., The Journal of the Japanese Pharmacological Society, 148, 100 to 104 (2016), Takao Inoue, Drug Delivery System 31-1, 10 to 22 (2016)) and patent publications (for example, Japanese Patent Application Publication No. Hei 10-304889, International Publication No. 2005/021570, Japanese Patent Application Publication No. Hei 10-195098, Published Japanese Translation of PCT International Application No. 2002-521310, International Publication No. 2007/143315, International Publication No. 2008/043753, International Publication No. 2008/029619, and International Publication No. 2008/049085) .

The "antibody binding to a Rubicon protein" used in the present invention may be a polyclonal antibody or a monoclonal antibody, or may be a functional fragment of an antibody. In addition, the "antibody" includes all the classes and subclasses of immunoglobulins. The "functional fragment" of an antibody means a part (partial fragment) of the antibody that specifically recognizes the Rubicon protein. Specifically, the "functional fragment" of an antibody includes Fab, Fab', F(ab')2, variable region fragments (Fv), disulfide bonded Fv, single chain Fv (scFv), sc(Fv)2, diabodies, polyspecific antibody, and polymers of these, and the like.

When the antibody is a polyclonal antibody, the antibody can be obtained by immunizing an immune animal with an antigen (a target protein, a partial peptide of the target protein, or a cell expressing these, or the like), and purifying the antiserum by a conventional approach (for example, salting-out, centrifugation, dialysis, column chromatography, or the like). In addition, the monoclonal antibody can be fabricated by the hybridoma method (Kohler & Milstein, Nature, 256:495 (1975)) or the recombinant DNA method (for example, P.J.Delves, Antibody Production: Essential Techniques, (1997), WILEY, P.Shepherd & C.Dean Monoclonal Antibodies, OXFORD UNIVERSITY PRESS (2000), Vandamme A.M. et al., Eur.J.Biochem. 192:767-775 (1990)).

The antibody binding to a Rubicon protein includes chimeric antibodies, humanized antibodies, and human antibodies. In the case where the antibody is administered to a human as a therapeutic drug, a chimeric antibody, a humanized antibody, or a human antibody is desirable from the viewpoint of reducing side effects.

The "chimeric antibody" is an antibody obtained by binding a variable region of an antibody of a certain species and a constant region of an antibody of a different species. A chimeric antibody can be obtained, for example, by immunizing a mice with an antigen, cleaving an antibody variable domain (variable region), which binds to the antigen, from the gene of the mouse monoclonal antibody, binding the antibody variable domain with an antibody constant domain (constant region) gene derived from a human bone marrow, incorporating the resultant into an expression vector, and introducing the expression vector into a host to produce the chimeric antibody (for example, Japanese Patent Application Publication No. Hei 8-280387, United States Patent No. 4816397, United States Patent No. 4816567, ad United States Patent No. 5807715) .

The "humanized antibody" is an antibody obtained by grafting a gene sequence of an antigen-binding site (CDR) of a non-human-derived antibody into a human antibody gene (CDR grafting), and its production method is publicly known(see, for example, EP 239400, EP 125023, WO 90/07861, and WO 96/02576).

The "human antibody" is an antibody whose regions are all derived from a human. In the fabrication of a human antibody, it is possible to use a transgenic animal (for example, a mouse) which is capable of producing repertoires of the human antibody through immunization. The method for fabricating a human antibody is publicly known (for example, Jakobovits, A. et al., Nature 362, 255-258 (1993, LONBERG, N. & HUSZAR, D., INTERN. REV. IMMUNOL. , 13, 65-93 (1995), Marks J.D. et al., J Mol Biol. 5; 222 (3): 581-97 (1991), Mendez M.J. et al., Nat Genet. 15(2): 146-156 (1997), Tomizuka K. et al., Proc Natl Acad Sci U S A. Jan 18; 97(2): 722-727 (2000), Japanese Patent Application Publication No. Hei 10-146194, Japanese Patent Application Publication No. Hei 10-155492, Japanese Patent No. 2938569, Japanese Patent Application Publication No. Hei 11-206387, Published Japanese Translation of PCT International Application No. Hei 8-509612, Published Japanese Translation of PCT International Application No. Hei 11-505107).

The "site-specific nuclease for editing the nucleic acid sequence of the Rubicon gene or DNA of an expression regulating region thereof or an RNA sequence of a transcription product of the Rubicon gene" used in the present invention is not limited as long as the site-specific nuclease can be used to site-specifically edit a nucleic acid. However, the CRISPR-Cas systems having Cas proteins as constituent elements are preferable. As the CRISPR-Cas systems, for example, CRISPR-Cas9, CRISPR-Cpf1 (Cas12a), CRISPR-Cas12b, CRISPR-CasX (Cas12e), CRISPR-Cas14, and the like can be used, and besides, as the site-specific nuclease, an artificial nuclease such as TALENs, ZFNs, or PPR (form fused with a base-modifying enzyme) can also be used.

The "low-molecular-weight compound binding to the Rubicon protein" used in the present invention may be a publicly-known compound or a compound identified by an evaluation method or a screening method of the present invention, which will be described later.

The composition of the present invention may be in the form of a pharmaceutical composition, foods and beverages (including animal feeds), or a reagent used for research purposes (for example, in vitro and in vivo experiments).

The pharmaceutical composition may be pharmaceutically formulated using publicly-known pharmaceutical methods. For example, the composition may be formulated into injections, suppositories, capsules, tablets, pills, liquids, powders, granules, subtle granules, film coating agents, pellets, troches, sublingual tablets, masticatory agents, buccal preparations, pastes, syrups, suspending agents, elixirs, emulsions, liniments, ointments, plasters, gel patchs, transdermal therapeutic systems (TTSs), lotions, inhalants, aerosols, or the like to be parenterally or orally used. In these pharmaceutical formulations, pharmacologically acceptable carriers, specifically, sterile water, saline, vegetable oils, solvents, bases, emulsifiers, suspending agents, surfactants, stabilizers, pH adjusters, flavor agents, fragrances, excipients, vehicles, preservatives, binders, diluents, tonicity agents, soothing agents, expanders, collapsing agents, buffers, coating agents, lubricants, coloring agents, sweetening agents, thickening agents, flavoring agents, solubilizers, or other additives, or the like may be incorporated as appropriate. In addition, the pharmaceutical composition may be prepared in the form of liposome delivery system or the like.

The mode of administration of the pharmaceutical composition of the present invention is not particularly limited, and includes, for example, intravenous administration, intraarterial administration, intraperitoneal administration, subcutaneous administration, intradermal administration, tracheobronchial administration, rectal administration, intramuscular administration, administration by transfusion, direct administration (for example, topical administration into affected sites), and the like.

The pharmaceutical composition of the present invention may bind to an auxiliary portion that promotes medically useful properties. Typical useful properties include, for example, promoting the delivery of the compound to the target region (for example, an affected site), maintaining the therapeutic concentration of the compound in the target region, modifying the pharmacokinetic properties and the pharmacodynamic properties of the compound, improving the therapeutic index or safety profile of the compound, and the like.

The molecule for delivery to a target region includes, for example, a brain barrier permeable substance in the case where the target region is the brain. As the brain barrier permeable substance, for example, anti-transferrin receptor antibodies (for example, International Publication No. 2016/208695) and a glycoprotein formed of 29 amino acids derived from rabies virus (see Kumar P. et al., Nature. 2007 5;448 (7149) :39-43 (2007)) are known. Besides, publicly-known molecules for delivery can be used depending on target regions such as the kidney and the liver.

In addition, the pharmaceutical composition of the present invention may be used together with another composition used for suppression of aging, prevention, amelioration, or treatment of an age-related disease or symptom, or extension of lifespan.

In the case where the composition of the present invention is used for foods and beverages, the foods and beverages may be, for example, health foods, functional foods, foods for specified health use, nutrition supplement foods, foods for sick people, food additives, or animal feeds. The foods and beverages can be taken as the compositions described above or can be taken as various foods and beverages. Specific examples of the foods and beverages include products containing oil such as food oils, dressings, mayonnaises, and margarines; liquid foods such as soups, milk beverages, soft drinks, tea beverages, alcohol beverages, nutritious supplement drinks, jelly beverages, and functional beverages; carbohydrate-containing foods such as cereals, noodles, and breads; livestock processed foods such as hams and sausages; marine processed foods such as boiled fish pastes, dried fishes, and salted fish guts; vegetable processed foods such as pickles; semisolid foods such as jellies and yogurts; fermentation foods such as misos and fermented beverages; various confectioneries such as pastries, Japanese sweets, candies, chewing gums, gummy candies, frozen desserts, and ice desserts; retort pouch foods such as curries, foods dressed with thick starchy sauces, and Chinese soups; instant foods such as instant soups and instant miso soups, microwave foods, and the like. Moreover, the foods and beverages include health foods and beverages prepared in the form of powders, granules, tablets, capsules, liquids, pastes, and jellies.

The foods and beverages can be produced in accordance with production technique known in the Technical Field. To the foods and beverages, one or more components effective in suppression of aging, prevention, amelioration, or treatment of an age-related disease or symptom, or extension of lifespan may be added. In addition, the foods and beverages may be combined with a component that exhibits another function or with another functional food to obtain multi-functional foods and beverages.

The composition of the present invention can be used for animals including humans. Animals other than humans are not particularly limited, and the composition may be used for various livestock, poultry, pets, experimental animals, and the like. The animals are preferably vertebrates, include, for example, mammals such as pig, cow, horse, sheep, goat, dog, cat, rabbit, hamster, mouse, rat, and monkey and birds such as chicken, duck, ostrich, and domestic duck, but are not limited to these. For the purpose of researches, the animals may be various experimental experimental living organisms.

In the case of administering or taking the composition of the present invention, the amount of administration or intake can be selected as appropriate depending on the type of the compositions (pharmaceutical products, foods and beverages, or the like), the types, ages, body weights, symptoms, health statuses, and the like of subjects, and the like. In addition, the dosage (in terms of the active ingredient) of the composition of the present invention may vary depending on the type of the active ingredient, but for example, generally 0.1 µg to 10 mg/kg body weight in the case where the active ingredient is an RNA or a DNA, and generally 1 to 10 mg/kg body weight in the case where the active ingredient is an antibody. In addition, the amount of administration or intake per day is also not particularly limited, and may be selected as appropriate in consideration of various factors as described above. In the case where the active ingredient is a DNA encoding the RNA binding to a transcription product of the Rubicon gene, an expression construct may be fabricated and administered such that an appropriate amount of the RNA is expressed.

The present invention also provides a method for suppression of aging, prevention, amelioration, or treatment of an age-related disease or symptom, or extension of lifespan in a subject, including administering the composition of the present invention to the subject or allowing the subject to take the composition of the present invention as described above.

The composition (a pharmaceutical product, foods and beverages, a reagent, or the like) of the present invention or the instruction of the composition may be attached with an indication that the composition is used for suppression of aging, prevention, amelioration, or treatment of an age-related disease or symptom, or extension of lifespan. Here, a "product or an instruction is attached with an indication" means that an indication is attached to the body of the product, a container, a package, or the like, or an indication is attached to an instruction, a package insert, an advertisement, or another printed matter that discloses information on the product. The above-described indication may contain information on the mechanism of action of the composition of the present invention. Such mechanism includes, for example, promotion of autophagy owing to the suppression of functions of the Rubicon gene, and the like.

### < Evaluation of Probability That Test Compound Has Activity of Suppressing Aging, and the like>

### - Method using binding to Rubicon protein as index -

As shown in Examples described below, it was revealed that suppressing the functions of the Rubicon gene brought amelioration of various age-related diseases and symptoms, such as amelioration of decrease in bone mass, bone mineral density and bone volume/bone tissue volume fraction, and change in bone morphology, which would cause metabolic bone diseases, reduction of aggregative proteins, which would cause neurodegenerations, reduction of eye degeneration, amelioration of decrease in locomotor function, improvement in stress resistances, reduction of organ fibrogenesis, and the like, and thus extended the lifespan. Hence, as one step for developing the composition for suppression of aging, prevention, amelioration, or treatment of an age-related disease or symptom, or extension of lifespan, it is effective to identify a compound that binds to the Rubicon protein. Specifically, the present invention provides a method for evaluating a probability that a test compound has an activity of suppressing aging, an activity of preventing, ameliorating, or treating an age-related disease or symptom, or an activity of extending lifespan in a subject, the method comprising the steps of: (a) bringing the test compound and a Rubicon protein into contact; and (b) detecting binding of the test compound and the Rubicon protein, as a first aspect of the evaluation method of the present invention.

The "test compound" as a target for the evaluation method of the present invention is not particularly limited, and includes, for example, expression products of gene libraries, synthesized low-molecular-weight compound libraries, peptide libraries, polynucleotide libraries, antibodies, bacteria releasing substances, extracts and culture supernatants of cells (microorganisms, plant cells, and animal cells), purified or partially purified polypeptides, extract sampled from marine living organisms, plants, or animals, and the like. The test compound may be a test compound synthesized based on an in silico design based on the conformation of the Rubicon protein.

The "Rubicon protein" used in the evaluation method of the present invention includes, for example, a protein made of an amino acid sequence described in SEQ ID NO: 2 and partial peptides thereof in the case of a human protein. As the Rubicon protein, besides the above-described natural protein or a partial peptide thereof, an engineered product or a modification product of the protein can be used as necessary. For example, in order to facilitate the detection or purification, a fusion protein with another protein (for example, an enzyme such as alkaline phosphatase (SEAP) and β-galactosidase, a fluorescent protein such as the green fluorescent protein (GFP), a tag such as glutathione-S-transferase (GST)) can be used. The "contact" between the test compound and the Rubicon protein can be achieved by adding the test compound into the evaluation system, or the like. For "detecting binding of the test compound and the Rubicon protein", a publicly-known method can be employed as appropriate. Such a method includes, for example, a method for identifying a compound that binds to the Rubicon protein by bringing these test compounds into contact with the fixed Rubicon protein. As means for detecting the binding of the test compound and the Rubicon protein, various publicly-known means can be used, but an example of preferable means includes a biosensor utilizing the surface plasmon resonance phenomenon.

In the case where the test compounds are a synthesized low-molecular-weight compound library, for example, the high throughput method utilizing the combinatorial chemistry technology (Wrighton N.C. et al., Science. 26; 273 (5274) 458-464 (1996), Verdine, G.L. Nature 384 11-13 (1996), Hogan J.C., Jr Directed combinatorial chemistry. Nature. 384:17-19 (1996)) can be utilized. In the case where the test compounds are a polynucleotide library, for example, the above-described in vitro selection method can be utilized, and in the case where the test compounds are a gene library, for example, a yeast two-hybrid system in the Rubicon protein is expressed as a bait protein to be used.

In a case where the test compound binds to the Rubicon protein As a result of the above-described method, it is evaluated that there is a probability that the test compound has an activity of suppressing aging, an activity of preventing, ameliorating, or treating an age-related disease or symptom, or an activity of extending lifespan.

### - Method Using Reduction in Amount of Rubicon Gene Expressed as Index -

As shown in Examples described below, it was suggested that the expression of the Rubicon gene is age-dependently upregulated, which causes the developments of various age-related diseases and symptoms. Hence, it is effective to identify a compound that reduces the amount of the Rubicon gene expressed as one step for developing a composition for suppression of aging, prevention, amelioration, or treatment of an age-related disease or symptom, or extension of lifespan. Specifically, the present invention provides a method for evaluating a probability that a test compound has an activity of suppressing aging, an activity of preventing, ameliorating, or treating an age-related disease or symptom, or an activity of extending lifespan in a subject, the method comprising the steps of: (a) bringing a test compound into contact with a cell that expresses a Rubicon gene; and (b) detecting expression of the Rubicon gene in the cell, as a second aspect of the evaluation method of the present invention.

The "test compound" used in the second aspect is the same as that in the first aspect. For the "Rubicon gene" to be detected, the living organism from which the Rubicon gene is derived is not particularly limited; however the Rubicon gene of humans is preferable in the development of a composition for humans. As the human gene, for example, the gene encoding a protein formed of an amino acid sequence described in SEQ ID NO: 2 (for example, a gene formed of a nucleic acid sequence described in SEQ ID NO: 1) may be detected. In addition, the "cell expressing the Rubicon gene" includes, for example, human cells such as RPE-1 cells, HEK293 cells, and HeLa cells, but is not limited to these. The "contact" of the test compound with the cell expressing the Rubicon gene may be achieved, for example, by adding the test compound into a culture solution of the cells, and the like. For "detecting expression of the Rubicon gene", a publicly-known method can be used. For "detecting expression of a gene", the gene may be detected at the transcription level (mRNA level) or may be detected at the translation level (protein level). The method for detecting a gene at the transcription level includes, for example, the RT-PCR, the northern blotting, the in situ hybridization, the dot blotting, the RNase protection assay, and the like. The method for detecting a gene at the translation level includes, for example, the western blotting method, the radioimmunoassay method, the chemiluminescent immunoassay method, the chemiluminescent enzyme immunoassay method, the enzyme immunoassay method, the immunoprecipitation method, the immunochromatography, the immunohistochemical staining method, the imaging cytometry, the flow cytometry, the latex agglutination method, the mass analysis method (MS), and the like.

In a case where the test compound reduces the expression of the Rubicon gene as a result of the above-described method compared with the case where the test compound is not brought into contact, it is evaluated that there is the probability that the test compound has an activity of suppressing aging, an activity of preventing, ameliorating, or treating an age-related disease or symptom, or an activity of extending lifespan.

In the case where the western blotting method is used, for example, reduction in the expression of the Rubicon gene can be evaluated by comparing the amount of the Rubicon protein detected in the presence of the test compound (for example, the band intensity derived from the Rubicon protein) and the amount (control value) of the Rubicon protein detected in the absence of the test compound. Specifically, in a case where the amount of the Rubicon protein in the presence of the test compound is lower than the amount of the Rubicon protein in the absence of the test compound (for example, in the case of 80% or less, 50% or less, 30% or less, or 10% or less of the control value), it can be evaluated that there is a probability that the test compound has an activity of suppressing aging, an activity of preventing, ameliorating, or treating an age-related disease or symptom, or an activity of extending lifespan. In the case of using a method other than the western blotting method in detection of the expression as well, the evaluation can be made using the amount of expression in the absence of the test compound as a control value.

### - Reporter System -

The evaluation on whether or not a test compound reduces the expression level of the Rubicon gene can also be conducted with a system using a reporter gene. Specifically, the present invention provides a method for evaluating a probability that a test compound has an activity of suppressing aging, an activity of preventing, ameliorating, or treating an age-related disease or symptom, or an activity of extending lifespan in a subject, the method comprising the steps of : (a) bringing the test compound into contact with a cell having a DNA in which a reporter gene is functionally bound to downstream of a promoter region of a Rubicon gene; and (b) detecting expression of the reporter gene in the cell, as a third aspect of the evaluation method of the present invention.

The "promoter region of a Rubicon gene" is a region that regulates transcription of the Rubicon gene, and means a region upstream of a coding region of the Rubicon gene to which a transcription factor can be bound. A person skilled in the art can obtain the "promoter region of a Rubicon gene", for example, through screening of a genomic DNA library using the nucleic acid sequence described in SEQ ID NO: 1 or a part thereof as a probe. In the case where a vector in which the reporter gene is bound downstream of an acquired genomic DNA is prepared and introduced into an appropriate cell, if expression of the reporter gene can be derived, the genomic DNA can be identified as the promoter region of the Rubicon gene.

The "reporter gene" is not particularly limited as long as expression of the reporter gene can be detected, and includes, for example, the CAT gene, the lacZ gene, the luciferase gene, the β-glucuronidase gene (GUS), and the GFP gene, which are generally used by a person skilled in the art.

Here, the condition that the reporter gene is "functionally bound" to downstream of the promoter region of the Rubicon gene means that the promoter region of the Rubicon gene and the reporter gene are bound such that expression of the reporter gene is derived by the transcription factor binding to the promoter region of the Rubicon gene.

Note that the "Rubicon gene", the "test compound", and the "contact" are the same as those in the second aspect. The "cell" used in the third aspect may be a cell in which once the reporter system is introduced, expression of the reporter gene is derived by the transcription factor binding to the promoter region of the Rubicon gene, and includes, for example, the RPE-1 cell, the HEK293 cell, the HeLa cell, and the like, but is not limited to these.

The expression of the reporter gene can be detected by a method known by a person skilled in the art depending on the type of the reporter gene used. For example, in the case where the reporter gene is the CAT gene, it is possible to detect expression of the reporter gene by detecting acetylation of chloramphenicol attributable to the gene product. It is possible to detect expression of the reporter gene, by detecting color emission of a dye compound due to the catalytic action of the gene expression product in the case where the reporter gene is the lacZ gene, or by detecting chemiluminescence due to the catalytic action of the gene expression product in the case where the reporter gene is the luciferase gene, or by detecting light emission of glucuronic acid or color emission of 5-bromo-4-chloro-3-indolyl-β-glucuronide (X-Gluc) due to the catalytic action of the gene expression product in the case where the reporter gene is the GUS, or by detecting fluorescence by the GFP protein in the case where the reporter gene is the GFP gene.

In a case where the test compound reduces the expression of the reporter gene as a result of the above-described method compared with the case where the test compound is not brought into contact, it is evaluated that there is the probability that the test compound has an activity of suppressing aging, an activity of preventing, ameliorating, or treating an age-related disease or symptom, or an activity of extending lifespan.

In the case where the luciferase gene is used as the reporter gene, for example, reduction in the expression of the reporter gene can be evaluated by comparing the intensity of chemiluminescence detected in the presence of the test compound and the intensity (control value) of chemiluminescence detected in the absence of the test compound. Specifically, in a case where the light emission intensity in the presence of the test compound is lower than the light emission intensity in the absence of the test compound (for example, in the case of 80% or less, 50% or less, 30% or less, or 10% or less of the control value), it can be evaluated that there is a probability that the test compound has an activity of suppressing aging, an activity of preventing, ameliorating, or treating an age-related disease or symptom, or an activity of extending lifespan. In the case of using a reporter gene other than the luciferase gene in detection of the expression as well, the evaluation can be made using the amount of expression detected in the absence of the test compound as a control value.

Although the evaluation method of the present invention has been described so far, it is possible to screen a compound having an activity of suppressing aging, an activity of preventing, ameliorating, or treating an age-related disease or symptom, or an activity of extending lifespan by conducting the evaluation method of the present invention on a plurality of test compounds and selecting a compound using binding to the Rubicon protein, reduction in the expression of the Rubicon gene, or reduction in the expression of the reporter gene as an index. Hence, the present invention also provides such a screening method.

For the compound identified by the evaluation method or the screening method of the present invention, it is preferable to further evaluate the activity of promoting autophagy in cells and verify whether or not the compound actually has the activity in animal experiments and clinical trials. The animal experiments include, for example, amelioration of an age-related disease or symptom in a model animal having the disease or symptom (for example, amelioration of decrease in bone mass, bone mineral density and bone volume/bone tissue volume fraction, and change in bone morphology, which would cause metabolic bone diseases, reduction of aggregative protein, which would cause neurodegenerations, reduction of eye degeneration, amelioration of decrease in locomotor function, improvement in stress resistance, reduction of organ fibrogenesis, and the like), extension of lifespan, or the like, as shown in Examples described below.

### <Test for degree of aging and the like>

As shown in Examples described below, it was suggested that the expression of the Rubicon gene is age-dependently upregulated, which causes the developments of various age-related diseases and symptoms and shortening of lifespans . Hence, it is possible to test the degree of aging, the risk of developing an age-related disease or symptom, or the risk of shortening lifespan using the amount of expression of the Rubicon gene as an index. Specifically, the present invention provides a method for testing a degree of aging, a risk of developing an age-related disease or symptom, or a risk of shortening lifespan in a subject, the method comprising the step of: detecting an expression product of a Rubicon gene in a test sample, wherein in a case where an amount of the expression product of the Rubicon gene is higher than that of control, it is evaluated that the degree of aging, the risk of developing an age-related disease or symptom, or the risk of shortening lifespan is high. The test method of the present invention can also be expressed as a method for providing information for diagnosis conducted by a doctor or a veterinarian or a method for assisting diagnosis conducted by a doctor or a veterinarian.

The test method of the present invention can be used for animals including humans. Animals other than humans are not particularly limited, and the test method can be used for various livestock, poultry, pets, experimental animals, and the like. Specifically, the animals include mammals such as pig, cow, horse, sheep, goat, dog, cat, rabbit, hamster, mouse, rat, and monkey and birds such as chicken, duck, ostrich, and domestic duck, but are not limited to these. For the purpose of researches, the animals may be various experimental experimental living organisms.

The "test sample" in the test method of the present invention is a biological sample separated from a subject to be tested on the degree of aging, the risk of developing an age-related disease or symptom, or the risk of shortening lifespan. The biological sample is not particularly limited as long as the biological sample contains an expression product (transcription product and/or translation product) of the Rubicon gene, but includes, for example, cells of liver and kidney, and the like.

For the "detection of an expression product of the Rubicon gene ", a publicly-known method can be used. The "detection of an expression product of a gene" may be conducted on a transcription product (mRNA) or on a translation product (protein). The method for detecting a transcription product includes, for example, the RT-PCR, the northern blotting, the in situ hybridization, the dot blotting, the RNase protection assay, and the like. The method for detecting a translation product includes, for example, the western blotting method, the radioimmunoassay method, the chemiluminescent immunoassay method, the chemiluminescent enzyme immunoassay method, the enzyme immunoassay method, the immunoprecipitation method, the immunochromatography, the immunohistochemical staining method, the imaging cytometry, the flow cytometry, the latex agglutination method, the mass analysis method (MS), and the like.

In a case where the amount of the expression product of the Rubicon gene in the test sample is higher than that of the control as a result of detecting the expression product of the Rubicon gene as described above, it is evaluated that the subject has a high degree of aging, a high risk of developing an age-related disease or symptom, or a high risk of shortening lifespan. Here, the "risk of developing" includes not only the risk of development or not but also the risk of earlier development. As the control, the amount of the expression product of the Rubicon gene in a sample separated from an individual that is not aged or an individual that has not developed an age-related disease or symptom can be used, for example.

In addition, the present invention provides a composition for detecting an expression product of a Rubicon gene in the above-described method, the composition comprising the following (a) or (b):
(a) an oligonucleotide primer or oligonucleotide probe binding to a transcription product of the Rubicon gene, or
(b) an antibody binding to a Rubicon protein.

The oligonucleotide primer (hereinafter, referred to simply as "primer") may be designed such that transcription products other than the transcription product of the Rubicon gene is amplified as little as possible based on the nucleic acid sequence information (for example, SEQ ID NO: 1) of a cDNA encoding Rubicon. A person skilled in the art can conduct such a primer designing by a conventional method. The length of the primer is generally 15 to 50 bases, and preferably 15 to 30 bases, but may be longer depending on the method and purpose.

The oligonucleotide probe (hereinafter, referred to simply as "probe") may be designed such that the probe binds to transcription products other than the transcription product of the Rubicon gene as little as possible based on the nucleic acid sequence information (for example, SEQ ID NO: 1) of a cDNA encoding Rubicon. A person skilled in the art can conduct such a probe designing by a conventional method. The length of the probe is generally 15 to 200 bases, preferably 15 to 100 bases, and further preferably 15 to 50 bases, but may be longer depending on the method and purpose. In addition, the primer and the probe can be labeled to be used as appropriate.

The primer and the probe can be fabricated, for example, by using a commercially-available oligonucleotide synthesizer. The probe can also be fabricated as a double-stranded DNA fragment obtained through a restriction enzyme treatment or the like. In addition, the primer and the probe may be formed of only natural nucleotides (deoxyribonucleotide (DNA) and ribonucleotide (RNA)), but the above-described chemically modified nucleic acids may be used partially or entirely as necessary. In addition, the primer and the probe can be labeled to be used as appropriate.

As the antibody binding to the Rubicon protein, in the case of detecting the Rubicon protein by the direct method, the antibody to which a labeling substance is bound is generally used. On the other hand, in the case of detecting the Rubicon protein by the indirect method, the Rubicon protein can be detected by using a secondary antibody to which a labeling substance is bound, or the like, without binding a labeling substance to the antibody binding to the Rubicon protein. Here, the "secondary antibody" is an antibody that exhibits reactivity to the antibody binding to the Rubicon protein. For example, in the case where the antibody binding to the Rubicon protein is prepared as a mouse antibody, an anti-mouse IgG antibody can be used as the secondary antibody. Labeled secondary antibodies usable for antibodies derived from various biological species such as rabbit, goat, and mouse are commercially available, and it is possible to select an appropriate secondary antibody for use depending on the biological species from which the antibody binding to the Rubicon protein is derived. In place of secondary antibodies, it is also possible to use protein G or protein A to which a labeling substance is bound.

The labeling substance is not particularly limited as long as the labeling substance can be detected, but includes, for example, radioisotopes such as 1251, 1311, 3H, 14C, 32P, 33P, and 35S, enzymes such as alkaline phosphatase (ALP), horseradish peroxidase (HRP), β galactosidase (β-gal), fluorescent dyes such as fluorescein isothiocyanate (FITC) and rhodamine isothiocyanate (RITC), and fluorescent protein such as allophycocyanin (APC) and phycoerythrin (R-PE), avidin, biotin, metal particles, latex, and the like.

The composition of the present invention may contain other components allowable as reagents such as sterile water, saline, a buffer, and a preservative as necessary besides the above-described molecule as the active ingredient.

In addition, the composition can be combined with other preparations necessary for the test into a kit. The other preparations includes, for example, substrates necessary for detecting labels, positive controls and negative controls, buffers used for diluting and washing samples, and the like. In addition, in the case where an unlabeled antibody is used as a preparation, the kit may contain a preparation obtained by labeling a substance (for example, a secondary antibody, protein G, protein A, or the like) binding to the antibody. Moreover, the kit may contain an instruction manual for the kit.

### [Examples]

Hereinafter, the present invention will be described in further detail based on Examples, but the present invention is not limited to Examples described below.

### [Materials and Methods]

### (1) Growth conditions and strains of C. elegans

Nematode was cultured on a nematode growth medium (NGM) agar plate having Escherichia coli strain OP50 at 20°C using a standard technique. In these Examples, the following worm strains were used.
N2(WT); DA2123, adls2122 [lgg-1p::GFP::lgg-1 + rol-6(su1006)]; AM140, rmlsl32 [unc-54p::Q35::YFP]; TU3401, sid-1(pk3321) V; uIs69 [pCFJ90(myo-2p::mCherry) + unc-119p::sid-1]; VP303, rde-1(ne219) V; kbIs7 [nhx-2p: :rde-1 + rol-6(su1006)]. NR350, rde-1(ne219) V; kzls20 [hlh-1p::rde-1 + sur-5p::NLS::GFP]; NR222, rde-1(ne219) V; kzIs9 [(pKK1260) lin-26p: :NLS: :GFP + (pKK1253) lin-26p::rde-1 + rol-6(su1006)]; CB1370, daf-2(e1370)III; DA465, eat-2(ad465) II; CB4037, glp-1(e2141ts)III; OP198, unc-119(ed3) III; wgIs198 [MML-1::TY1::EGFP::3xFLAG(92C12) + unc-119(+)]; MQ887, isp-1(qm150)IV; MAH215, sqIs11 [lgg-1p::mCherry::GFP::lgg-1 + rol-6].

### (2) Plasmid construction and transgenesis

As a CeRubicon:EGFP translational fusion construct, a coding sequence was cloned into pDC4 vector containing EGFP tag in addition to CeRubicon 4kb endogenous promoter. The microinjection of the construct was conducted together with co-injection marker myo-2p::mCherry to generate CeRubicon::EGFP.

### (3) Mice

CAG-Cre mice and Nestin-Cre mice were obtained from The Jackson Laboratory and Dr. Jun-ichi Miyazaki's Laboratory (Osaka University), respectively. CAG-Cre mice were crossed with Rubiconflow mice (Tanaka, S. et al. , Hepatology 64, 1994-2014 (2016)) to produce mice with systemic Rubicon knockout. Resultant mice with the Rubicon-allele were backcrossed into the C57BL/6J wild-type strain five times, followed by intercrossing between Rubicon+/- mice to generate Rubicon-/- mice and wild-type controls. Nestin-Cre mice were crossed with Rubiconflow mice to produce mice having homozygous deletion of Rubicon specifically in the brain. All the mice used in this Example were maintained on a C57BL/6J background with the exception of calorie-restricted mice. For these experiments, an adult onset 40% calorie restriction protocol developed by Turturro et al. (Turturro, A. et al., J Gerontol a-Biol 54, B492-B501 (1999)) was used. Female BDF1 mice were reared individually in cases. The CR protocol was continued until 9 months of age. Water was provided ad libitum.

### (4) Fly stocks and culture conditions

Flies were grown on a standard cornmeal-agar-yeast based medium at 25°C.

Flies having UAS-MJDtr-Q27 (#8149), UAS-MJDtr-Q78s (#8150, for eye degeneration), UAS-MJDtr-Q78w (#8141), UAS-GFP-IR (#9330), UAS-dRubicon (CG12772)-IR (#43276), UAS-GFP-mCherry-Atg8a (#37749), da-GAL4 (#55849), and elav-GAL4c155 (#458) were obtained from the Bloomington Stock Center. Transgenic fly lines having gmr-GAL4 have been reported so far (Yamaguchi, M. et al., Oncogene 18, 6767-6775 (1999)).

### (5) Individual number statistical analysis

For worms, synchronized eggs were obtained from 4 to 6-hour egg lays on RNAi plates. Starting with day 1 adults, lifespan experiments were set up at a density of 15 to 20 animals per plate, and carried out at 20°C. Worms were transferred to new plates every other day. Survivorship was also counted every other day. All of RNAi lifespan was carried out from egg. Death was recorded as the absence of any movement after stimulation with a platinum wire. Worms that underwent internal hatching or bursting vulva or that crawled off the plates were excluded. For TOR RNAi experiments, knockdown was performed from adults onward. Statistical analysis was conducted on Excel (Microsoft) by using the Mantel-Cox log-rank method. Experimental flies were grown on a standard medium, and were crossed for 48 hours after birth, and thereafter, were sorted using CO2 anesthesia. Vials were changed to fresh food without anesthesia every 2 to 3 days, and deaths were recorded until all the flies were died.

### (6) RNA interference

RNA interference(RNAi) was conducted by feeding HT115 (DE3) bacteria transformed with vector L4440, which produces dsRNA against the target gene. Synchronized eggs were placed on the indicated RNAi plate containing IPTG and ampicillin. RNAi clones were obtained from the Ahringer or Vidal RNAi library. let-363/TOR RNAi clones were supplied by Dr. Hansen (Sanford-Burnham Medical Research Institute). In double knockdown experiments, equal volumes of bacteria for respective RNAis are mixed and seeded on plates. Empty vector (L4440) and luciferase (L4440::Luc) RNAi were used as non-targeting controls.

### (7) RNA extraction and qRT-PCR

Tissue samples of worms, flies, and mice were collected in TRIzol (Invitrogen) or QIAZOL (Qiagen). Total RNA was extracted using RNeasy kit (QIAGEN). cDNA was generated using iScript (Bio-Rad) or PrimeScript RT reagent kit (Takara). qRT-PCR was performed with Power SYBR Green (Applied Biosystems) on ViiA7 Real-Time PCR System (Applied Biosystems) or with KAPA SYBR Fast qPCR kit (KAPA Biosystems) on CFX96 Real-Time PCR Detection System (Bio-Rad). Four technical replicates were performed for each reaction. As internal controls, ama-1 (worms), Rpl32 (flies), 18s rRNA (mice), and GAPDH (humans) were used.

### (8) Multi-worm tracking analysis

A 13 cm×10 cm agar-filled plate was divided into four regions having equal area. To keep animals from moving between regions, the regions are surrounded with glycerol, which is an aversive stimulus for C. elegans. Worms from a given experimental group were placed in one of the four regions, and multiple experimental groups were tested simultaneously. An adapted version of the Multi-Worm Tracker (Swierczek, N. A. et al, Nat Methods 8, 592-598 (2011)) was used to record the locomotion of C. elegans on the agar plate. The locomotion was recorded for 10 minutes, and the recording was analyzed using Choreography (part of the Multi-Worm Tracker software) and custom-written scripts to organize and summarize the data. Animal tracks were collected as time series of centroid position for each frame of the final 2 minutes of the recording. The initial 8 minutes were ignored to allow animal recognition by the tracker to stabilize. Choreography filters (shadowless and -t10) were used to avoid image artefacts. The speed of an individual was calculated as the sum of distances between sequential centroid positions, divided by the duration of the track. Experimental groups were summarized using mean weighted by the duration of the animal's track and s.e.m. of the mean.

### (9) Climbing assay

The climbing assay was performed according to a published protocol (Suzuki, M. et al., Human molecular genetics 24, 6675-6686 (2015)) with slight modification. Ten to twenty flies were placed in a conical glass tube (length 15 cm; diameter 2.5 cm) without anesthesia. Ten seconds after tapping the flies to the bottom of the tube, the number of flies in each vertical area was counted and scored as follows: score 0 (0 to 2 cm), 1 (2 to 3.9 cm), 2 (4 to 5.9 cm), 3 (6 to 7.9 cm), 4 (8 to 9.9 cm), 5 (10 to 15 cm). Three trials were performed on each group at intervals of 20 seconds, and the climbing score was calculated as follows : each score multiplied by the number of flies was divided by the total number of flies, and the mean score of the 3 trials was calculated. Results are presented as the mean ± s.e.m. of the scores obtained in 3 to 9 independent experiments.

### (10) Use of microscopy and quantification

To monitor autophagic activity, GFP::LGG-1 and mCherry::GFP::LGG-1 animals at the L4 stage were anesthetized in 0.1% sodium azide, and images were acquired using an Olympus FV1000 confocal microscope. Using these images, GFP and/or mCherry puncta in the anterior intestinal region or in the pharyngeal region were counted and quantified. For CeRubicon::EGFP and Q35::YFP worms, fluorescent images of the aligned whole worms on ice cold empty plates were captured by stereomicroscope SZX16 equipped with DP80 CCD camera (Olympus) and the number of polyQ aggregates per worms was counted.

### (11) Imaging of fly eyes

Light microscopic images of 1-day-old adult female flies were taken using a stereomicroscope model SZX16 (Olympus) equipped with CCD camera (DP22, Olympus). The number of necrotic patches per compound eye was counted for each genotype.

### (12) Autophagic flux of flies

To match the feeding status, 4-day-old flies expressing GFP-mCherry-Atg8a either with or without dRubicon-IR under the da-GAL4 driver were starved by 0.75% agar for 15 hours and refed for 4 hours. The brains were dissected, fixed in 4% formalin in PBS, incubated with 80% glycerol overnight, and then mounted with DAPI fluoromount-G (SouthernBiotech). Fluorescent images of the KC layers were taken by confocal microscope (Leica TCS SP8) and the number of GFP and/or mCherry puncta in 25×50 µm region of interest containing 150 to 200 cells were counted. Three un-overlapping images were used for quantification of each KC layer.

### (13) Stress resistance assay

Day-1 adult worms were subjected to oxidative stress (4.4 mM H₂O₂ in the empty plate) or heat stress (35°C) for 3 hours or 7 hours, respectively, and the survived worms were counted. 30 worms were used per condition and the experiments was repeated three times

### (14) Western blotting

Worm or mouse tissues were lysed in lysis buffer (50 mM Tris/HCl [pH 7.4], 150 mM NaCl, 1 mM EDTA, 0.1% NP-40, protease and phosphatase inhibitor cocktail [Roche]) using a homogenizer. After centrifugation, the resultant supernatants were subjected to protein quantification and western blotting. Protein lysates of worm and mouse tissues were separated by SDS-polyacrylamide gel electrophoresis and transferred to PVDF membranes, which were then blocked and incubated with specific primary antibodies. Primary antibodies and dilutions used for mouse tissues western blotting were as follows:
Rubicon (Cell Signalling Technology, #8465, 1:500), LC3 (Cell Signalling Technology, #2755, 1:1000), p62 (MEDICAL & BIOLOGICAL LABORATORIES CO., LTD., PM045, 1:1000), phospho-p70 S6 kinase (Thr398) (Cell Signalling, #9209, 1:500), and β-actin (Sigma Aldrich, A5316, 1, 8000) .

### (15) Immunohistochemistry

For flies, adult female brains (3 days after eclosion) were dissected, fixed in 4% formalin in PBS, blocked with 50% Block Ace (Sumitomo Dainippon Pharma Co. , Ltd.) in PBS/T (0.5% Triton X-100 in PBS) and incubated with an anti-HA antibody (3F10, 1:500, Sigma) to stain MJDtrQ78 protein with HA tag. The immunostaining were visualized with an Alexa 488-conjugated anti-rat antibody and nuclei were stained with DAPI. Images of Kenyon cell layer (KC layer) were taken by confocal microscope (LSM780, Zeiss). Signal intensities of HA staining were quantified using Image J 1.51 s software. Ten bilateral KC layers from five animals were analyzed for each group. For mice, immunohistochemical staining for collagen-I was performed on paraffin-embedded section. After antigen retrieval by autoclaving in 0.01 mmol/L citrate buffer (pH 6.0) for 10 minutes at 120°C, the sections were blocked with 3% BSA in PBS for 60 minutes. The blocked sections were incubated with primary antibodies, anti-collagen-I (abcam, ab34710, 1:400) at 4°C overnight, incubated for 30 minutes at room temperature, followed by detection using a HRP-diaminobenzidine compound (Nichirei Corp.). The sections were counterstained with hematoxylin.

### (16) Injection and detection of recombinant α-Syn fibrils

Injection of fibrils and their detection were reported previously (Luk, K. C. et al., Science 338, 949-953 (2012), Watanabe, Y. et al., Autophagy 13, 133-148 (2017)).

In brief, prepared fibrils were diluted in sterile PBS and sonicated before intracerebral injection. mice between 8 to 10 weeks of age were anesthetized with chloral hydrate (250 mg/kg, i.p.) and stereotaxically injected in both both hemispheres (coordinates: +0.2 mm relative to bregma, +2.0 mm from midline) with recombinant α-Syn fibril (1 µl of 1 mg/ml) . Animals were killed at 10 months after birth. Frozen brain sections including the fibril-injected areas were obtained using CM3050S cryostat (Leica Biosystems) . The sections were incubated with mouse monoclonal antibody pSyn#64 (dilution, 1:1000, Wako Pure Chemical Industries, Ltd.), followed by Alexa Fluor 488 labeled secondary antibody (dilution, 1:1000, Invitrogen) for detection. Images were acquired and quantified on Biorevo BZ-9000 (Keyence Corporation).

### (17) Cell culturing

RPE-1 cells were inoculated at a density of 5.0×104 cells per 6 wells. After one day, cells were treated with 20 nM siRNA relative to luciferase and MondoA (Santa Cruz) . After the siRNA treatment over 48 hours, the RPE1 cells were lysed in a sample buffer and subjected to SDS-polyacrylamide gel electrophoresis and western blotting. The Rubicon protein levels were quantified by western blotting. One day before the siRNA treatment, RPE-1 cells were inoculated at a density of 5.0×104 per 6 wells.

### (18) Statistical analysis

Results are presented as the mean ± s.e.m. Statistical tests were performed through one-way or two-way ANOVA with Tukey's test or the t-test using GraphPad Prism (GraphPad Software or Excel (Microsoft Office 2011).

### (19) TRAP staining

Bone marrow cells were cultured into a 24-well plate and derived to osteoclasts by adding M-CSF and RANKL. The resultant was fixed with 4% paraformaldehyde for 5 minutes, and after washing with distilled water, 50 mM tartaric acid-containing buffer (Ph 5.0) and chromogenic substrate were added, followed by reaction at 37°C for 45 minutes. The stain by TRAP activity was microscopically observed with BZ-X700 (Keyence).

### (20) Pit formation assay

were cultured into an osteo assay plate (Corning #3988), and derived to osteoclasts by adding M-CSF and RANKL. Osteoclasts dissolved the bone substrate to form resorption lacunae (Pit), which were microscopically observed with BZ-X700 (Keyence).

### (21) ALP staining

Cells were cultured for 5 days in a bone differentiation medium with 12-well plate for the primary culture system and 96-well plate for the culture cell system. The bone differentiation medium was prepared with αMEM supplemented with 10% FBS, 50µg/ml L-ascorbate phosphate (Sigma), 10 mM β-glycerophosphate (Sigma) . The resultants were fixed with 4% paraformaldehyde (in 0.1M cacodylate buffer; pH 7.3) for 30 minutes, and Bromo-Chloro-Indolyl phosphate/Nitro Blue Tetrazolium Chloride was added to stain at 37°C for 20 minutes, which were then microscopically observed with BZ-X700 (Keyence).

### (22) Alizarin staining

Cells were cultured for 4 weeks in a bone differentiation medium with 12-well plate for the primary culture system and 96-well plate for the culture cell system. The bone differentiation medium was prepared with αMEM supplemented with 10% FBS, 50µg/ml L-ascorbate phosphate (Sigma), 10 mM β-glycerophosphate (Sigma). The bone differentiation medium was replaced every two days, and the resultant was fixed with 4% paraformaldehyde (in 0.1M cacodylate buffer; pH 7.3) for 30 minutes . The resultant was washed with 0.1M cacodylate buffer (pH 7.3) and stained with Alizarin Red S (pH 4.0) for 5 minutes, followed by washing again with distilled water, which were then dried and microscopically observed with BZ-X700 (Keyencne).

### (23) Bone analysis

C57BL/6 background male mice of 12 weeks of age were used for analysis. Note that in osteoporotic models, ovaries were extirpated from female mice of 9 weeks of age, and bone morphology was measured after 4 weeks. For bone morphology, the distal ends of the femurs of mice were CT-scanned (3D micro-X-ray CT for small and medium experimental animals/CosmoScan AX; Rigaku). Thereafter, the 3D micro-CT data were analyzed with TRI/3D-BON software (RATOC Systems) to analyze the bone morphology.

### [Examples]

### (1) Regulation of lifespan through adjustment of autophagy by Rubicon

We searched for C. elegans Rubicon homologs and found that Y56A3A.16 has sequence similarity to human Rubicon (KIAA0226), human KIAA0226L (recently identified as Pacer) (Cheng, X. et al., Mol Cell 65, 1029-1043 (2017)) and the fruit fly Rubicon (dRubicon, CG12772) (Banreti, A. et al., Dev Cell 28, 56-69 (2014)) (Fig. 5A).

In mammals, Rubicon inhibits autophagy, but Pacer is reportedly required for normal progression of autophagy. Therefore, we next, sought to determine which of these two proteins is more functionally similar to Y56A3A.16. As observed in Rubicon knockdown mammalian cells (NPL 6), knockdown of Y56A3A.16 by RNAi significantly increased the number of GFP::LGG-1 dots, which are an autophagosome marker, in enterocytes ((i), (ii) in Fig. 1A) . This increase was completely abolished by concomitant knockdown of bec-1/Beclin 1, both of which function in the autophagosome formation. This fact suggests that knockdown of Y56A3A. 16 increased autophagic vacuoles ((i), (ii) in Fig. 1A).

To further determine whether knockdown of Y56A3A.16 increases or blocks the autophagic activity, we used the recently developed transgenic worms expressing tandem fluorescent LGG-1 (mCherry::GFP::LGG-1) to monitor autophagic flux (NPL 1). We observed that knockdown of Y56A3A.16 significantly increases the numbers of autophagosomes (AP) and autolysosomes (AL) in intestines and pharyngeal muscles ((i), (ii) in Fig. 1B, (i), (ii) in Fig. 5B). This indicates the autophagic flux is enhanced by the knockdown. Taken together with the fact that there is only one Rubicon homolog in zebrafish and fruit fly (Fig. 5A) (Banreti, A. et al., Dev Cell 28, 56-69 (2014)), this result implies that Y56A3A.16 represents the ancestral homolog of Rubicon (hence, Y56A3A.16 is referred to as "CeRubicon"). Interestingly, we found that gene expression experiments using CeRubicon: :EGFP transgenic worms and qRT-PCR analysis revealed that CeRubicon was upregulated in old worms (day 7) in comparison with young worms (day 1) ((i), (ii) in Fig. 1C). These results led us to hypothesis that age-dependent increase of Rubicon, a negative regulator of autophagy, could be a causal factor for age-dependent impairment of autophagy. Consistent with this idea, CeRubicon knockdown by RNAi significantly extended wild-type worm lifespan ((i), (ii) in Fig. 1D, (i) in Fig. 5C). The CeRubicon knockdown does not alter the pharyngeal pumping rates, indicating that the bacteria containing RNAi are properly taken in ((i) in Fig. 6B). The transcription level of Rubicon was decreased to about 50% of the normal levels of these animals ((ii) in Fig. 5C) .

In mammalian cells, Rubicon has been shown to repress both autophagy and endocytosis, but an increase in lifespan was completely abolished when the autophagy regulators bec-1/Beclin 1, unc-51/ULK1, and atg-18/ATG 18 were knocked down by RNAi with CeRubicon ((i), (ii) in Fig. 1D, (i) in Fig. 5C). This indicates that the longevity enhancement conferred by the CeRubicon knockdown is dependent on autophagic activity. Conversely, we found that the overexpression of CeRubicon shortened the lifespan of wild-type worms ((i), (ii) in Fig. 1E).

These results as described above suggest that CeRubicon regulates the lifespans by modulating autophagic activities.

### (2) Improvement of phenotypes depending on the age by systemic Rubicon knockdown

We investigated whether the knockdown of Rubicon would impact other age-onset phenotypes. Worms expressing polyglutamine (polyQ) -YFP in body wall muscles exhibit age-onset aggregation of the polyQ fusion protein (Morley, J. F. et al., P Natl Acad Sci USA 99, 10417-104229 (2002)). Although the CeRubicon knockdown reduced the level of aggregation, the concomitant knockdown of bec-1/Beclin 1 reverted the reduction. This indicates that the CeRubicon knockdown reduces aggregation of polyQ fusion protein in an autophagy-dependent manner (Fig. 2A, (i) in Fig. 2B). Locomotion activity goes down with age, and velocity correlates well with longevity (Hahm, J. H. et al., Nature Communications 6, doi:ARTN 8919). A multi-worm tracking system allowed us to monitor the tracks of individual worms and calculate their locomotion speeds (Swierczek, N. A. et al., Nat Methods 8, 592-598 (2011)). This analysis revealed that the CeRubicon knockdown slowed down the age-dependent decline in locomotion activity in an autophagy-dependent manner ((ii) in Fig. 2B, Fig. 6A).

Many long-lived animals often show the resistances against several stresses including oxidative stresses and heat stresses. We found that the CeRubicon knockdown increases the oxidative stress resistance in an autophagy-dependent manner ((iii) in Fig. 2B). On the other hand, the heat stress resistance did not change ((ii) in Fig. 6B). This indicates that the CeRubicon knockdown confers different downstream outputs with regard to the stress resistances.

To extend the findings in C. elegans, we asked if the Rubicon knockdown also extends the lifespans of flies. As a result, the systemic knockdown of fruit fly Rubicon (dRubicon) significantly increased the autophagic flux in the brain and extended the lifespan in female-specific manner (Fig. 2C, (i), (ii) in Fig. 6C, (i) in Fig. 6D). Notably, the knockdown of dRubicon also improved compound eye degeneration in MJDtr-Q78 (a truncated form of MJD/SCA3 containing an expanded length glutamine tract) polyQ disease model female flies ((i), (ii) in Fig. 2D) . Rapamycin, an inhibitor in mTOR kinase treatment, increases more in female mice than in male mice. Thus, this observation might reflect the sexual dimorphic contribution of autophagy to the animal lifespan. Similar to C. elegans, we found that the Rubicon protein level in kidney and liver was higher in older mice (20-month old) than in younger mice (2-month old) ((i), (ii) in Fig. 2E, (ii) in Fig. 6D). To understand the role of Rubicon regarding animal aging in mice, we developed Rubicon systemic-knockout mice which exhibit higher levels of LC3-II and reduced levels of autophagy substrate p62, suggesting activation of basal autophagy ((i) in Fig. 2F). Progressive fibrosis is a histological hallmark of aging kidney (Yamamoto, T. et al., Autophagy 12, 801-813 (2016)). Rubicon systemic-knockout mice exhibited a reduction in age-related fibrosis, as determined by the immunohistochemistry of collagen-I ((ii) in Fig. 2F, (i) in Fig. 2G). This phenotype was confirmed by qRT-PCR detection of mRNA encoding fibrotic markers ((ii) in Fig. 2G). In summary, knockdown or deletion of Rubicon ameliorated age-related phenotypes in worms, flies, and mice.

### (3) Slowing down aging due to decrease in Rubicon levels in neurons

We also investigated which tissues of CeRubicon are responsible for lifespan regulation in C. elegans. We used tissue-specific, RNAi-sensitive strains, including TU3401 (neuron-specific, using the sid-1 system) (Calixto, A. et al., Nature Methods 7, 554-U102 (2010)), NR350 (muscle), VP303 (intestine), and NR222 (hypodermis) for CeRubicon knockdown ((i) in Fig. 3A, (i) to (iii) in Fig. 7A) (Qadota, H. et al., Gene 400, 166-173 (2007)). The knockdown of CeRubicon in neuronal cells extended the lifespans most efficiently ((i) in Fig. 3A). While lower than the above, hypodermal and intestinal knockdown of CeRubicon extended the lifespan to a lesser extent but significantly ((i) to (iii) in Fig. 7A). Because neuronal cells are normally resistant to RNAi knockdown, the extension of lifespans conferred by the CeRubicon knockdown in wild-type worms might reflect the combinational effect of hypodermis, intestine, and other unidentified tissues ((i), (ii) in Fig. 1D, (i) in Fig. 5C) .

We next investigated whether increased autophagy activity in neurons by CeRubicon knockdown would be sufficient to extend animal lifespan. As a result, longevity enhancement conferred by CeRubicon knockdown in neuronal cells was completely abolished by concomitant knockdown of atg-18/WIPI ((i) in Fig. 7B). This suggests that the neuron activation of autophagy is sufficient to increase lifespan. Similarly, neuron-specific knockdown of Rubicon in fruit fly also extends lifespan in female fly ((ii) in Fig. 3A, (ii) in Fig. 7B). Moreover, we found that Rubicon knockdown decreased the polyQ inclusions in female brain neurons ((i), (ii) in Fig. 3B) and mitigated the age-associated decline in locomotor function, which is accelerated by the polyQ cytotoxicity ((iii) in Fig. 3B) .

Autophagy is implicated in several age-associated neurodegenerative disorders, including Parkinson's disease. In sporadic and familial Parkinson's disease, affected neurons develop inclusions containing α-synuclein (a-Syn). Elevated amounts of this protein are sufficient to cause Parkinson's disease. Overexpressed and misfolded aggregate-prone forms of α-Syn are degraded by autophagy. Therefore, we investigated whether forced activation of autophagy by deletion of Rubicon in neurons would be sufficient to prevent aggregation of α-Syn in the brain. To explore this possibility, we injected preformed α-Syn fibrils into the striatum of control and Rubicon neuronal (Nestin-cre) knockout mice as previously reported (Luk, K. C. et al., Science 338, 949-953 (2012), Watanabe, Y. et al., Autophagy 13, 133-148 (2017)), and compared the formation of Lewy body and Lewy neurite-like α-Syn inclusions after 10 months. Remarkably, the level of phosphorylated α-Syn positive signals was significantly reduced in neuron-specific Rubicon knockout mice ((i), (ii) in Fig. 3C), suggesting that neuronal Rubicon deletion suppressed the expansion of α-synuclein pathology. Together, these results suggest that neuronal knockdown of Rubicon is sufficient to ameliorate aging phenotypes in multiple organisms.

### (4) Downregulation of Rubicon in several lifespan-extending conditions

We investigated whether Rubicon expression is altered by several lifespan-extending conditions. In worms, several different longevity pathways and corresponding long-lived mutants have been well characterized. These include reduction of insulin/IGF-1 signalling (daf-2), germline removal (glp-1), calorie restriction (eat-2), and mitochondrial dysfunction (isp-1) (Kenyon, C. J. Nature 464, 504-512 (2010)). CeRubicon expression was suppressed in all of these long-lived worms in comparison with the wild-type ((i) in Fig. 4A, (ii) in Fig. 8B). Moreover, mice subjected to 9-month calorie restriction exhibited reduced Rubicon expression in liver and kidney ((ii) in Fig. 4A, (i) in Fig. 8B). In addition, we found that CeRubicon knockdown does not further extend longevity of daf-2, glp-1, eat-2, and isp-1 ((i) to (iv) in Fig. 8A). This result indicates that Rubicon is regulated downstream of multiple longevity paradigms, and the longevity of these animals is partly conferred by the reduction in Rubicon expression.

In worms, MML-1/Mondo has been identified as one of convergent transcription factors essential for multiple longevity pathways (Nakamura, S. et al., Nat Co mmun 7, 10944 (2016), Johnson, D. W. et al., Plos Genet 10 (2014)). In particular, knockdown of MML-1 abolishes autophagic activity and longevity in germline-deficient long-lived glp-1 animals. However, how MML-1 regulates autophagy is not fully characterized. Hence, we sought to address the possible involvement of MML-1 in regulation of CeRubicon. Interestingly, suppression of CeRubicon in glp-1 animals was completely reverted after knockdown of MML-1 or its heterodimeric partner, mxl-2 ((i) in Fig. 4B) although CeRubicon was still largely suppressed in other long-lived animals ((ii) in Fig. 8B). Moreover, MML-1 overexpression, which results in gradual lifespan extension (Nakamura, S. et al. described above), decreased CeRubicon expression in comparison with wild-type ((ii) in Fig. 4B). In addition, knockdown of MondoA, a mammal homolog of MML-1, increased Rubicon protein levels in mammalian cells ((i), (ii) in Fig. 4C) although the Rubicon transcription level was unchanged. These results indicate that Rubicon is regulated downstream of MML-1/MondoA especially in germline longevity context, and this regulation seems conserved in mammals.

The nutrient sensor mTOR is inactivated in response to nutrient deprivation, and inhibition of mTOR induces autophagy and extends the lifespan of a number of species (Kapahi, P. et al., Cell Metab 11, 453-465 (2010)). In addition, MML-1 regulates TOR activity by repressing the leucine sensor lars-1 (Nakamura, S. et al. described above). Hence, we examined the possible crosstalk between Rubicon and the mTOR pathway. As a result, surprisingly, we found that double knockdown of CeRubicon and let-363/mTOR further extended lifespan in comparison with either single knockdown (Fig. 4D). Knockdown of CeRubicon does not affect TOR activity, while TOR knockdown does not change CeRubicon expression ((i), (ii) in Fig. 8C). These results indicate that TOR and Rubicon, two major negative regulators of autophagy, contribute to longevity through partly independent downstream mechanisms.

### (5) Adjustment of bone metabolism through autophagy by Rubicon

We took images of the femurs of systemic Rubicon knockout mice with CT, and found that the bone mineral density (BMD), the bone volume/bone tissue volume fraction (BV/TV), and the bone mineral content (BMC) of the femurs were significantly increased in Rubicon knockout mice (Fig. 9A). Hence, we created primary culture systems of osteoclasts and osteoblasts, and evaluated the differentiation potential and function of these. We evaluated differentiation of osteoclasts through TRAP staining to find that the differentiation was facilitated in Rubicon knockout osteoclasts (Fig. 9B). In addition, we evaluated the bone resorption function of osteoclasts through pit formation assay to find that the bone resorption function was facilitated in Rubicon knockout osteoblasts (Fig. 9C). Next, we investigated the differentiation potential and the mineralization function of osteoblasts through the primary culture system and the culture cell system. Rubicon knockout cells in the culture cell system were formed with the Crisper-Cas9 system. We evaluated the differentiation potential through ALP staining to find that the differentiation potential was significantly increased in Rubicon knockout osteoblasts (Fig. 9D). In addition, we evaluated the mineralization function through alizarin staining to find that the mineralization function was facilitated in Rubicon knockout osteoblasts (Fig. 9E). In systemic Rubicon KO mice, differentiation potentials and functions were facilitated in both osteoclasts and osteoblasts, while in Rubicon knockout mice, the bone morphology of the femur was enhanced, leading us to assume that the function of osteoblasts is more important. In addition, we observed whether autophagy was facilitated in Rubicon knockout osteoblasts through LC3flux assay. As a result, LC3 flux was facilitated both under nutrient condition and under starvation condition in Rubicon knockout osteoblasts (Fig. 10).

In forming osteoblast-specific mice, we crossed Osterix-cre mice and Rubicon^{flow/flox} mice to form Rubicon^{flow/flox};Osterix-Cre mice. At the same time, we crossed Osterix-cre mice with Atg5^{flox/flox} mice and Rubicon^{flow/flox};Atg5^{flox/flox} mice to form osteoblast-specific Atg5 knockout mice and Rubicon/Atg5 knockout mice, and CT-analyzed their bone morphologies. As a result, osteoblast-specific Rubicon knockout mice exhibited significantly increased bone mineral density. On the other hand, it is reported that osteoblast-specific Atg7 knockout mice exhibit decreased bone mineral density in (Li, H. et al., Autophagy 14(10), 1726-1741 (2018)), and in this experiment as well, Atg5 knockout mice exhibited significantly decreased bone mineral density. In addition, Rubicon/Atg5 knockout mice exhibited bone mineral density decreased to a similar extent to Atg5 knockout mice (Fig. 11A). This fact indicates that osteogenesis in osteoblast-specific Rubicon knockout mice occurs in an autophagy-dependent manner.

In the present Examples, osteoblast-specific Rubicon knockout mice exhibited change in osteogenic ability, and we investigated whether this contributes to amelioration of osteoporosis by forming an osteoporotic model. As the osteoporotic model, we used a post-menopause osteoporotic model formed by extirpating both ovaries to deplete estrogen for 4 weeks. As a result, in sham mice, osteoporosis was induced, bone mineral density decreased, and bone volume/bone tissue volume fraction also decreased. On the other hand, in Rubicon knockout mice, bone mineral density and bone volume/bone tissue volume fraction were significantly ameliorated in the osteoporotic model (Fig. 11B).

Change in osteogenic ability was observed in an autophagy-dependent manner in Rubicon knockout mice, and autophagy, differentiation, and osteogenic function were facilitated in Rubicon knockout osteoblasts. This fact suggests that autophagy is involved in differentiation of osteoblasts. It has been known that the signal pathway of wnt, BMP, or Notch is involved in differentiation of osteoblasts (Zanotti, S. & Canalis, E. Molecular and Cellular Biology, 30(4), 886-896 (2010), Muguruma, Y. et al., Journal of Cellular Physiology, 232(9), 2569-2580 (2017)), in the present Examples, we focused on the signal pathway of Notch. This is because it has already been reported that osteoblast-specific Notch2 knockout mice (Notch has 1 to 4 isoforms, and in osteoblasts, Notch2 is highly expressed) exhibits change in osteogenic ability and differentiation and function of osteoblasts are also facilitated, and Notch receptor decreases in an autophagy-dependent manner (Yorgan, T. et al., Bone 87(C), 136-146 (2016), Wu, X. et al., Nature Communications 7, 1-17 (2016), Cao, Y. et al., Stem Cells and Development 24(22), 2660-2673 (2015)). In the previous report, the mechanism with which Notch2 knockout mice exhibit change in osteogenic ability of osteoblast has been reported as follows: specifically, deletion of Notch2 receptor lowers NICD which is transferred into nuclei to repress transcription factors downstream of Notch. Since Hes and Hey downstream of Notch negatively regulate Runx2 and Osterix, osteogenic transcription factors, decrease of Hes and Hey allows osteogenic transcription factors such as Runx2 and Osterix to increase, facilitating the differentiation potential and the osteogenesis function of osteoblasts. In view of this, the present inventors predicted that in Rubicon knockout osteoblasts, facilitated autophagy promotes degradation of the Notch receptor, so that Hes and Hey downstream of Notch decrease, and further Runx2 and Osterix increase. We observed expression of mRNAs of the transcription factor downstream of Notch and the osteogenic transcription factor, and found that the expression of mRNA decreased in the Rubicon knockout osteoblasts in Hey1, HeyL, Hes1, Hes3, and Hes7 downstream of Notch, whereas the expression of mRNA increased in the osteogenic transcription factors such as Runx2 and osteocalcin (Fig. 12A). Moreover, we observed whether Notch2 and NICD were degraded in Rubicon knockout osteoblasts through western blotting, and it was suggested that flux of Notch2 and NICD by lysosome inhibition increased in Rubicon knockout osteoblasts, and more Notch2 and NICD are degraded in Rubicon knockout cell (Fig. 12B) .

Although degradation of Notch receptor and NICD was facilitated in Rubicon knockout osteoblasts, it was unclear whether NICD functioned downstream of Rubicon. In view of this, NICD was overexpressed in Rubicon knockout osteoblasts, and the differentiation potential of NICD was evaluated through ALP staining. The differentiation was repressed to a similar extent to wild-type osteoblasts. This fact revealed that NICD was regulated downstream of Rubicon (Fig. 13A). Since NICD has two LIR motifs, we considered a possibility that NICD served as an autophagic substrate. Hence, we used NICD added with Flag tag and observed the binding of NICD and LC3 by the immunoprecipitation method, in which immunoprecipitation was conducted with Flag, and LC3 was detected through immunoblotting. The result revealed that LC3 and NICD directly bind to each other (Fig. 13B).

### [Industrial Applicability]

As described above, the present invention makes it possible to suppress aging associated with attenuation of autophagy with time, widely prevent, ameliorate, or treat an age-related disease or symptom, and extend lifespan, by targeting Rubicon. In addition, the present invention also makes it possible to test the degree of aging, the risk of developing these diseases or symptoms, or the risk of shortening lifespan, by targeting Rubicon. Therefore, the present invention greatly contributes particularly to the medical field.

## Claims

1. A composition for suppression of aging, prevention, amelioration, or treatment of an age-related disease or symptom, or extension of lifespan in a subject, the composition comprising a molecule that suppresses functions of a Rubicon gene as an active ingredient.

2. The composition according to claim 1, wherein
the molecule that suppresses functions of a Rubicon gene is a molecule described in any one of the following (a) to (c):
(a) an RNA binding to a transcription product of the Rubicon gene or a DNA encoding the RNA,
(b) an RNA or DNA binding to a Rubicon protein, and
(c) an antibody binding to the Rubicon protein.

3. The composition according to claim 1 or 2, wherein
the age-related disease or symptom is a metabolic bone disease or a symptom thereof, improvement in stress resistance, or an organ fibrosis.

4. The composition according to any one of claims 1 to 3, wherein
the subject is a vertebrate.

5. A method for evaluating a probability that a test compound has an activity of suppressing aging, an activity of preventing, ameliorating, or treating an age-related disease or symptom, or an activity of extending lifespan in a subject, the method comprising the steps of:
bringing the test compound and a Rubicon protein into contact; and
detecting binding of the test compound and the Rubicon protein, wherein
in a case where the test compound binds to the Rubicon protein, it is estimated that there is the probability that the test compound has an activity of suppressing aging, an activity of preventing, ameliorating, or treating an age-related disease or symptom, or an activity of extending lifespan.

6. A method for evaluating a probability that a test compound has an activity of suppressing aging, an activity of preventing, ameliorating, or treating an age-related disease or symptom, or an activity of extending lifespan in a subject, the method comprising the steps of:
bringing the test compound into contact with a cell expressing a Rubicon gene; and
detecting expression of the Rubicon gene in the cell, wherein
in a case where the test compound reduces the expression of the Rubicon gene compared with a case where the test compound is not brought into contact, it is evaluated that there is the probability that the test compound has an activity of suppressing aging, an activity of preventing, ameliorating, or treating an age-related disease or symptom, or an activity of extending lifespan.

7. A method for evaluating a probability that a test compound has an activity of suppressing aging, an activity of preventing, ameliorating, or treating an age-related disease or symptom, or an activity of extending lifespan in a subject, the method comprising the steps of:
bringing the test compound into contact with a cell having a DNA in which a reporter gene is functionally bound to downstream of a promoter region of a Rubicon gene; and
detecting expression of the reporter gene in the cell, wherein
in a case where the test compound reduces the expression of the reporter gene compared with a case where the test compound is not brought into contact, it is evaluated that there is the probability that the test compound has an activity of suppressing aging, an activity of preventing, ameliorating, or treating an age-related disease or symptom, or an activity of extending lifespan.

8. A method for testing a degree of aging, a risk of developing an age-related disease or symptom, or a risk of shortening lifespan in a subject, the method comprising the step of:
detecting an expression product of a Rubicon gene in a test sample, wherein
in a case where an amount of the expression product of the Rubicon gene is higher than that of control, it is evaluated that the degree of aging, the risk of developing an age-related disease or symptom, or the risk of shortening lifespan is high.

9. The method according to any one of claims 5 to 8, wherein
the age-related disease or symptom is a metabolic bone disease or a symptom thereof, an improvement in stress resistance, or an organ fibrosis.

10. The method according to any one of claims 5 to 9, wherein
the subject is a vertebrate.

11. A composition for detecting an expression product of a Rubicon gene in the method according to any one of claims 8 to 10, the composition comprising the following (a) or (b) :
(a) an oligonucleotide primer or oligonucleotide probe binding to a transcription product of the Rubicon gene; or
(b) an antibody binding to a Rubicon protein.


### [Sequence Listing]IBPF19-525WO-seq-fin.txt

SEQUENCE LISTING

<110>  Osaka University

<120>
\230V\211»\202Ì\227}\220§\201A\211Á\227î\220«\202Ì\216¾\212³\202à\202µ\202-
\202Í\217Ç\217ó\202Ì\227\\226h\201A\211ü\221P\201A\202à\202µ\202-\202Í\216¡
\227Ã\201A\202Ü\202½\202Í\216õ\226½\202Ì\211\204\222•\202Ì\202½\202ß\202Ì\221
g\220¬\225¨

<130>  IBPF19-525WO

<150>  JP 2018-141252
<151>  2018-7-27

<160>  7

<170>  PatentIn version 3.5

<210>  1
<211>  2919
<212>  DNA
<213>  Homo sapiens

<220>
<221>  CDS
<222>  (1)..(2919)

<400>  1
atg cgg ccg gag ggc gcg gga atg gag ctc gga ggc ggc gag gag cgc      48
Met Arg Pro Glu Gly Ala Gly Met Glu Leu Gly Gly Gly Glu Glu Arg
1               5                   10                  15

ctg cct gag gag agc agg agg gag cac tgg cag ttg ctg ggt aat ttg      96
Leu Pro Glu Glu Ser Arg Arg Glu His Trp Gln Leu Leu Gly Asn Leu
            20                  25                  30

aag acg acg gtg gag ggt ttg gta tca acc aac agc ccc aac gtc tgg      144
Lys Thr Thr Val Glu Gly Leu Val Ser Thr Asn Ser Pro Asn Val Trp
        35                  40                  45

tct aag tat ggt ggc ttg gag cgg ctt tgc agg gac atg cag agc atc      192
Ser Lys Tyr Gly Gly Leu Glu Arg Leu Cys Arg Asp Met Gln Ser Ile
    50                  55                  60

ctc tat cac ggg ctt atc cgt gac cag gcg tgc cgc cgc cag acg gat      240
Leu Tyr His Gly Leu Ile Arg Asp Gln Ala Cys Arg Arg Gln Thr Asp
65                  70                  75                  80

tac tgg cag ttc gtg aaa gac atc cgg tgg ctc agt ccc cac tca gcc      288
Tyr Trp Gln Phe Val Lys Asp Ile Arg Trp Leu Ser Pro His Ser Ala
                85                  90                  95

ctt cac gtg gag aag ttc atc agc gtg cac gag aac gac cag agc agt      336
Leu His Val Glu Lys Phe Ile Ser Val His Glu Asn Asp Gln Ser Ser
            100                 105                 110

gct gat ggt gcc agt gaa cgt gct gtt gcc gag ctg tgg ctg cag cac      384
Ala Asp Gly Ala Ser Glu Arg Ala Val Ala Glu Leu Trp Leu Gln His
        115                 120                 125

agc ctg cag tac cac tgc ctc tca gcc cag ctc cgg ccc ctg ctc ggg      432

Ser Leu Gln Tyr His Cys Leu Ser Ala Gln Leu Arg Pro Leu Leu Gly
    130                 135                 140

gat aga cag tat atc aga aaa ttc tac aca gat gct gcc ttc ctg cta      480
Asp Arg Gln Tyr Ile Arg Lys Phe Tyr Thr Asp Ala Ala Phe Leu Leu
145                 150                 155                 160

agt gac gct cat gtc acg gcc atg ctg cag tgc ctg gaa gca gtg gaa      528
Ser Asp Ala His Val Thr Ala Met Leu Gln Cys Leu Glu Ala Val Glu
                165                 170                 175

cag aac aac ccc cgc ctc ctg gct cag atc gat gcg tcc atg ttt gcc      576
Gln Asn Asn Pro Arg Leu Leu Ala Gln Ile Asp Ala Ser Met Phe Ala
            180                 185                 190

aga aag cac gag agc ccg ctc ctg gtg aca aag agc cag agc ctg aca      624
Arg Lys His Glu Ser Pro Leu Leu Val Thr Lys Ser Gln Ser Leu Thr
        195                 200                 205

gcc ctg ccc agt tcc aca tac acc cct cca aac agc tat gct cag cat      672
Ala Leu Pro Ser Ser Thr Tyr Thr Pro Pro Asn Ser Tyr Ala Gln His
    210                 215                 220

tcc tac ttt ggg tcc ttc tct agc ctc cac caa tcc gtg ccc aac aat      720
Ser Tyr Phe Gly Ser Phe Ser Ser Leu His Gln Ser Val Pro Asn Asn
225                 230                 235                 240

ggc tca gag aga aga tct act tcc ttt cca ctc tct ggc cct ccc cgg      768
Gly Ser Glu Arg Arg Ser Thr Ser Phe Pro Leu Ser Gly Pro Pro Arg
                245                 250                 255

aaa cct caa gaa agc aga ggg cac gtc tca cca gca gag gat caa acc      816
Lys Pro Gln Glu Ser Arg Gly His Val Ser Pro Ala Glu Asp Gln Thr
            260                 265                 270

atc caa gcc ccc cca gtt tca gtc tct gca cta gcc agg gat tcc cct      864
Ile Gln Ala Pro Pro Val Ser Val Ser Ala Leu Ala Arg Asp Ser Pro
        275                 280                 285

ttg acc cca aat gaa atg agc tcc agt act ctg acc agc ccc ata gag      912
Leu Thr Pro Asn Glu Met Ser Ser Ser Thr Leu Thr Ser Pro Ile Glu
    290                 295                 300

gca tcc tgg gtc agc agc cag aat gat tcc cca ggt gat gcc agt gag      960
Ala Ser Trp Val Ser Ser Gln Asn Asp Ser Pro Gly Asp Ala Ser Glu
305                 310                 315                 320

ggg cct gag tac ctg gcc att ggc aac ttg gac ccc cga ggc cgg act     1008
Gly Pro Glu Tyr Leu Ala Ile Gly Asn Leu Asp Pro Arg Gly Arg Thr
                325                 330                 335

gcc agc tgt cag agt cac agc agc aat gcc gag agc agc agt tcc aat     1056
Ala Ser Cys Gln Ser His Ser Ser Asn Ala Glu Ser Ser Ser Ser Asn
            340                 345                 350

ttg ttc tcc tcc agc agc tcc cag aag cca gat tct gct gcc tct tcc     1104
Leu Phe Ser Ser Ser Ser Ser Gln Lys Pro Asp Ser Ala Ala Ser Ser
        355                 360                 365

tta ggg gac cag gaa gga ggt ggg gag agc cag ctg tcc agt gtc ctc     1152
Leu Gly Asp Gln Glu Gly Gly Gly Glu Ser Gln Leu Ser Ser Val Leu
    370                 375                 380

cgc agg tcc agc ttc tca gag ggg cag aca ctc act gtc acc agt ggg 1200
Arg Arg Ser Ser Phe Ser Glu Gly Gln Thr Leu Thr Val Thr Ser Gly
385                 390                 395                 400

gca aag aaa agc cac att cgc tcc cat tcg gat acc agc att gcc tcc 1248
Ala Lys Lys Ser His Ile Arg Ser His Ser Asp Thr Ser Ile Ala Ser
                405                 410                 415

agg gga gct cca gaa tcc tgc aat gat aag gcg aag ttg aga ggc cct 1296
Arg Gly Ala Pro Glu Ser Cys Asn Asp Lys Ala Lys Leu Arg Gly Pro
            420                 425                 430

ttg ccc tac tct ggt caa agc agt gaa gtc agc aca ccc agc tct ctg 1344
Leu Pro Tyr Ser Gly Gln Ser Ser Glu Val Ser Thr Pro Ser Ser Leu
        435                 440                 445

tac atg gaa tat gaa ggt ggt cgg tac ctg tgc tca ggg gaa ggc atg 1392
Tyr Met Glu Tyr Glu Gly Gly Arg Tyr Leu Cys Ser Gly Glu Gly Met
    450                 455                 460

ttc cga aga cca tca gaa gga cag tcc ctc atc agc tac ctc tct gag 1440
Phe Arg Arg Pro Ser Glu Gly Gln Ser Leu Ile Ser Tyr Leu Ser Glu
465                 470                 475                 480

caa gac ttc ggc agc tgt gcc gac ctg gaa aag gag aat gcc cac ttc 1488
Gln Asp Phe Gly Ser Cys Ala Asp Leu Glu Lys Glu Asn Ala His Phe
                485                 490                 495

agc atc tca gag tcc tta att gct gcc atc gag cta atg aag tgc aac 1536
Ser Ile Ser Glu Ser Leu Ile Ala Ala Ile Glu Leu Met Lys Cys Asn
            500                 505                 510

atg atg agc cag tgc cta gag gag gag gaa gtg gaa gag gaa gac agt 1584
Met Met Ser Gln Cys Leu Glu Glu Glu Glu Val Glu Glu Glu Asp Ser
        515                 520                 525

gat aga gag atc cag gag ctg aag cag aag atc cgc ctt cgg cgc cag 1632
Asp Arg Glu Ile Gln Glu Leu Lys Gln Lys Ile Arg Leu Arg Arg Gln
    530                 535                 540

caa atc cgc acc aag aac ctg ctc ccc atg tac cag gag gct gag cac 1680
Gln Ile Arg Thr Lys Asn Leu Leu Pro Met Tyr Gln Glu Ala Glu His
545                 550                 555                 560

gga agc ttt cgg gtc acc tcc agc agc tcc cag ttc agc tca cgt gat 1728
Gly Ser Phe Arg Val Thr Ser Ser Ser Ser Gln Phe Ser Ser Arg Asp
                565                 570                 575

tcg gca cag ctc tct gac tct ggc tct gct gat gag gtt gat gaa ttt 1776
Ser Ala Gln Leu Ser Asp Ser Gly Ser Ala Asp Glu Val Asp Glu Phe
            580                 585                 590

gaa atc caa gat gct gac atc aga agg aac aca gcc tca agc agc aaa 1824
Glu Ile Gln Asp Ala Asp Ile Arg Arg Asn Thr Ala Ser Ser Ser Lys
        595                 600                 605

tcc ttc gtt tcc tcc cag tcc ttc tcc cac tgc ttc ctg cac tcc acg 1872
Ser Phe Val Ser Ser Gln Ser Phe Ser His Cys Phe Leu His Ser Thr
    610                 615                 620

tct gct gag gcg gtg gcc atg ggg ctc ctg aag cag ttt gag ggg atg 1920
Ser Ala Glu Ala Val Ala Met Gly Leu Leu Lys Gln Phe Glu Gly Met
625                 630                 635                 640

cag ctt cca gcc gcc tcg gag ctg gag tgg ctt gtc ccg gag cat gat 1968
Gln Leu Pro Ala Ala Ser Glu Leu Glu Trp Leu Val Pro Glu His Asp
                645                 650                 655

gcc cct cag aag ctc ctg ccc att cct gac tca ctg ccc atc tca ccg 2016
Ala Pro Gln Lys Leu Leu Pro Ile Pro Asp Ser Leu Pro Ile Ser Pro
            660                 665                 670

gat gac ggg cag cac gct gac atc tac aag ctg cgg att cgt gtt cgt 2064
Asp Asp Gly Gln His Ala Asp Ile Tyr Lys Leu Arg Ile Arg Val Arg
        675                 680                 685

ggc aac ttg gag tgg gcc ccg ccc cgg cct cag ata att ttt aat gtt 2112
Gly Asn Leu Glu Trp Ala Pro Pro Arg Pro Gln Ile Ile Phe Asn Val
    690                 695                 700

cat cca gcc cca acg agg aaa att gcc gtg gcc aag cag aat tac cgc 2160
His Pro Ala Pro Thr Arg Lys Ile Ala Val Ala Lys Gln Asn Tyr Arg
705                 710                 715                 720

tgt gca gga tgt ggc atc cgg act gac cct gat tac atc aag cga ctg 2208
Cys Ala Gly Cys Gly Ile Arg Thr Asp Pro Asp Tyr Ile Lys Arg Leu
                725                 730                 735

cgg tac tgt gag tac ctg ggc aag tac ttc tgc cag tgc tgc cac gag 2256
Arg Tyr Cys Glu Tyr Leu Gly Lys Tyr Phe Cys Gln Cys Cys His Glu
            740                 745                 750

aat gcc cag atg gcc atc ccc agc cgg gtt ctg cgc aag tgg gac ttc 2304
Asn Ala Gln Met Ala Ile Pro Ser Arg Val Leu Arg Lys Trp Asp Phe
        755                 760                 765

agc aag tac tac gtc agc aac ttc tcc aag gac ctg ctc att aag atc 2352
Ser Lys Tyr Tyr Val Ser Asn Phe Ser Lys Asp Leu Leu Ile Lys Ile
    770                 775                 780

tgg aat gat cct ctc ttc aac gtg cag gac ata aac agt gcc ctc tat 2400
Trp Asn Asp Pro Leu Phe Asn Val Gln Asp Ile Asn Ser Ala Leu Tyr
785                 790                 795                 800

agg aag gtc aag ctg ctc aat caa gtc cgg ctg ctg cgg gtc cag ctg 2448
Arg Lys Val Lys Leu Leu Asn Gln Val Arg Leu Leu Arg Val Gln Leu
                805                 810                 815

tgt cac atg aag aac atg ttc aag act tgc cga ctg gcc aag gag ctt 2496
Cys His Met Lys Asn Met Phe Lys Thr Cys Arg Leu Ala Lys Glu Leu
            820                 825                 830

ctg gat tcc ttt gac aca gtc cca ggc cac ctg aca gag gac ctc cac 2544
Leu Asp Ser Phe Asp Thr Val Pro Gly His Leu Thr Glu Asp Leu His
        835                 840                 845

ctg tac tca ctg aat gac ctg act gcg acc agg aag ggg gag ctg ggg 2592
Leu Tyr Ser Leu Asn Asp Leu Thr Ala Thr Arg Lys Gly Glu Leu Gly
    850                 855                 860

ccc cgg ctt gct gag ctc acc agg gca ggg gct acc cat gtg gag aga 2640
Pro Arg Leu Ala Glu Leu Thr Arg Ala Gly Ala Thr His Val Glu Arg
865                 870                 875                 880

tgc atg ctc tgc caa gcc aaa ggc ttc atc tgt gag ttc tgt cag aat 2688
Cys Met Leu Cys Gln Ala Lys Gly Phe Ile Cys Glu Phe Cys Gln Asn

885                 890                 895

gag gat gac atc atc ttt ccc ttt gag ctc cat aag tgc cgg acc tgt        2736
Glu Asp Asp Ile Ile Phe Pro Phe Glu Leu His Lys Cys Arg Thr Cys
            900                 905                 910

gaa gag tgt aaa gcg tgt tac cat aaa gcc tgc ttc aag tct gga agc        2784
Glu Glu Cys Lys Ala Cys Tyr His Lys Ala Cys Phe Lys Ser Gly Ser
        915                 920                 925

tgt ccg cgc tgc gag cgg ctg cag gcc cgg cgg gag gca ctg gcc agg        2832
Cys Pro Arg Cys Glu Arg Leu Gln Ala Arg Arg Glu Ala Leu Ala Arg
    930                 935                 940

cag agc ctg gag tct tac ctg tca gac tac gag gag gag ccc gcg gaa        2880
Gln Ser Leu Glu Ser Tyr Leu Ser Asp Tyr Glu Glu Glu Pro Ala Glu
945                 950                 955                 960

gcg ctg gcc ctg gaa gcc gcc gtc ctg gag gcc acc tga                    2919
Ala Leu Ala Leu Glu Ala Ala Val Leu Glu Ala Thr
                965                 970

<210>  2
<211>  972
<212>  PRT
<213>  Homo sapiens

<400>  2

Met Arg Pro Glu Gly Ala Gly Met Glu Leu Gly Gly Gly Glu Glu Arg
1               5                   10                  15

Leu Pro Glu Glu Ser Arg Arg Glu His Trp Gln Leu Leu Gly Asn Leu
            20                  25                  30

Lys Thr Thr Val Glu Gly Leu Val Ser Thr Asn Ser Pro Asn Val Trp
        35                  40                  45

Ser Lys Tyr Gly Gly Leu Glu Arg Leu Cys Arg Asp Met Gln Ser Ile
    50                  55                  60

Leu Tyr His Gly Leu Ile Arg Asp Gln Ala Cys Arg Arg Gln Thr Asp
65                  70                  75                  80

Tyr Trp Gln Phe Val Lys Asp Ile Arg Trp Leu Ser Pro His Ser Ala
                85                  90                  95

Leu His Val Glu Lys Phe Ile Ser Val His Glu Asn Asp Gln Ser Ser
            100                 105                 110

Ala Asp Gly Ala Ser Glu Arg Ala Val Ala Glu Leu Trp Leu Gln His
        115                 120                 125

Ser Leu Gln Tyr His Cys Leu Ser Ala Gln Leu Arg Pro Leu Leu Gly

130                 135                 140

Asp Arg Gln Tyr Ile Arg Lys Phe Tyr Thr Asp Ala Ala Phe Leu Leu
145                 150                 155                 160

Ser Asp Ala His Val Thr Ala Met Leu Gln Cys Leu Glu Ala Val Glu
                165                 170                 175

Gln Asn Asn Pro Arg Leu Leu Ala Gln Ile Asp Ala Ser Met Phe Ala
            180                 185                 190

Arg Lys His Glu Ser Pro Leu Leu Val Thr Lys Ser Gln Ser Leu Thr
        195                 200                 205

Ala Leu Pro Ser Ser Thr Tyr Thr Pro Pro Asn Ser Tyr Ala Gln His
    210                 215                 220

Ser Tyr Phe Gly Ser Phe Ser Ser Leu His Gln Ser Val Pro Asn Asn
225                 230                 235                 240

Gly Ser Glu Arg Arg Ser Thr Ser Phe Pro Leu Ser Gly Pro Pro Arg
                245                 250                 255

Lys Pro Gln Glu Ser Arg Gly His Val Ser Pro Ala Glu Asp Gln Thr
            260                 265                 270

Ile Gln Ala Pro Pro Val Ser Val Ser Ala Leu Ala Arg Asp Ser Pro
        275                 280                 285

Leu Thr Pro Asn Glu Met Ser Ser Ser Thr Leu Thr Ser Pro Ile Glu
    290                 295                 300

Ala Ser Trp Val Ser Ser Gln Asn Asp Ser Pro Gly Asp Ala Ser Glu
305                 310                 315                 320

Gly Pro Glu Tyr Leu Ala Ile Gly Asn Leu Asp Pro Arg Gly Arg Thr
                325                 330                 335

Ala Ser Cys Gln Ser His Ser Ser Asn Ala Glu Ser Ser Ser Ser Asn
            340                 345                 350

Leu Phe Ser Ser Ser Ser Ser Gln Lys Pro Asp Ser Ala Ala Ser Ser
        355                 360                 365

Leu Gly Asp Gln Glu Gly Gly Gly Glu Ser Gln Leu Ser Ser Val Leu
    370                 375                 380

Arg Arg Ser Ser Phe Ser Glu Gly Gln Thr Leu Thr Val Thr Ser Gly
385                 390                 395                 400

Ala Lys Lys Ser His Ile Arg Ser His Ser Asp Thr Ser Ile Ala Ser
                405                 410                 415

Arg Gly Ala Pro Glu Ser Cys Asn Asp Lys Ala Lys Leu Arg Gly Pro
            420                 425                 430

Leu Pro Tyr Ser Gly Gln Ser Ser Glu Val Ser Thr Pro Ser Ser Leu
        435                 440                 445

Tyr Met Glu Tyr Glu Gly Gly Arg Tyr Leu Cys Ser Gly Glu Gly Met
    450                 455                 460

Phe Arg Arg Pro Ser Glu Gly Gln Ser Leu Ile Ser Tyr Leu Ser Glu
465                 470                 475                 480

Gln Asp Phe Gly Ser Cys Ala Asp Leu Glu Lys Glu Asn Ala His Phe
                485                 490                 495

Ser Ile Ser Glu Ser Leu Ile Ala Ala Ile Glu Leu Met Lys Cys Asn
            500                 505                 510

Met Met Ser Gln Cys Leu Glu Glu Glu Glu Val Glu Glu Glu Asp Ser
        515                 520                 525

Asp Arg Glu Ile Gln Glu Leu Lys Gln Lys Ile Arg Leu Arg Arg Gln
    530                 535                 540

Gln Ile Arg Thr Lys Asn Leu Leu Pro Met Tyr Gln Glu Ala Glu His
545                 550                 555                 560

Gly Ser Phe Arg Val Thr Ser Ser Ser Ser Gln Phe Ser Ser Arg Asp
                565                 570                 575

Ser Ala Gln Leu Ser Asp Ser Gly Ser Ala Asp Glu Val Asp Glu Phe
            580                 585                 590

Glu Ile Gln Asp Ala Asp Ile Arg Arg Asn Thr Ala Ser Ser Ser Lys
        595                 600                 605

Ser Phe Val Ser Ser Gln Ser Phe Ser His Cys Phe Leu His Ser Thr
    610                 615                 620

Ser Ala Glu Ala Val Ala Met Gly Leu Leu Lys Gln Phe Glu Gly Met
625                 630                 635                 640

Gln Leu Pro Ala Ala Ser Glu Leu Glu Trp Leu Val Pro Glu His Asp
                645                 650                 655

Ala Pro Gln Lys Leu Leu Pro Ile Pro Asp Ser Leu Pro Ile Ser Pro
            660                 665                 670

Asp Asp Gly Gln His Ala Asp Ile Tyr Lys Leu Arg Ile Arg Val Arg
        675                 680                 685

Gly Asn Leu Glu Trp Ala Pro Pro Arg Pro Gln Ile Ile Phe Asn Val
    690                 695                 700

His Pro Ala Pro Thr Arg Lys Ile Ala Val Ala Lys Gln Asn Tyr Arg
705                 710                 715                 720

Cys Ala Gly Cys Gly Ile Arg Thr Asp Pro Asp Tyr Ile Lys Arg Leu
                725                 730                 735

Arg Tyr Cys Glu Tyr Leu Gly Lys Tyr Phe Cys Gln Cys Cys His Glu
            740                 745                 750

Asn Ala Gln Met Ala Ile Pro Ser Arg Val Leu Arg Lys Trp Asp Phe
        755                 760                 765

Ser Lys Tyr Tyr Val Ser Asn Phe Ser Lys Asp Leu Leu Ile Lys Ile
    770                 775                 780

Trp Asn Asp Pro Leu Phe Asn Val Gln Asp Ile Asn Ser Ala Leu Tyr
785                 790                 795                 800

Arg Lys Val Lys Leu Leu Asn Gln Val Arg Leu Leu Arg Val Gln Leu
                805                 810                 815

Cys His Met Lys Asn Met Phe Lys Thr Cys Arg Leu Ala Lys Glu Leu
            820                 825                 830

Leu Asp Ser Phe Asp Thr Val Pro Gly His Leu Thr Glu Asp Leu His
        835                 840                 845

Leu Tyr Ser Leu Asn Asp Leu Thr Ala Thr Arg Lys Gly Glu Leu Gly
    850                 855                 860

Pro Arg Leu Ala Glu Leu Thr Arg Ala Gly Ala Thr His Val Glu Arg
865                 870                 875                 880

Cys Met Leu Cys Gln Ala Lys Gly Phe Ile Cys Glu Phe Cys Gln Asn
                885                 890                 895

Glu Asp Asp Ile Ile Phe Pro Phe Glu Leu His Lys Cys Arg Thr Cys
            900                 905                 910

Glu Glu Cys Lys Ala Cys Tyr His Lys Ala Cys Phe Lys Ser Gly Ser
        915                 920                 925

Cys Pro Arg Cys Glu Arg Leu Gln Ala Arg Arg Glu Ala Leu Ala Arg
    930                 935                 940

Gln Ser Leu Glu Ser Tyr Leu Ser Asp Tyr Glu Glu Glu Pro Ala Glu
945                 950                 955                 960

Ala Leu Ala Leu Glu Ala Ala Val Leu Glu Ala Thr
                965                 970

<210> 3
<211> 956
<212> PRT
<213> Mus musculus

<400> 3
   Met Arg Pro Glu Gly Ala Gly Met Asp Leu Gly Gly Gly Asp Gly Glu
   1 5 10 15
   Arg Leu Leu Glu Lys Ser Arg Arg Glu His Trp Gln Leu Leu Gly Asn
   20 25 30
   Leu Lys Thr Thr Val Glu Gly Leu Val Ser Ala Asn Cys Pro Asn Val
   35 40 45
   Trp Ser Lys Tyr Gly Gly Leu Glu Arg Leu Cys Arg Asp Met Gln Asn
   50 55 60
   Ile Leu Tyr His Gly Leu Ile His Asp Gln Val Cys Cys Arg Gln Ala
   65 70 75 80
   Asp Tyr Trp Gln Phe Val Lys Asp Ile Arg Trp Leu Ser Pro His Ser
   85 90 95
   Ala Leu His Val Glu Lys Phe Ile Ser Leu His Glu Ser Asp Gln Ser
   100 105 110
   Asp Thr Asp Ser Val Ser Glu Arg Ala Val Ala Glu Leu Trp Leu Gln
   115 120 125
   His Ser Leu Gln Cys His Cys Leu Ser Ala Gln Leu Arg Pro Leu Leu
   130 135 140
   Gly Asp Arg Gln Tyr Ile Arg Lys Phe Tyr Thr Glu Thr Ala Phe Leu
   145                 150                 155                 160
   Leu Ser Asp Ala His Val Thr Ala Met Leu Gln Cys Leu Glu Ala Val
                   165                 170                 175
   Glu Gln Asn Asn Pro Arg Leu Leu Ala Gln Ile Asp Ala Ser Met Phe
               180                 185                 190
   Ala Arg Lys Gln Glu Ser Pro Leu Leu Val Thr Lys Ser Gln Ser Leu
           195                 200                 205
   Thr Ala Leu Pro Gly Ser Thr Tyr Thr Pro Pro Ala Ser Tyr Ala Gln
       210                 215                 220
   His Ser Tyr Phe Gly Ser Ser Ser Ser Leu Gln Ser Met Pro Gln Ser
   225                 230                 235                 240
   Ser His Ser Ser Glu Arg Arg Ser Thr Ser Phe Ser Leu Ser Gly Pro
                   245                 250                 255
   Ser Trp Gln Pro Gln Glu Asp Arg Glu Cys Leu Ser Pro Ala Glu Thr
               260                 265                 270
   Gln Thr Thr Pro Ala Pro Leu Pro Ser Asp Ser Thr Leu Ala Gln Asp
           275                 280                 285
   Ser Pro Leu Thr Ala Gln Glu Met Ser Asp Ser Thr Leu Thr Ser Pro
       290                 295                 300
   Leu Glu Ala Ser Trp Val Ser Ser Gln Asn Asp Ser Pro Ser Asp Val
   305                 310                 315                 320
   Ser Glu Gly Pro Glu Tyr Leu Ala Ile Gly Asn Pro Ala Pro His Gly
                   325                 330                 335
   Arg Thr Ala Ser Cys Glu Ser His Ser Ser Asn Gly Glu Ser Ser Ser
               340                 345                 350
   Ser His Leu Phe Ser Ser Ser Ser Ser Gln Lys Leu Glu Ser Ala Ala
           355                 360                 365
   Ser Ser Leu Gly Asp Gln Glu Glu Gly Arg Gln Ser Gln Ala Gly Ser
       370                 375                 380
   Val Leu Arg Arg Ser Ser Phe Ser Glu Gly Gln Thr Ala Pro Val Ala
   385                 390                 395                 400
   Ser Gly Thr Lys Lys Ser His Ile Arg Ser His Ser Asp Thr Asn Ile
                   405                 410                 415
   Ala Ser Arg Gly Ala Ala Gly Gly Pro Arg Asn Ile Thr Ile Ile Val
               420                 425                 430
   Glu Asp Pro Ile Ala Glu Gly Gly Gln Tyr Leu Cys Ser Gly Glu Gly
           435                 440                 445
   Met Phe Arg Arg Pro Ser Glu Gly Gln Ser Leu Ile Ser Tyr Leu Ser
       450                 455                 460
   Glu Gln Asp Phe Gly Ser Cys Ala Asp Leu Glu Lys Glu Asn Ala His
   465                 470                 475                 480
   Phe Ser Ile Ser Glu Ser Leu Ile Ala Ala Ile Glu Leu Met Lys Cys
                   485                 490                 495
   Asn Met Met Ser Gln Cys Leu Glu Glu Glu Glu Val Glu Glu Glu Asp
               500                 505                 510
   Ser Asp Arg Glu Ile Gln Glu Leu Lys Gln Lys Ile Arg Leu Arg Arg
           515                 520                 525
   Gln Gln Ile Arg Thr Lys Asn Leu Leu Pro Ala Tyr Arg Glu Thr Glu
       530                 535                 540
   Asn Gly Ser Phe Arg Val Thr Ser Ser Ser Ser Gln Phe Ser Ser Arg
   545                 550                 555                 560
   Asp Ser Thr Gln Leu Ser Glu Ser Gly Ser Ala Glu Asp Ala Asp Asp
                   565                 570                 575
   Leu Glu Ile Gln Asp Ala Asp Ile Arg Arg Ser Ala Val Ser Asn Gly
               580                 585                 590
   Lys Ser Ser Phe Ser Gln Asn Leu Ser His Cys Phe Leu His Ser Thr
           595                 600                 605
   Ser Ala Glu Ala Val Ala Met Gly Leu Leu Lys Gln Phe Glu Gly Met
       610                 615                 620
   Gln Leu Pro Ala Ala Ser Glu Leu Glu Trp Leu Val Pro Glu His Asp
   625                 630                 635                 640
   Ala Pro Gln Lys Leu Leu Pro Ile Pro Asp Ser Leu Pro Ile Ser Pro
                   645                 650                 655
   Asp Asp Gly Gln His Ala Asp Ile Tyr Lys Leu Arg Ile Arg Val Arg
               660                 665                 670
   Gly Asn Leu Glu Trp Ala Pro Pro Arg Pro Gln Ile Ile Phe Asn Val
           675                 680                 685
   His Pro Ala Pro Thr Arg Lys Ile Ala Val Ala Lys Gln Asn Tyr Arg
       690                 695                 700
   Cys Ala Gly Cys Gly Ile Arg Thr Asp Pro Asp Tyr Ile Lys Arg Leu
   705                 710                 715                 720
   Arg Tyr Cys Glu Tyr Leu Gly Lys Tyr Phe Cys Gln Cys Cys His Glu
                   725                 730                 735
   Asn Ala Gln Met Val Val Pro Ser Arg Ile Leu Arg Lys Trp Asp Phe
               740                 745                 750
   Ser Lys Tyr Tyr Val Ser Asn Phe Ser Lys Asp Leu Leu Leu Lys Ile
           755                 760                 765
   Trp Asn Asp Pro Leu Phe Asn Val Gln Asp Ile Asn Ser Ala Leu Tyr
       770                 775                 780
   Arg Lys Val Lys Leu Leu Asn Gln Val Arg Leu Leu Arg Val Gln Leu
   785                 790                 795                 800
   Tyr His Met Lys Asn Met Phe Lys Thr Cys Arg Leu Ala Lys Glu Leu
                   805                 810                 815
   Leu Asp Ser Phe Asp Val Val Pro Gly His Leu Thr Glu Asp Leu His
               820                 825                 830
   Leu Tyr Ser Leu Ser Asp Leu Thr Ala Thr Lys Lys Gly Glu Leu Gly
           835                 840                 845
   Pro Arg Leu Ala Glu Leu Thr Arg Ala Gly Ala Ala His Val Glu Arg
       850                 855                 860
   Cys Met Leu Cys Gln Ala Lys Gly Phe Ile Cys Glu Phe Cys Gln Asn
   865                 870                 875                 880
   Glu Glu Asp Val Ile Phe Pro Phe Glu Leu His Lys Cys Arg Thr Cys
                   885                 890                 895
   Glu Glu Cys Lys Ala Cys Tyr His Lys Thr Cys Phe Lys Ser Gly Arg
               900                 905                 910
   Cys Pro Arg Cys Glu Arg Leu Gln Ala Arg Arg Glu Leu Leu Ala Lys
           915                 920                 925
   Gln Ser Leu Glu Ser Tyr Leu Ser Asp Tyr Glu Glu Glu Pro Thr Glu
       930                 935                 940
   Ala Leu Ala Leu Glu Ala Thr Val Leu Glu Thr Thr
   945                 950                 955

<210> 4
<211> 948
<212> PRT
<213> Gallus gallus

<400> 4

Phe Leu Cys Arg Lys Glu His Trp Lys Leu Leu Gly Asn Leu Lys Thr
1               5                   10                  15

Thr Val Glu Gly Leu Val Ser Thr Ser Asn Pro Asn Val Trp Ser Lys
            20                  25                  30

Tyr Gly Gly Leu Glu Arg Leu Cys Arg Asp Met His Ser Ile Leu Tyr
        35                  40                  45

His Gly Leu Ile His Asp Lys Val Tyr Cys Arg Gln Lys Asp Tyr Trp
    50                  55                  60

Gln Phe Val Lys Asp Ile Arg Arg Leu Ser Pro Ser Ser Ala His His
65                  70                  75                  80

Leu Glu Lys Phe Ile Ser Leu His Glu Asn Gly Gln Pro Ser Pro Asp
                85                  90                  95

Ser His Ser Asp Gln Ala Ile Ala Gln Leu Trp Leu Gln His Ser Leu
            100                 105                 110

Gln Phe His Cys Leu Ser Ala Gln Leu Arg Pro Leu Leu Gly Asn Arg
        115                 120                 125

Gln Tyr Ile Arg Lys Phe Tyr Ala Asp Thr Ala Phe Leu Leu Ser Asp
    130                 135                 140

Ala His Val Thr Ala Met Leu Gln Cys Leu Glu Ala Val Glu Gln Asn
145                 150                 155                 160

Asn Pro Arg Leu Leu Ala Gln Ile Asp Thr Ser Met Leu Thr Arg Lys
                165                 170                 175

Gly Glu Ser Leu Ser Ser Val Thr Lys Ser Gln Ser Leu Thr Ala Leu
            180                 185                 190

Pro Gly Ser Phe Tyr Thr Leu Ser Pro Gly Cys Met Gln Gln His His
        195                 200                 205

Cys Gly Ser Phe Ser Ser Leu Pro Tyr Pro Ala Ser Ser Gly Leu Pro
    210                 215                 220

Asp Arg Arg Gln Ala Ser Ser Ser Ile Ser Ser Ser Ser Ser Gln Pro
225                 230                 235                 240

Gln Glu His Cys Leu Ala Ser Arg Ser Ser Ser Phe Ser Glu Gly Arg
                245                 250                 255

Ser Pro Leu Glu Gln Pro Ser Ala Val Pro Pro Ser His Ile Pro Ser
            260                 265                 270

Pro Lys Asp Pro Phe Ser Pro Ala Asn Glu Met Ser Ser Ser Thr Thr
        275                 280                 285

Ser Gln Ser Glu Asp Thr Trp Thr Gly Ser Gln Asp Asp Pro Gln Ser
    290                 295                 300

Asp Ala Asn Asp Gly Pro Glu Tyr Leu Ala Ile Gly Asn Leu Gly Arg
305                 310                 315                 320

Arg Gly Arg Ala Cys Ser Ser Ala Ser Thr Ser Ser Ser Lys Ser Ser
                325                 330                 335

Ser Ser Asn Met Phe Ser Ser Ser Ser Ser Gln Lys Leu Asp Ser Gly
            340                 345                 350

Asp Arg Gly Ala Asn Gly Cys Gly Ser Arg Gly Leu Gly Leu Leu Arg
        355                 360                 365

Arg Ser Ser Phe Ser Glu Gly Gln Ser Ser Val Pro Gln Gly Ile Leu
    370                 375                 380

Lys Lys Ser His Val Arg Ser His Ser Asp Thr Asn Val Ala Ser Gly
385                 390                 395                 400

Lys Leu His Glu Ser His Ala Asp Thr Val Gly Gly Arg Gly Thr Val
                405                 410                 415

Ser Ser Ser Thr Gln Ser Ser Glu Leu Ser Thr Pro Ser Ser Leu Tyr
            420                 425                 430

Met Glu Tyr Asp Ser Gly Gln Tyr Leu Ser Ser Gly Glu Gly Met Phe
        435                 440                 445

Arg Arg Pro Ser Glu Gly Gln Ser Leu Ile Ser Tyr Leu Ser Glu Gln
    450                 455                 460

Asp Phe Gly Ser Cys Ala Asp Leu Glu Lys Glu Asn Ala His Phe Ser
465                 470                 475                 480

Ile Ser Glu Ser Leu Ile Ala Ala Ile Glu Leu Met Lys Cys Asn Met
                485                 490                 495

Met Ser Arg Gln Leu Glu Glu Glu Glu Glu Asp Ser Asp Lys Glu Ile
            500                 505                 510

Gln Glu Leu Lys Gln Lys Ile Arg Ile Arg Arg Gln Gln Ile Arg Thr
        515                 520                 525

Lys His Leu Phe Ser Thr Cys Gln Glu Met Gly Ser Asp Ser Leu Ala
    530                 535                 540

Ala Thr Asp Ser Glu Ser Gln Phe Ser Ser His Gly Ser Met Arg Leu
545                 550                 555                 560

Ser Asp Ser Gly Ser Ala Asp Asp Val Glu Glu Tyr Glu Ile Arg Asp
                565                 570                 575

Ala Asp Ile Lys Arg Asn Pro Asp Ser Ser Arg Lys Pro Phe Leu Ser
            580                 585                 590

Ser Glu Ser Ile Ser His Ser Phe Gly Asn Ser Asn Ser Ala Glu Ala
        595                 600                 605

Val Ala Met Gly Leu Leu Lys Gln Phe Glu Gly Met Gln Leu Pro Ala
    610                 615                 620

Ala Ser Glu Leu Glu Trp Leu Val Pro Glu His Asp Ala Pro Gln Lys
625                 630                 635                 640

Leu Leu Pro Ile Pro Asp Ser Leu Pro Val Ser Pro Asp Asp Gly Glu
                645                 650                 655

His Ala Asp Ile Tyr Lys Leu Arg Ile Arg Val Arg Gly Asn Leu Glu

            660                 665                 670

Trp Ala Pro Pro Arg Pro Gln Ile Ile Phe Asn Val His Pro Ala Pro
        675                 680                 685

Thr Arg Lys Val Ala Val Ala Lys Gln Asn Tyr Arg Cys Ala Gly Cys
    690                 695                 700

Gly Ile Arg Thr Asp Pro Asp Tyr Ile Lys Arg Leu Arg Tyr Cys Glu
705                 710                 715                 720

Tyr Leu Gly Lys Tyr Phe Cys Gln Cys Cys His Glu Asn Ala Gln Thr
                725                 730                 735

Val Ile Pro Ser Arg Ile Leu Arg Lys Trp Asp Phe Ser Lys Tyr Tyr
            740                 745                 750

Val Ser Asn Phe Ser Lys Asp Leu Leu Ser Lys Ile Trp Ser Asp Pro
        755                 760                 765

Leu Phe Asn Val Gln Asp Ile Asn Pro Ala Leu Tyr Arg Lys Val Lys
    770                 775                 780

Ser Leu Asn Gln Val Trp Leu Leu Arg Ile Gln Leu Phe His Met Lys
785                 790                 795                 800

Asn Met Phe Lys Thr Cys Arg Leu Ala Lys Asp Leu Leu Asp Ser Phe
                805                 810                 815

Asp Ala Val Pro Gly His Leu Thr Glu Asp Leu His Leu Tyr Ser Leu
            820                 825                 830

Ser Asp Leu Ser Ala Thr Lys Arg Gly Asp Leu Val Pro Arg Leu Thr
        835                 840                 845

Glu Leu Leu Lys Ala Gly Ser Leu His Val Glu Lys Cys Met Leu Cys
    850                 855                 860

Gln Ala Lys Gly Phe Ile Cys Glu Phe Cys Gln Asn Glu Gly Asp Ile
865                 870                 875                 880

Ile Phe Pro Phe Glu Leu Asn Lys Cys Lys Thr Cys Glu Glu Cys Lys
                885                 890                 895

Ala Cys Tyr His Lys Ser Cys Phe Lys Ser Thr Arg Cys Pro Arg Cys
            900                 905                 910

Glu Arg Leu Gln Ala Arg Arg Glu Leu Leu Ala Lys Gln Ser Met Glu
        915                 920                 925

Ser Tyr Ile Ser Asp Tyr Glu Asp Asp Glu Leu Glu Gln Gln Glu Ala
    930                 935                 940

Ala Thr Ala Thr
945

<210>  5
<211>  914
<212>  PRT
<213>  Danio rerio

<400>  5

Met Glu Ile Gln Glu Gln Glu Asp Gly Glu Arg Arg Trp Glu Leu Trp
1               5                   10                  15

Lys Leu Leu Tyr Asn Leu Lys Thr Thr Val Glu Gly Leu Leu Ser Thr
            20                  25                  30

Asn Asn Pro Asn Val Trp Ser Arg Tyr Gly Gly Leu Gln Arg Leu His
        35                  40                  45

Lys Asp Met Asn Asn Ile Leu Gly His Gly Leu Lys His Glu Gln Ile
    50                  55                  60

Tyr Tyr Lys Gln Lys Asp Tyr Trp Arg Phe Val Trp Cys Val Arg Tyr
65                  70                  75                  80

Leu Cys Pro His Leu Ala Arg His Val Glu Gln Phe Ser Gln Leu Glu
                85                  90                  95

Pro Val Leu Ser Ser Ser Val Gln Ser Phe Gly Glu Gly Tyr Lys Ala
            100                 105                 110

Glu Arg Trp Leu Leu His Ser Leu Gln Ala His Val Leu Ser Ala Gln
        115                 120                 125

Leu Lys Pro Leu Leu Gln His Lys Met Asn Thr Gln Lys Tyr Tyr Asn
    130                 135                 140

Asp Gly Ala Phe Leu Leu Ser Glu Pro His Val Ser Ala Met Phe Gln
145                 150                 155                 160

Cys Leu Glu Ala Val Glu Gln Asn Asn Pro Arg Met Leu Ala Leu Ile
                165                 170                 175

Asp Thr Val Arg Leu Ser His Leu Lys Glu Ser Pro Leu Gly Leu Met
        180                 185                 190

Lys Ser Gln Ser Leu Cys Leu Leu Pro Gly Ala Pro Gly His Cys Arg
    195                 200                 205

Pro Ala Asp Ala Ser Gln Ser His Arg Leu Thr Thr Ser Gln Ser Cys
    210                 215                 220

Leu Thr Glu Ile Ser Asn Ser Pro Gln Ser Lys Ser Ser Ser Asp Val
225                 230                 235                 240

Ala Pro Ser Ser Ser Ala Gln Gly Arg Gly Asn Gln Val Ala Gly Ile
                245                 250                 255

Ser Phe Thr Glu Pro Val Leu Pro Glu Ser Arg Asp Phe Gly Asp Gly
        260                 265                 270

Asp Leu Asp Asp Gly Pro Glu Tyr Leu Ala Ile Gly Asn Leu Gly Lys
    275                 280                 285

Gln Lys Arg Arg Asp Ser Ser Ser Ser Val His Ser Ser Glu His Met
    290                 295                 300

Gly Thr Arg Gly Asp Ala His Thr Pro Leu Ala Pro Pro Pro Gln Arg
305                 310                 315                 320

Arg Cys Ser Phe Ser Glu Ala Gln Lys Ala Thr Gly Arg Ser Ser Arg
                325                 330                 335

Gly His Ile Arg Ser Leu Ser Asp Thr Gly Val Thr Gln Lys Gln Lys
        340                 345                 350

Asn Glu Ser Val Ala Ala Thr Ala Cys Ser Lys Ser Cys Gly Pro Phe
    355                 360                 365

Ser Pro Gln Ser Ser Asp Ser Ser Ala Asn Ser Ser Leu Tyr Met Glu
    370                 375                 380

Ser Asn Gly Ser Gln Tyr Thr Ser Val Pro Asp Gly Leu Phe Arg Arg
385                 390                 395                 400

Pro Ser Glu Gly Gln Ser Leu Ile Ser Tyr Leu Ser Glu Gln Asp Phe
                405                 410                 415

Gly Ser Cys Ala Asp Leu Glu Lys Glu Asn Ala His Phe Ser Ile Ser
        420                 425                 430

Glu Ser Leu Ile Ala Ala Ile Glu Leu Met Lys Cys Asn Met Arg Arg
        435                 440                 445

Pro Ala Glu Glu Ala Glu Glu Glu Gly Asp Ser Asp Ser Glu Ile Gln
    450                 455                 460

Gln Leu Lys Gln Lys Ile Arg Leu Arg Arg Leu Gln Ile Arg Arg Thr
465                 470                 475                 480

Arg Leu Lys Pro Ala Ala Ala Ala Ser Ser Asn Gln Val Gln Ser Val
                485                 490                 495

Asp Ser Gly Gly Ser Gln Arg Ser Ser Gln Asp Ser Phe His Pro Ser
            500                 505                 510

Asp Ser Gly Ser Asp Arg Glu Val Glu Asp Ile Glu Leu Arg Asp Gly
        515                 520                 525

Ser Asp Gly Gln Ser Leu Leu Ala Val Ser Gln Ser Gly Leu Ser Leu
    530                 535                 540

Ser Leu Ala Ser Leu Phe Ser Asp Ala Asp Ile Lys Arg Ser Ala Ser
545                 550                 555                 560

Ser Ser Arg Ser Phe Leu Ser Ser Glu Ser Ile Ser Pro Ser Met Leu
                565                 570                 575

Gln Ser Asn Ser Ala Glu Ser Val Ala Met Gly Leu Leu Arg Gln Phe
            580                 585                 590

Glu Gly Met Gln Leu Pro Ala Ala Ser Glu Leu Asp Trp Leu Val Pro
        595                 600                 605

Glu His Asp Ala Pro Gln Lys Leu Leu Pro Ile Pro Asp Ser Leu Pro
    610                 615                 620

Ile Ser Pro Asp Asp Gly Glu His Ala Asp Ile Tyr Lys Leu Arg Ile
625                 630                 635                 640

Arg Val Arg Gly Asn Leu Glu Trp Ala Pro Pro Arg Pro Gln Ile Ile
                645                 650                 655

Phe Asn Ile His Pro Pro Pro Lys Arg Lys Val Val Ile Ala Lys Gln
            660                 665                 670

Asn Tyr Arg Cys Ala Gly Cys Gly Ile Arg Ile Glu Gln Asp Tyr Met
        675                 680                 685

Lys Arg Leu Arg Tyr Cys Glu Tyr Leu Gly Arg Tyr Phe Cys Gln Cys
    690                 695                 700

Cys His Glu Asn Ala Gln Ser Val Ile Pro Ala Lys Ile Ile Arg Lys
705                 710                 715                 720

Trp Asp Phe Ser Lys Ser Tyr Val Ser Asn Phe Ser Arg Asp Leu Leu
                725                 730                 735

Ala Lys Ile Ser Gly Asp Pro Leu Phe Asn Leu Ser Asp Ile Asn Ser
            740                 745                 750

Ala Leu Tyr Lys Lys Val Lys Ala Leu Asp Thr Val Arg Ala Leu Arg
        755                 760                 765

Glu Gln Leu Asn Leu Met Lys Asn Leu Leu Lys Thr Cys Arg Leu Ala
    770                 775                 780

Lys Asp Val Leu Glu Glu Phe Asp Ser Leu Leu Pro Gly His Leu Thr
785                 790                 795                 800

Glu Asp Met Gln Leu Phe Ser Leu Ser Asp Leu Asn Ala Val Arg Ser
                805                 810                 815

Gly Ser Leu Thr Pro Gln Leu Arg His Leu Leu Arg Leu Gly Ser Thr
            820                 825                 830

His Val Thr His Cys Met Leu Cys Gln Ala Lys Gly Phe Ile Cys Glu
        835                 840                 845

Phe Cys Gly Asn Asp Lys Asp Ile Ile Phe Pro Tyr Glu Leu Asn Lys
    850                 855                 860

Cys Leu Arg Cys Glu Asp Cys His Ala Cys Tyr His Lys Ser Cys Phe
865                 870                 875                 880

Lys Thr Gly Thr Gln Cys Pro Arg Cys Leu Arg Leu Thr Glu Arg Arg
                885                 890                 895

Glu Arg Met Ala Arg Arg Asn Met Glu Glu Gln Glu Asp Glu Ala Cys
            900                 905                 910

Gly Thr

<210>  6

<211>  827
<212>  PRT
<213>  Drosophila melanogaster

<400>  6

Met Thr Thr Pro Pro Pro Pro Pro Thr Leu Ala Ser Asp Ala Ile Glu
1               5                   10                  15

Gly Ala Val Tyr Ala Ala Gly Glu Ala Leu His His Gln His Asp His
            20                  25                  30

Pro Gln Gln Gln Gln Gln Gln Gln Gln Gln Gln Gln Leu Ala Arg Asn
        35                  40                  45

Ser Leu Leu Ala Asp Leu Arg Arg Ser Thr Asn Phe Trp Phe Arg Ala
    50                  55                  60

Ala Lys Ser Thr Asp Gly Leu Gln Leu Leu Ala Gln Ser Cys Thr Arg
65                  70                  75                  80

Ile Leu Gln His Gly Leu Leu Gln Pro Val Glu Leu Arg Arg Leu Ala
                85                  90                  95

Asp Asp Phe Glu Phe Leu Leu Ala Ser Ala Gly Lys Gln Gln Gln Gln
            100                 105                 110

Thr Lys Pro Gln Pro Tyr Arg Pro Asp Tyr Ala Ala Arg Asp Ser Ala
        115                 120                 125

Ser Met Val Asn Gly Val Glu Glu Phe Leu Gly Glu Trp Thr Thr Arg
    130                 135                 140

Cys Leu Arg Ala Arg Cys Leu Ser Gln Cys Leu Gln Thr Leu Val Ser
145                 150                 155                 160

Asp Pro Glu Leu Met Glu Leu Tyr Tyr Pro Ser Glu Glu Ala Phe Leu
                165                 170                 175

Arg Arg Ser Gly Asp Ala Thr Ala Leu Phe Val Cys Leu Thr Ala Val
            180                 185                 190

Gln Leu Asn Gln Ser Gly Leu Leu Ser Gln Leu Glu Val Arg His Gln
        195                 200                 205

Val Arg His Arg Arg Thr Cys Ser Gln Pro Asn Phe Ser Ile Ser Pro
    210                 215                 220

Lys Leu Gln Ala Val Pro Glu Glu Arg Leu Gln Lys Arg Ile Phe Leu

225                 230                 235                 240

Arg Arg Leu Lys Ser Leu Pro Asn Leu Ser Gln Glu Asp Glu Glu Leu
                245                 250                 255

Asp Asp Arg Ala Ala Phe Arg Arg Arg Cys Gln Thr Phe Ser Ser Ser
            260                 265                 270

Arg Ser His Lys Leu Arg Glu Asp His Leu Asp Ala Pro Ser Thr Ser
        275                 280                 285

Ser Cys Ser Ser His Asp Ser Pro Arg Arg Ile Gln Leu Ile Asn Cys
    290                 295                 300

Asp Asp Ile Lys Ile Trp Thr Asp Gln Thr Pro Gln Glu Val Pro Glu
305                 310                 315                 320

Glu Val His Glu Lys Leu Gln Gln Ala Ser Lys Ala Ser Thr Ser Val
                325                 330                 335

Gly Gly Tyr Leu Gly Ser Phe Phe Gly Ser Pro Pro Leu Tyr Thr Ser
            340                 345                 350

Trp Phe His Arg Gly Val Gly Asp Asp Leu Asn Ala Ser Gly Asp Gly
        355                 360                 365

Gly Met Leu Asp Thr Phe Leu Pro Val Asn Gly Arg Lys Leu Lys Lys
    370                 375                 380

Gln His Thr Leu Phe Glu Gly Val Ser Met Leu Asp Glu Ala Ala Ser
385                 390                 395                 400

Ser Thr Ala Cys Ile Ser Ser Ala Pro Trp Asp Ile Gln Gly Ser Ala
                405                 410                 415

Gly Asp Ser Thr Thr Ser Thr Thr Ala Ser Ser Ser Val Ser Ile Thr
            420                 425                 430

Pro Gly Ser Asp Lys Met Asp Gln Gln Ser Leu Ala Ser Phe Leu Gln
        435                 440                 445

Met Ser Arg Gln Thr His Asn Asn Thr Gln Leu Glu Lys Glu Asn Ala
    450                 455                 460

His Phe Arg Ile Ser Glu Ala Cys Ile Thr Ala Ile Glu His Val Lys
465                 470                 475                 480

Trp Ser Arg Arg Leu Ala Thr Pro Ala Gly Pro Thr Ser His Asn Leu
                485                 490                 495

Ser Glu Pro Asn Leu Met Pro Tyr Ala Glu Gln Ile Pro Gly Gly Gly
            500                 505                 510

Met Gln Asn His Ser Ala Glu Ala Val Gly Leu Gln Leu Ile Ser Arg
        515                 520                 525

Phe Ser Glu Gln Gln Leu Pro Arg Leu Gly His Leu Lys Trp Leu Val
    530                 535                 540

Ser Glu Gln Glu Ala Pro Gln Gln Leu Leu Pro Leu Pro Thr Pro Lys
545                 550                 555                 560

Gln Asp His Glu Gln Ala Ser Leu Thr Arg Gly Thr Ser Ser Trp Ala
                565                 570                 575

Pro Pro Arg Gln Gln Ile Ile Phe Thr Glu His Pro Ser Glu Ser Arg
            580                 585                 590

Ser Lys Val Leu Gln Lys Gln Asn His Arg Cys Ala Gly Cys Gly Met
        595                 600                 605

Arg Val Ala Lys His Leu Gln Gln His Phe Arg Tyr Cys Thr Tyr Leu
    610                 615                 620

Gly Lys Tyr Leu Cys Thr Gly Cys His Arg Asn Gln Ile Ser Ala Ile
625                 630                 635                 640

Pro Ala Arg Ile Leu Arg Ser Trp Asp Phe Arg Cys Tyr Pro Val Cys
                645                 650                 655

Ser Phe Ala Tyr Arg Leu Ile Glu Gln Met Tyr Ala Phe Pro Leu Phe
            660                 665                 670

His Val Pro Asp Leu Asn Ser Gln Leu Tyr Lys His Lys Glu Leu Ala
        675                 680                 685

Lys Ala Arg Lys Arg Arg Leu Gln Leu Gln Ala Val Lys Gly Phe Ile
    690                 695                 700

Ala Asn Cys Arg Phe Ala Thr Arg Glu Gln Ser Phe Phe Asn Ala Ile
705                 710                 715                 720

Pro Ala His Ile Thr Gln Asp Pro Asp Met Trp Ser Met Cys Asp Phe
                725                 730                 735

Val Asp Val Gln Asn Thr Ser Met Asn Arg Ser Ile Lys Glu Val Ile
        740                 745                 750

Ala Leu Ser Glu Gln His Val His Asn Cys Val Leu Cys Thr Gly Arg
    755                 760                 765

Ala Phe Val Cys Glu His Cys Lys Gly Gly Glu Leu Ile Tyr Pro Trp
    770                 775                 780

Gln Arg Lys Val Gln Arg Cys Asp Arg Cys Gly Ala Cys Phe His Asn
785                 790                 795                 800

Gly Cys Trp Lys Val Arg Ser Arg Cys Arg Asn Gln Asn Asp Pro Cys
                805                 810                 815

Pro Arg Cys Ser Arg Leu Gln Ser Arg Ala Ser
        820                 825

<210>  7
<211>  597
<212>  PRT
<213>  Caenorhabditis elegans

<400>  7

Met Phe Asp Pro Ser Lys Trp Phe Gly Arg Ala Pro Tyr Gln Gln Val
1               5                   10                  15

Gly Asp Thr Asp Asp Pro Ile Ile Glu Val Asp Gln Ile Ser Phe Thr
            20                  25                  30

Ser Ser Cys Ser Ser Gly Glu His Glu Gly Tyr Val Thr Ala Arg Asp
        35                  40                  45

Thr Thr Lys Asn Arg Val Asn Gly Asp Ser Val Asn Asp Leu Ile Ser
    50                  55                  60

Phe Glu Glu Asp Leu Asn Leu Pro Gly Thr Ser Glu Glu Asn Ser Leu
65                  70                  75                  80

Leu Ser Pro Asp Ser Pro Ser Ser Ser Thr Pro Asn Phe Pro Pro Gln
                85                  90                  95

Tyr Glu Ser Gln Asn Leu Lys Glu Ser Val Ile Val Arg Asn Ser Ile
            100                 105                 110

Phe Trp Phe Thr Glu Ser Glu Val Asp Ala Ala Lys Leu Val Leu Pro
        115                 120                 125

Ile Ser Asn Lys Gly Arg Val Cys Arg Phe Asp Asn Asp Val Asp Asp
    130                 135                 140

Asn Leu Leu Glu Thr Thr Cys Arg Thr Leu Gln Ile Ser Met Gly Leu
145                 150                 155                 160

Cys Glu Arg Ser Arg Trp Leu Asn His Leu Ala Arg Asp Pro Val Met
                165                 170                 175

Ser Arg Leu Val Arg Glu Leu Asp Glu Asp Phe Ala Ser Thr Ser Asp
            180                 185                 190

Glu Pro Glu Asp Gln Gly Ala Glu Asn Gly Glu Glu Val Ser Arg Pro
        195                 200                 205

Pro Ala Ser Ser Phe Ser Ser Ser Ser Gly Ser Tyr His Thr Ile Val
    210                 215                 220

Gly Ile Leu Asp Glu Thr Val Gln Leu Asn Pro Leu Asn Pro Gly Met
225                 230                 235                 240

Thr Arg Val Ile Val Pro Ala Arg Arg Gly Ile Gln Ser Val Asp Ile
                245                 250                 255

Arg Arg Arg Ile Asn Glu Thr Pro Ser Ala Val Cys Ser Ala Ile Ile
            260                 265                 270

Arg Gln Ala Val Gln Lys Asp Pro Pro Ile Asp Gln Arg Met Met Glu
        275                 280                 285

Trp Met Arg Gly Phe Ala Glu Val Gly Thr Trp Ile Gln Glu Val Ala
    290                 295                 300

Pro Ala Ser Arg Lys His Asp Lys Tyr Ser Tyr Glu Val Pro Pro Leu
305                 310                 315                 320

Ser Glu Asp Trp Ile Leu Thr Ile His Lys Gln Pro Pro Ile Arg Gln
                325                 330                 335

Gly Leu Glu Ser Gln Gln Trp Lys Cys Ala Ala Cys Arg Gln Ser Leu
            340                 345                 350

His Thr His Asn Ile Asn Asp Arg Glu Gln Cys Pro Arg Phe Cys Asp
        355                 360                 365

Tyr Tyr Gly Leu Phe Phe Cys Ser Leu Cys His Gly Gly Glu Lys Ser
    370                 375                 380

Ile Leu Pro Ala Arg Val Leu Asn Gln Trp Ser Phe Thr Glu Leu Pro
385                 390                 395                 400

Val Ser Asp Arg Ala Gln Arg Phe Leu Arg Ala Val Arg Glu Ser Pro
                405                 410                 415

Val Phe Arg Ile Arg Asp Leu Pro Gly Asp Leu Val Lys Lys Asn Lys
            420                 425                 430

Ala Leu Arg Ala Val Val Glu Leu Arg Gln Lys Leu Lys His Met Glu
        435                 440                 445

Gly Phe Ile Lys Ile Cys Ile Asp Ala Ser Asn Gln Val Phe Glu Phe
    450                 455                 460

Gly Asn Leu Ser Thr Met Phe Ala Ser Ile Asp Arg Tyr Leu Leu Glu
465                 470                 475                 480

His Asp Asp Leu Phe Ser Leu Asn Asp Leu Gln Arg Ile Tyr Asn Lys
                485                 490                 495

Phe Gln Asp Leu Leu Ser Leu Leu Glu Pro Leu Ala Lys Arg Ala Arg
            500                 505                 510

Glu His Ile Ile His Cys Lys Lys Cys Arg Leu Gln Ala Pro Val Cys
        515                 520                 525

Val Arg Cys Asn Asp Met Thr Asp Arg Leu Phe Ala Phe Glu Glu Arg
    530                 535                 540

Ala Val Ser Arg Cys Glu Gly Cys Gly His Leu Ser His Ser Pro Lys
545                 550                 555                 560

Cys Pro Arg Arg Asp Val Pro Arg Asp Thr His Cys Pro Lys Cys Ala
                565                 570                 575

Arg Leu Lys Thr Arg Ala Asn Gly Gly Gly Gly Arg Ser Ser Arg Ile
            580                 585                 590

Ile Gly Asp Val Val
        595